# EUROPEAN PATENT APPLICATION

(11) **EP 4 559 523 A2**
(43) Date of publication of application: **28.05.2025**
(21) Application number: 25155404.4
(22) Date of filing: 25.04.2018
(51) Int. Cl.: A61P 27/02

(54) **FORMULATIONS FOR EXTENDING LIFESPAN AND HEALTHSPAN**

(30) Priority: 25.04.2017 US 201762489884 P; 22.03.2018 US 201862646734 P
(62) Divisional of application: 18790088.1
(71) Applicant: Buck Institute for Research on Aging, Novato, CA 94945 (US)
(72) Inventor: KENNEDY, Brian, Novato, 94945 (US); LITHGOW, Gordon J., Novato, 94945 (US); WELDON, Thomas, Novato, 94945 (US); EDGAR, Daniel, Novato, 94945 (US); LUCANIC, Mark, Novato, 94945 (US)
(74) Representative: HGF

(57) **Abstract**

Described herein are compositions for delaying onset or delaying progression of frailty, reversing aging phenotype, extending healthspan, compressing morbidity, increasing lifespan and reducing formation of senescent cells.

## Description

### CROSS-REFERENCE

This application claims the benefit of U.S. Provisional Application No. 62/489,884 filed April 25, 2017; and U.S. Provisional Application No. 62/646,734 filed March 22, 2018, which are incorporated by reference herein in their entirety.

### BACKGROUND OF THE INVENTION

Pharmacological treatment and prevention of the natural declines of age and aging-related diseases has presented challenges to the medical community in part because of the stringent characteristics required of pharmacological agents for this purpose. Aging patient populations impose unusually high burdens on pharmacological therapies to be bioavailable, nontoxic, and lacking long-term adverse effects. Prevention requires treatment of patients which may be at risk to aging-related disorders but experiencing no or mild symptoms, requiring agents that do not damage existing health. Treatment of patients with existing age-related disease requires a high requirement for nontoxicity, so as to not exacerbate existing health conditions. Moreover, treatment or prevention of aging-related disorders in general likely requires treatment with pharmacological agents for many years, thus requiring such agents to lack cumulative toxicity or long-term deleterious effects on organ systems.

Accordingly, there is a need for discovery of pharmacological agents and combinations of pharmacological agents that are nontoxic and suitable for administration to subjects before age related disorders are apparent or acute, or for the longer-term administration.

The primary and secondary metabolites present in dietary fruits and vegetables are a chemically diverse pharmacopeia which meets the criteria for treatment of aging related diseases. Due to their longstanding presence in the human diet, many of these compounds have very low toxicity and well-understood pharmacokinetic parameters. Screening of compounds from these sources thus provides great potential for the therapy of aging and aging-related conditions.

### SUMMARY OF THE INVENTION

Disclosed herein, in certain embodiments, are methods of extending lifespan in a subject in need thereof comprising administering to the subject a therapeutically effective amount of berberine, a vitamin A compound, and α-ketoglutarate (AKG). In some instances, berberine, the vitamin A compound and AKG is administered to the subject as a single composition. In some instances, the berberine, the vitamin A compound, and α-ketoglutarate are administered to the subject separately. In some instances, the berberine, the vitamin A compound, and α-ketoglutarate are administered to the subject within a 24 hour period. In some instances, the vitamin A compound is selected from the group consisting of retinol, retinal, retinoic acid, retinyl palmitate, alpha-carotene, beta-carotene, and gamma-carotene. In some instances, the vitamin A compound is retinoic acid or retinyl palmitate. In some instances, AKG is formulated as a calcium salt. In some instances, the subject is a mammal. In some instances, the mammal is a human. In some instances, the mammal is a dog. In some instances, the mammal is a cat. In some instances, the mammal is livestock. In some instances, the livestock is selected from the group consisting of cattle, sheep, goats, swine, poultry, and equine animals. In some instances, the berberine, the vitamin A compound, and α-ketoglutarate are administered once daily. In some instances, the berberine, the vitamin A compound, and α-ketoglutarate are administered twice daily. In some instances, the berberine, the vitamin A compound, and α-ketoglutarate are administered in the morning and evening. In some instances, the berberine, the vitamin A compound, and α-ketoglutarate are administered once a week. In some instances, the berberine, the vitamin A compound, and α-ketoglutarate are administered once a month.

Disclosed herein, in certain embodiments, are methods of delaying onset or delaying progression of frailty in a subject in need thereof comprising administering to the subject a therapeutically effective amount of berberine, a vitamin A compound, and α-ketoglutarate (AKG). In some instances, berberine, the vitamin A compound and AKG is administered to the subject as a single composition. In some instances, the berberine, the vitamin A compound, and α-ketoglutarate are administered to the subject separately. In some instances, the berberine, the vitamin A compound, and α-ketoglutarate are administered to the subject within a 24 hour period. In some instances, the vitamin A compound is selected from the group consisting of retinol, retinal, retinoic acid, retinyl palmitate, alpha-carotene, beta-carotene, and gamma-carotene. In some instances, the vitamin A compound is retinoic acid or retinyl palmitate. In some instances, AKG is formulated as a calcium salt. In some instances, the subject is a mammal. In some instances, the mammal is a human. In some instances, the mammal is a dog. In some instances, the mammal is a cat. In some instances, the mammal is livestock. In some instances, the livestock is selected from the group consisting of cattle, sheep, goats, swine, poultry, and equine animals.In some instances, the berberine, the vitamin A compound, and α-ketoglutarate are administered once daily. In some instances, the berberine, the vitamin A compound, and α-ketoglutarate are administered twice daily. In some instances, the berberine, the vitamin A compound, and α-ketoglutarate are administered in the morning and evening. In some instances, the berberine, the vitamin A compound, and α-ketoglutarate are administered once a week. In some instances, the berberine, the vitamin A compound, and α-ketoglutarate are administered once a month.

Disclosed herein, in certain embodiments, are compositions comprising a therapeutically effective amount of berberine, a therapeutically effective amount of a vitamin A compound, a therapeutically effective amount of AKG, and a pharmaceutically acceptable excipient. In some instances, the vitamin A compound is selected from the group consisting of retinol, retinal, retinoic acid, retinyl palmitate, alpha-carotene, beta-carotene, and gamma-carotene. In some instances, the vitamin A compound is retinoic acid or retinyl palmitate. In some instances, AKG is formulated as a calcium salt. In some instances, the composition further comprises silymarin. In some instances, the therapeutically effective amount of berberine is at least 1,000 mg and no greater than 5,000 mg. In some instances, the therapeutically effective amount of the vitamin A compound is at least 10,000 IU and no greater than 20,000 IU. In some instances, the therapeutically effective amount of α-ketoglutarate is at least 350 mg and no greater than 750 mg. In some instances, the composition is formulated into an orally administered form. In some instances, the orally administered form comprises a tablet, a powder, a suspension, a serum, an emulsion, a capsule, a granule, a pill, a gel, a solution, or a syrup. In some instances, the orally administered form is a sustained release dosage form. In some instances, the orally administered form is formulated into animal feed. In some instances, the orally administered form is the gel. In some instances, the composition is formulated into an injectable administered form. In some instances, the injectable administered form comprises a liquid, a suspension, or a solution. In some instances, the injectable administered form is a sustained release dosage form. In some instances, the composition is formulated into a topically administered form. In some instances, the topically administered form is a cream, a foam, a gel, a lotion, an ointment, or a serum. In some instances, the composition is formulated into a hair care product. In some instances, the hair care product is a shampoo, a conditioner, hair spray, or a moisturizer. In some instances, the pharmaceutically acceptable excipient comprises an antiadherent, a binder, a coating, a color, disintegrant, a flavor, a glidant, a lubricant, a preservative, a sorbent, or a vehicle.

Disclosed herein, in certain embodiments, are methods of delaying onset or delaying progression of frailty in a subject in need thereof comprising administering to the subject a therapeutically effective amount of AKG and a vitamin A compound. In some instances, AKG and the vitamin A compound is administered to the subject as a single composition. In some instances, AKG and the vitamin A compound are administered to the subject separately. In some instances, AKG and the vitamin A compound are administered to the subject within a 24 hour period. In some instances, the vitamin A compound is selected from the group consisting of retinol, retinal, retinoic acid, retinyl palmitate, alpha-carotene, beta-carotene, and gamma-carotene. In some instances, the vitamin A compound is retinoic acid or retinyl palmitate. In some instances, AKG is formulated as a calcium salt. In some instances, the subject is a mammal. In some instances, the mammal is a human. In some instances, the mammal is a dog. In some instances, the mammal is a cat. In some instances, the mammal is livestock. In some instances, the livestock is selected from the group consisting of cattle, sheep, goats, swine, poultry, and equine animals. In some instances, the AKG and the vitamin A compound are administered once daily. In some instances, AKG and the vitamin A compound are administered twice daily. In some instances, AKG and the vitamin A compound are administered in the morning and evening. In some instances, AKG and the vitamin A compound are administered once a week. In some instances, AKG and the vitamin A compound are administered once a month. In some instances, the subject is female.

Disclosed herein, in certain embodiments, are methods of treating or preventing alopecia in a subject in need thereof comprising administering to the subject a therapeutically effective amount of AKG and a vitamin A compound. In some instances, AKG and the vitamin A compound is administered to the subject as a single composition. In some instances, AKG and the vitamin A compound are administered to the subject separately. In some instances, AKG and the vitamin A compound are administered to the subject within a 24 hour period. In some instances, the vitamin A compound is selected from the group consisting of retinol, retinal, retinoic acid, retinyl palmitate, alpha-carotene, beta-carotene, and gamma-carotene. In some instances, the vitamin A compound is retinoic acid or retinyl palmitate. In some instances, AKG is formulated as a calcium salt. In some instances, the subject is a mammal. In some instances, the mammal is a human. In some instances, the mammal is a dog. In some instances, the mammal is a cat. In some instances, the mammal is livestock. In some instances, the livestock is selected from the group consisting of cattle, sheep, goats, swine, poultry, and equine animals. In some instances, the AKG and the vitamin A compound are administered once daily. In some instances, AKG and the vitamin A compound are administered twice daily. In some instances, AKG and the vitamin A compound are administered in the morning and evening. In some instances, AKG and the vitamin A compound are administered once a week. In some instances, AKG and the vitamin A compound are administered once a month.

Disclosed herein, in certain embodiments, are methods of extending lifespan in a subject in need thereof comprising administering to the subject a therapeutically effective amount of AKG and vitamin D. In some instances, AKG and vitamin D is administered to the subject as a single composition. In some instances, AKG and vitamin D are administered to the subject separately. In some instances, AKG and vitamin D are administered to the subject within a 24 hour period. In some instances, AKG is formulated as a calcium salt. In some instances, the subject is a mammal. In some instances, the mammal is a human. In some instances, the mammal is a dog. In some instances, the mammal is a cat. In some instances, the mammal is livestock. In some instances, the livestock is selected from the group consisting of cattle, sheep, goats, swine, poultry, and equine animals. In some instances, AKG and vitamin D are administered once daily. In some instances, AKG and vitamin D are administered twice daily. In some instances, AKG and vitamin D are administered in the morning and evening. In some instances, AKG and vitamin D are administered once a week. In some instances, AKG and vitamin D are administered once a month. In some instances, the subject is female.

Disclosed herein, in certain embodiments, are methods of delaying onset or delaying progression of frailty in a subject in need thereof comprising administering to the subject a therapeutically effective amount of AKG and vitamin D. In some instances, AKG and vitamin D is administered to the subject as a single composition. In some instances, AKG and vitamin D are administered to the subject separately. In some instances, AKG and vitamin D are administered to the subject within a 24 hour period. In some instances, AKG is formulated as a calcium salt. In some instances, the subject is a mammal. In some instances, the mammal is a human. In some instances, the mammal is a dog. In some instances, the mammal is a cat. In some instances, the mammal is livestock. In some instances, the livestock is selected from the group consisting of cattle, sheep, goats, swine, poultry, and equine animals. In some instances, AKG and vitamin D are administered once daily. In some instances, AKG and vitamin D are administered twice daily. In some instances, AKG and vitamin D are administered in the morning and evening. In some instances, AKG and vitamin D are administered once a week. In some instances, AKG and vitamin D are administered once a month. In some instances, the subject is female.

Disclosed herein, in certain embodiments, are methods of extending lifespan in a subject in need thereof comprising administering to the subject a composition comprising a therapeutically effective amount of a calcium salt of alpha-ketoglutarate (Ca-AKG). In some instances, the composition consists essentially of a therapeutically effective amount of a calcium salt of alpha-ketoglutarate (Ca-AKG). In some instances, the subject is a mammal. In some instances, the mammal is a human. In some instances, the mammal is a dog. In some instances, the mammal is a cat. In some instances, the mammal is livestock. In some instances, the livestock is selected from the group consisting of cattle, sheep, goats, swine, poultry, and equine animals. In some instances, the composition is administered once daily. In some instances, the composition is administered twice daily. In some instances, the composition is administered in the morning and evening. In some instances, the composition is administered once a week. In some instances, the composition is administered once a month. In some instances, the subject is female.

Disclosed herein, in certain embodiments, are methods of delaying onset or delaying progression of frailty in a subject in need thereof comprising administering to the subject a composition comprising a therapeutically effective amount of a calcium salt of alpha-ketoglutarate (Ca-AKG). In some instances, the delaying onset or delaying progression of frailty comprises delaying onset or delaying progression of a frailty phenotype. In some instances, the frailty phenotype is selected from the group consisting of dermatitis, kyphosis, tremor, and alopecia. In some instances, the frailty phenotype is dermatitis. In some instances, the frailty phenotype is kyphosis. In some instances, the frailty phenotype is tremor. In some instances, the frailty phenotype is alopecia. In some instances, the composition consists essentially of a therapeutically effective amount of a calcium salt of alpha-ketoglutarate (Ca-AKG). In some instances, the subject is a mammal. In some instances, the mammal is a human. In some instances, the mammal is a dog. In some instances, the mammal is a cat. In some instances, the mammal is livestock. In some instances, the livestock is selected from the group consisting of cattle, sheep, goats, swine, poultry, and equine animals. In some instances, the composition is administered once daily. In some instances, the composition is administered twice daily. In some instances, the composition is administered in the morning and evening. In some instances, the composition is administered once a week. In some instances, the composition is administered once a month. In some instances, the subject is female. In some instances, the composition is formulated into an orally administered form. In some instances, the orally administered form comprises a tablet, a powder, a suspension, a serum, an emulsion, a capsule, a granule, a pill, a gel, a solution, or a syrup. In some instances, the orally administered form is a sustained release dosage form. In some instances, the orally administered form is formulated into animal feed. In some instances, the orally administered form is the gel. In some instances, the composition is formulated into an injectable administered form. In some instances, the injectable administered form comprises a liquid, a suspension, or a solution. In some instances, the injectable administered form is a sustained release dosage form. In some instances, the composition is formulated into a topically administered form. In some instances, the topically administered form is a cream, a foam, a gel, a lotion, an ointment, or a serum. In some instances, the composition is formulated into a hair care product. In some instances, the hair care product is a shampoo, a conditioner, hair spray, or a moisturizer. In some instances, the composition further comprises a pharmaceutically acceptable excipient selected from the group consisting of an antiadherent, a binder, a coating, a color, disintegrant, a flavor, a glidant, a lubricant, a preservative, a sorbent, or a vehicle. In some instances, the composition is administered to the subject for at least 4 months. In some instances, the composition is administered to the subject for at least 6 months, at least 8 months, at least 10 months, at least 12 months, at least 14 months, at least 16 months, or at least 18 months.

Disclosed herein, in certain embodiments, are methods of extending healthspan in a subject in need thereof comprising administering to the subject a composition comprising a therapeutically effective amount of a calcium salt of alpha-ketoglutarate (Ca-AKG). In some instances, extending healthspan comprises a delay in onset or progression of an age-related phenotype. In some instances, the age-related phenotype is selected from the group consisting of graying hair, hair loss, and increased cell senescence. In some instances, the age-related phenotype is graying hair. In some instances, the age-related phenotype is hair loss. In some instances, the age-related phenotype is increased cell senescence. In some instances, the composition consists essentially of a therapeutically effective amount of a calcium salt of alpha-ketoglutarate (Ca-AKG). In some instances, the subject is a mammal. In some instances, the mammal is a human. In some instances, the mammal is a dog. In some instances, the mammal is a cat. In some instances, the mammal is livestock. In some instances, the livestock is selected from the group consisting of cattle, sheep, goats, swine, poultry, and equine animals. In some instances, the composition is administered once daily. In some instances, the composition is administered twice daily. In some instances, the composition is administered in the morning and evening. In some instances, the composition is administered once a week. In some instances, the composition is administered once a month. In some instances, the subject is female. In some instances, the composition is formulated into an orally administered form. In some instances, the orally administered form comprises a tablet, a powder, a suspension, a serum, an emulsion, a capsule, a granule, a pill, a gel, a solution, or a syrup. In some instances, the orally administered form is a sustained release dosage form. In some instances, the orally administered form is formulated into animal feed. In some instances, the orally administered form is the gel. In some instances, the composition is formulated into an injectable administered form. In some instances, the injectable administered form comprises a liquid, a suspension, or a solution. In some instances, the injectable administered form is a sustained release dosage form. In some instances, the composition is formulated into a topically administered form. In some instances, the topically administered form is a cream, a foam, a gel, a lotion, an ointment, or a serum. In some instances, the composition is formulated into a hair care product. In some instances, the hair care product is a shampoo, a conditioner, hair spray, or a moisturizer. In some instances, the composition further comprises a pharmaceutically acceptable excipient selected from the group consisting of an antiadherent, a binder, a coating, a color, disintegrant, a flavor, a glidant, a lubricant, a preservative, a sorbent, or a vehicle. In some instances, the composition is administered to the subject for at least 4 months. In some instances, the composition is administered to the subject for at least 6 months, at least 8 months, at least 10 months, at least 12 months, at least 14 months, at least 16 months, or at least 18 months.

Disclosed herein, in certain embodiments, are methods for helping to maintain health in a subject comprising administering to the subject a composition comprising a therapeutically effective amount of berberine, a vitamin A compound, and α-ketoglutarate (AKG). In some instances, the vitamin A compound is selected from the group consisting of retinol, retinal, retinoic acid, retinyl palmitate, alpha-carotene, beta-carotene, and gamma-carotene. In some instances, the vitamin A compound is retinoic acid or retinyl palmitate. In some instances, AKG is formulated as a calcium salt. In some instances, the helping to maintain health comprises helping to maintain a healthy musculoskeletal system. In some instances, the composition is formulated into an orally administered form. In some instances, the orally administered form comprises a tablet, a powder, a suspension, a serum, an emulsion, a capsule, a granule, a pill, a gel, a solution, or a syrup. In some instances, the subject is a human. In some instances, the composition is administered to the subject once daily. In some instances, the composition is administered to the subject twice daily.

Disclosed herein, in certain embodiments, are methods for helping to maintain health in need thereof comprising administering to the subject a composition comprising a therapeutically effective amount of a vitamin A compound, and α-ketoglutarate (AKG). In some instances, the vitamin A compound is selected from the group consisting of retinol, retinal, retinoic acid, retinyl palmitate, alpha-carotene, beta-carotene, and gamma-carotene. In some instances, the vitamin A compound is retinoic acid or retinyl palmitate. In some instances, AKG is formulated as a calcium salt. In some instances, the helping to maintain health comprises helping to maintain a healthy musculoskeletal system. In some instances, the composition is formulated into an orally administered form. In some instances, the orally administered form comprises a tablet, a powder, a suspension, a serum, an emulsion, a capsule, a granule, a pill, a gel, a solution, or a syrup. In some instances, the subject is a human. In some instances, the composition is administered to the subject once daily. In some instances, the composition is administered to the subject twice daily.

Disclosed herein, in certain embodiments, are methods for helping to maintain health in a subject comprising administering to the subject a composition comprising a therapeutically effective amount of a vitamin D, and α-ketoglutarate (AKG). In some instances, AKG is formulated as a calcium salt. In some instances, the helping to maintain health comprises helping to maintain a healthy musculoskeletal system. In some instances, the composition is formulated into an orally administered form. In some instances, the orally administered form comprises a tablet, a powder, a suspension, a serum, an emulsion, a capsule, a granule, a pill, a gel, a solution, or a syrup. In some instances, the subject is a human. In some instances, the composition is administered to the subject once daily. In some instances, the composition is administered to the subject twice daily.

Disclosed herein, in certain embodiments, are methods for helping to maintain health in a subject comprising administering to the subject a composition comprising a therapeutically effective amount of a calcium salt of alpha-ketoglutarate (Ca-AKG). In some instances, the helping to maintain health comprises helping to maintain a healthy musculoskeletal system. In some instances, the composition is formulated into an orally administered form. In some instances, the orally administered form comprises a tablet, a powder, a suspension, a serum, an emulsion, a capsule, a granule, a pill, a gel, a solution, or a syrup. In some instances, the subject is a human. In some instances, the composition is administered to the subject once daily. In some instances, the composition is administered to the subject twice daily. In some instances, the helping to maintain health comprises helping to maintain hair density. In some instances, the helping to maintain health comprises helping to maintain hair pigmentation. In some instances, the helping to maintain health comprises helping to maintain skin health. In some instances, the helping to maintain health comprises helping to maintain normal spine curvature.

Disclosed herein, in certain embodiments, are methods of treating or preventing alopecia in a subject in need thereof comprising administering to the subject a therapeutically effective amount of AKG and vitamin D. In some instances, AKG and vitamin D are administered to the subject as a single composition. In some instances, AKG and vitamin D are administered to the subject separately. In some instances, AKG and vitamin D are administered to the subject within a 24 hour period. In some instances, AKG is formulated as a calcium salt. In some instances, the subject is a mammal. In some instances, the mammal is a human. In some instances, the mammal is a dog. In some instances, the mammal is a cat. In some instances, the mammal is livestock. In some instances, the livestock is selected from the group consisting of cattle, sheep, goats, swine, poultry, and equine animals. In some instances, the AKG and vitamin D are administered once daily. In some instances, AKG and vitamin D are administered twice daily. In some instances, AKG and vitamin D are administered in the morning and evening. In some instances, AKG and vitamin D are administered once a week. In some instances, AKG and vitamin D are administered once a month.

Disclosed herein, in certain embodiments, are methods of extending lifespan in a subject in need thereof comprising administering to the subject a therapeutically effective amount of a vitamin A compound and α-ketoglutarate (AKG). In some instances, the vitamin A compound and AKG is administered to the subject as a single composition. In some instances, the vitamin A compound and α-ketoglutarate are administered to the subject separately. In some instances, the vitamin A compound and α-ketoglutarate are administered to the subject within a 24 hour period. In some instances, the vitamin A compound is selected from the group consisting of retinol, retinal, retinoic acid, retinyl palmitate, alpha-carotene, beta-carotene, and gamma-carotene. In some instances, the vitamin A compound is retinoic acid or retinyl palmitate. In some instances, AKG is formulated as a calcium salt. In some instances, the subject is a mammal. In some instances, the mammal is a human. In some instances, the mammal is a dog. In some instances, the mammal is a cat. In some instances, the mammal is livestock. In some instances, the livestock is selected from the group consisting of cattle, sheep, goats, swine, poultry, and equine animals. In some instances, the vitamin A compound and α-ketoglutarate are administered once daily. In some instances, the vitamin A compound and α-ketoglutarate are administered twice daily. In some instances, the vitamin A compound and α-ketoglutarate are administered in the morning and evening. In some instances, the vitamin A compound and α-ketoglutarate are administered once a week. In some instances, the vitamin A compound and α-ketoglutarate are administered once a month.

Disclosed herein, in certain embodiments, are methods of extending healthspan in a subject in need thereof comprising administering to the subject a therapeutically effective amount of a vitamin A compound and α-ketoglutarate (AKG). In some instances, the vitamin A compound and AKG is administered to the subject as a single composition. In some instances, the vitamin A compound and α-ketoglutarate are administered to the subject separately. In some instances, the vitamin A compound and α-ketoglutarate are administered to the subject within a 24 hour period. In some instances, the vitamin A compound is selected from the group consisting of retinol, retinal, retinoic acid, retinyl palmitate, alpha-carotene, beta-carotene, and gamma-carotene. In some instances, the vitamin A compound is retinoic acid or retinyl palmitate. In some instances, AKG is formulated as a calcium salt. In some instances, extending healthspan comprises a delay in onset or progression of an age-related phenotype. In some instances, the age-related phenotype is selected from the group consisting of graying hair, hair loss, and increased cell senescence. In some instances, the age-related phenotype is graying hair. In some instances, the age-related phenotype is hair loss. In some instances, the age-related phenotype is increased cell senescence. In some instances, the subject is a mammal. In some instances, the mammal is a human.

Disclosed herein, in certain embodiments, are methods of extending healthspan in a subject in need thereof comprising administering to the subject a therapeutically effective amount of vitamin D and α-ketoglutarate (AKG). In some instances, vitamin D and AKG is administered to the subject as a single composition. In some instances, vitamin D and AKG are administered to the subject separately. In some instances, vitamin D and α-ketoglutarate are administered to the subject within a 24 hour period. In some instances, AKG is formulated as a calcium salt. In some instances, extending healthspan comprises a delay in onset or progression of an age-related phenotype. In some instances, the age-related phenotype is selected from the group consisting of graying hair, hair loss, and increased cell senescence. In some instances, the age-related phenotype is graying hair. In some instances, the age-related phenotype is hair loss. In some instances, the age-related phenotype is increased cell senescence. In some instances, the subject is a mammal. In some instances, the mammal is a human.

Disclosed herein, in certain embodiments, are methods for helping to maintain health in a subject comprising administering to the subject a composition that modulates pathways associated with normal aging process wherein the composition comprises a therapeutically effective amount of berberine, a vitamin A compound, and α-ketoglutarate (AKG).

Disclosed herein, in certain embodiments, are methods for helping to maintain health in a subject comprising administering to the subject a composition that modulates pathways associated with normal aging process wherein the composition comprises a therapeutically effective amount of a vitamin A compound and α-ketoglutarate (AKG).

Disclosed herein, in certain embodiments, are methods for helping to maintain health in a subject comprising administering to the subject a composition that modulates pathways associated with normal aging process wherein the composition comprises a therapeutically effective amount of vitamin D and α-ketoglutarate (AKG).

Disclosed herein, in certain embodiments, are methods for helping to maintain health in a subject comprising administering to the subject a composition that modulates pathways associated with normal aging process wherein the composition comprises a therapeutically effective amount of a calcium salt of alpha-ketoglutarate (Ca-AKG).

Disclosed herein, in certain embodiments, are methods for helping to maintain health in a subject comprising administering to the subject a composition that modulates pathways associated with normal aging process wherein the composition comprises a therapeutically effective amount of nicotinamide mononucleotide (NMN) and α-ketoglutarate (AKG).

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

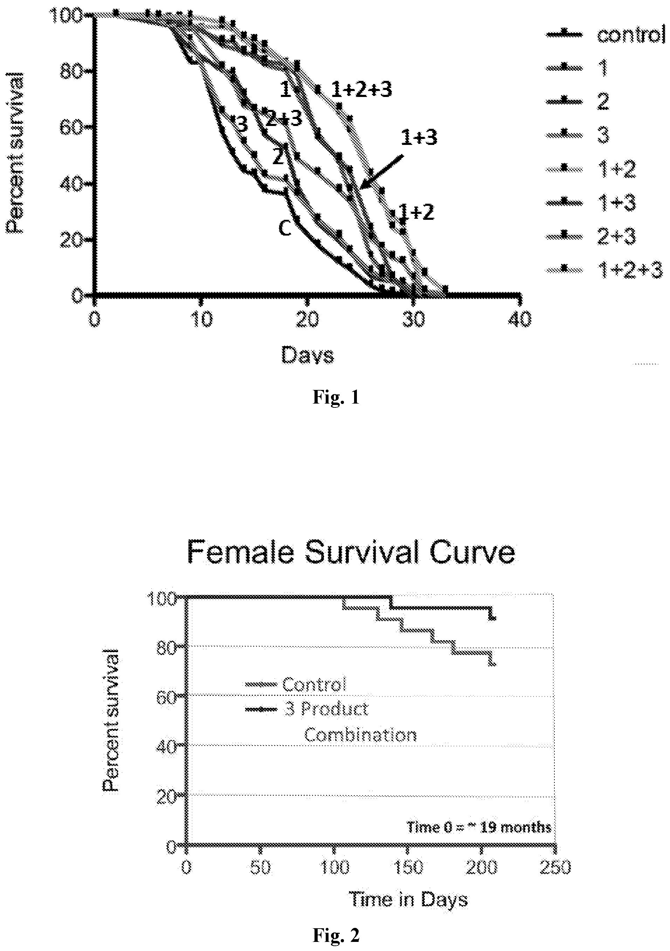

**Fig. 1** shows the lifespan effects of indicated compounds on *C*. *elegans* aging. Compound 1 is Ca-AKG. Compound 2 is retinoic acid (RA), a metabolite of vitamin A that mediates its functions. Compound 3 is berberine. Both Ca-AKG + RA and the combination of all three compounds have robust effects on longevity.
**Fig. 2** shows the effect of the combination of berberine, Ca-AKG, and retinoic acid (RA) has on survival in female mice compared to control female mice. (Cohort 1)

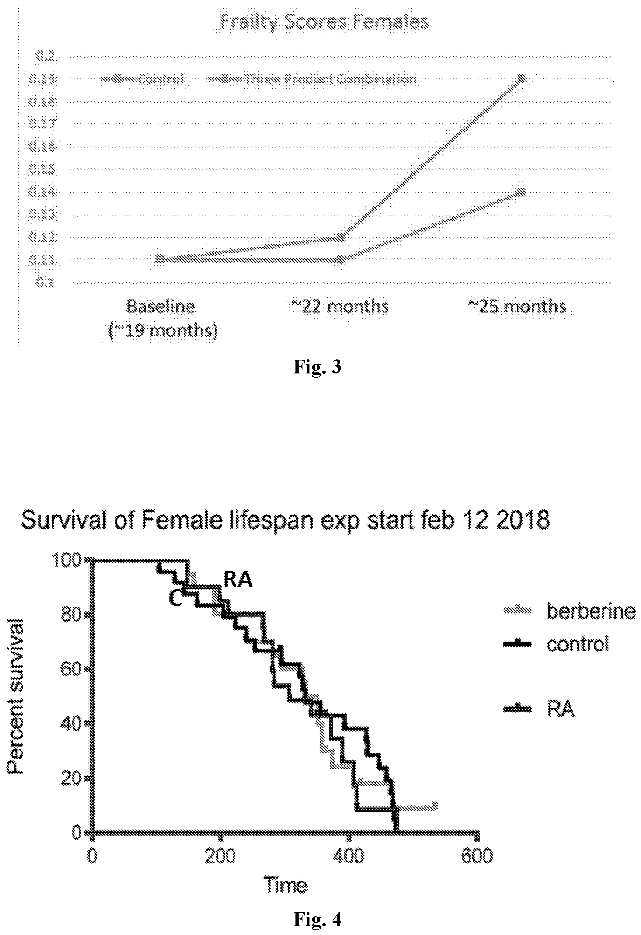

**Fig. 3** shows the effect of the combination of berberine, Ca-AKG, and vitamin A has on frailty scores in female mice (bottom line) compared to control female mice (top line). (Cohort 1)
**Fig. 4** shows the effect of berberine, control, or retinoic acid (RA) on female mice lifespan. (Cohort 1)

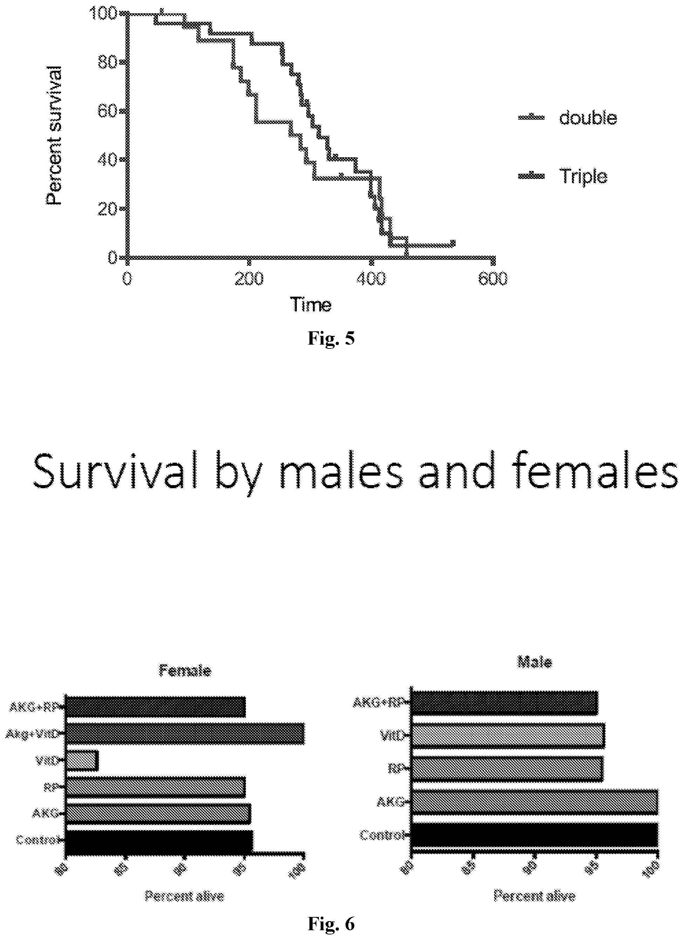

**Fig. 5** shows the effect of the combination of berberine, Ca-AKG, and retinoic acid (RA) (triple) and the combination of Ca-AKG and berberine (double) on female mice lifespan. (Cohort 1)
**Fig. 6** shows the effect of Ca-AKG + RP, Ca-AKG + Vitamin D, Vitamin D, retinyl palmitate (RP), Ca-AKG, and control on female mice lifespan (left), and the effect Ca-AKG + RP, Vitamin D, RP, Ca-AKG, and control on male mice lifespan (right). (Cohort 2)

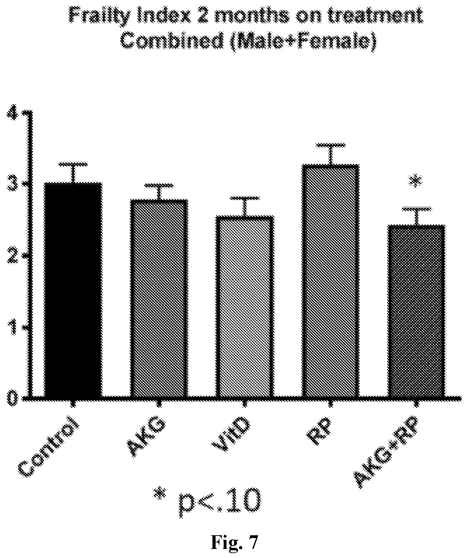

**Fig. 7** shows the effect of Ca-AKG, Vitamin D, RP, and the combination of Ca-AKG + RP, has on frailty scores in combined female and male mice compared to control. (Cohort 2)
**Fig. 8** shows the effect of Ca-AKG, Vitamin D, RP, Ca-AKG + RP, Ca-AKG + Vitamin D and control on female mice frailty scores after 2 months of treatment (left), and the effect Ca-AKG, Vitamin D, RP, Ca-AKG + RP, and control on male mice frailty scores after 2 months of treatment (right). (Cohort 2)

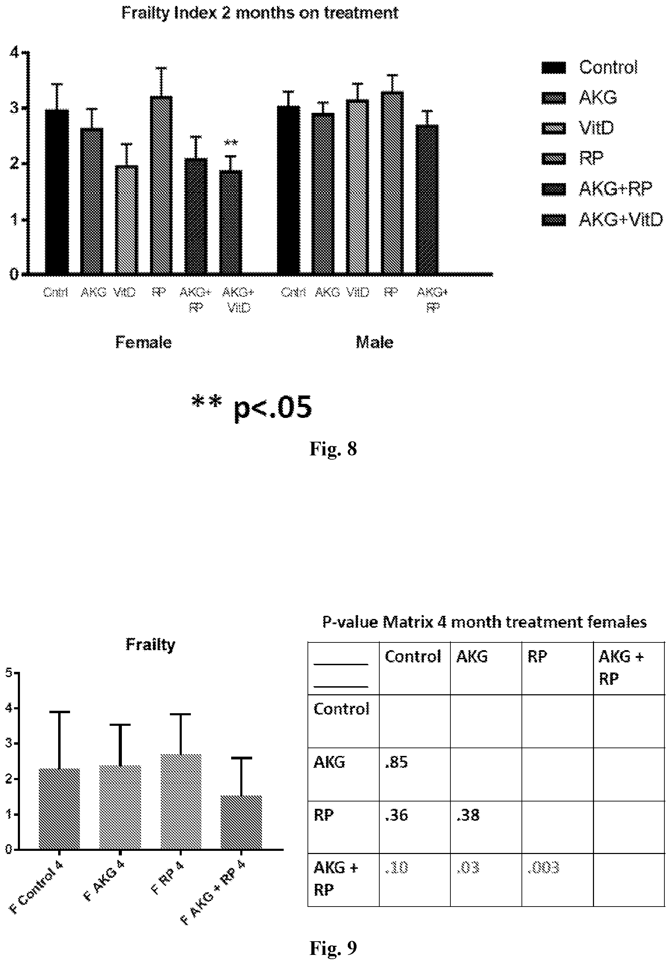

**Fig. 9** shows the effect of Ca-AKG, retinyl palmitate (RP), and the combination of Ca-AKG + RP on frailty scores in female mice compared to control (left) after four months of treatment. Starting at 18 months of age, female mice were provided with the natural products as indicated. Frailty was monitored at the initiation of the experiment and after four months treatment. The bar graph indicates the median cumulative frailty measure for each set of mice. The data shows that only the combined treatment of RP + Ca-AKG results in a statistically significant reduction in frailty. Statistical analysis is provided on the right, where the combined Ca-AKG + RP treatment shows a statistically significant reduction relative to all other treatments, indicative of a synergistic benefit of the two compounds in reducing frailty. (Cohort 2)

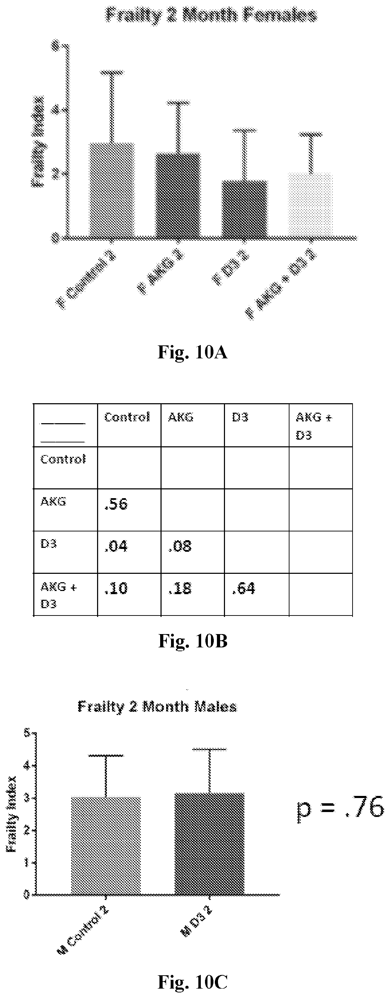

**Fig. 10A** **-** **Fig. 10C** show frailty index mice after 2 months of treatment with indicated compounds. **Fig. 10A** shows the effect of Ca-AKG, Vitamin D3, and the combination of Ca-AKG + vitamin D3 on frailty scores in female mice compared to control (left) after two months of treatment (>20 mice per cohort). Both vitamin D3 and Ca-AKG + vitamin D3 have lower frailty. In **Fig. 10B****,** the p values for each comparison are shown, demonstrating statistical significance for vitamin D3 and Ca-AKG + vitamin D3 in various contexts. In **Fig. 10C****,** a similar approach was taken toward male mice, showing no significant reduction in frailty from vitamin D3. This indicates a sex-specific benefit of vitamin D3 in females. (Cohort 2)

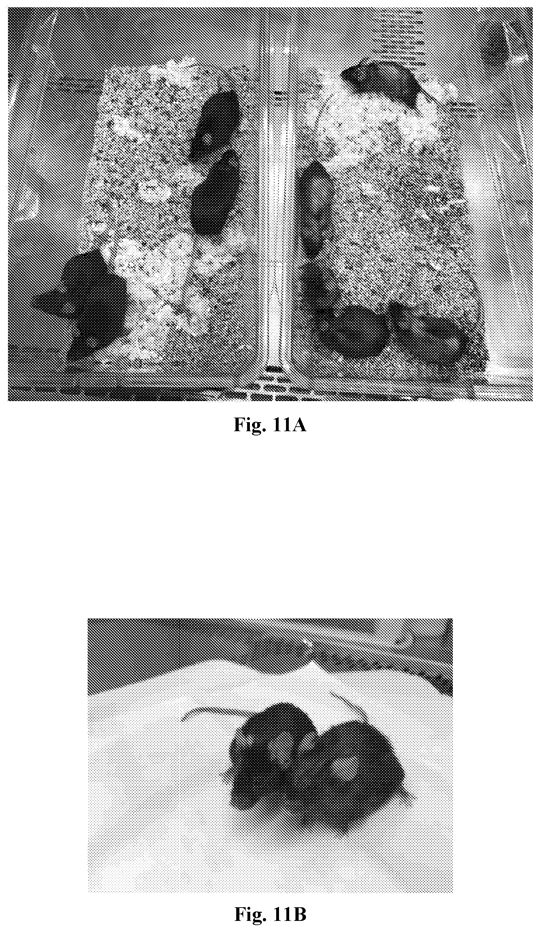

**Fig. 11A****-** **Fig. 11B** shows the effect of Ca-AKG on a mouse frailty phenotype. **Fig. 11A** shows a side-by-side comparison of cages of female C57BL6 mice treated with placebo (right) or Ca-AKG (left). Coat graying is dramatically reduced in cages where Ca-AKG is administered to the mice. **Fig. 11B** shows a side-by-side comparison of individual mice treated with placebo (right) or Ca-AKG (left). (Cohort 1)

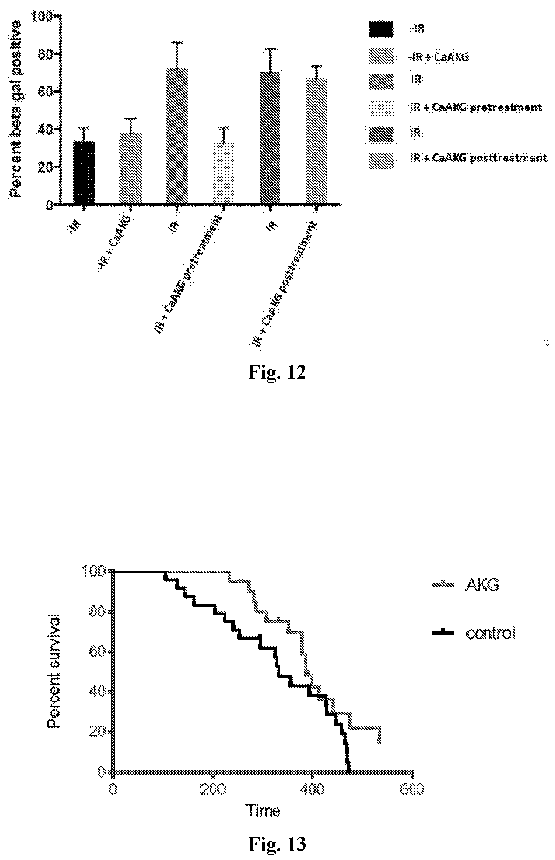

**Fig. 12** shows the effect of irradiation (IR) on cells and the expression of a marker of cellular senescence, senescence-associated β-galactosidase (beta gal). As shown, cells when exposed to irradiation demonstrate an increase in the expression of beta gal. When cells are pre-treated with Ca-AKG prior to exposure to irradiation, the expression of beta gal decreases relative to control. (Cohort 1)
**Fig. 13** shows the effect of Ca-AKG has on survival in female mice compared to control female mice. (Cohort 1)

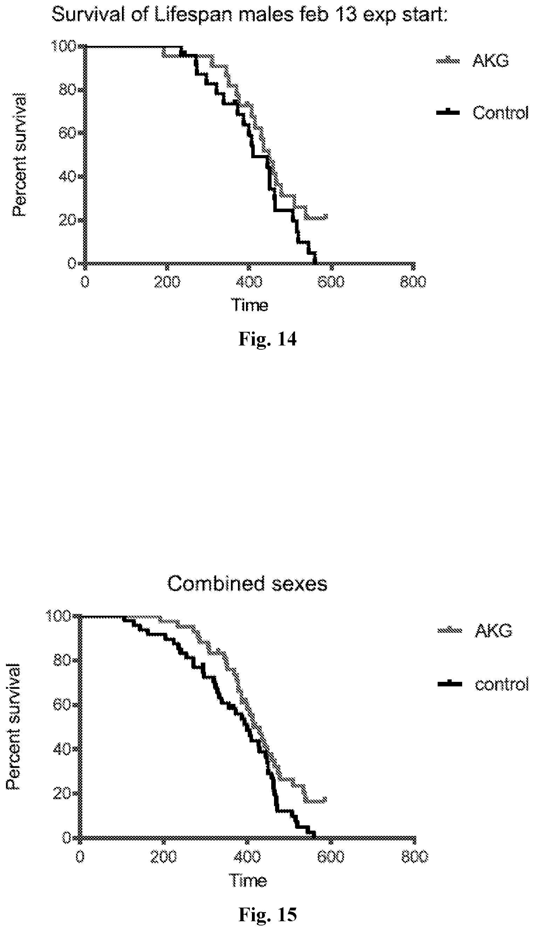

**Fig. 14** shows the effect of Ca-AKG has on survival in male mice compared to control male mice. (Cohort 1)
**Fig. 15** shows the effect on combined sexes of Ca-AKG and control on mice lifespan. (Cohort 1)

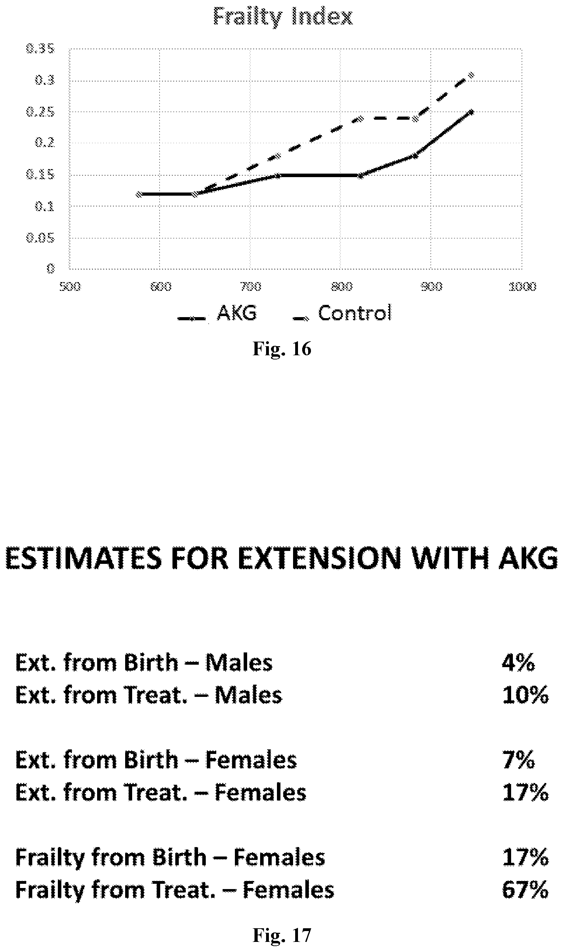

**Fig. 16** shows the effect of Ca-AKG has on frailty scores in female mice (bottom line) compared to control female mice (top line). (Cohort 1)
**Fig. 17** shows estimates for extension of lifespan in mice with treatment of Ca-AKG. (Cohort 1)

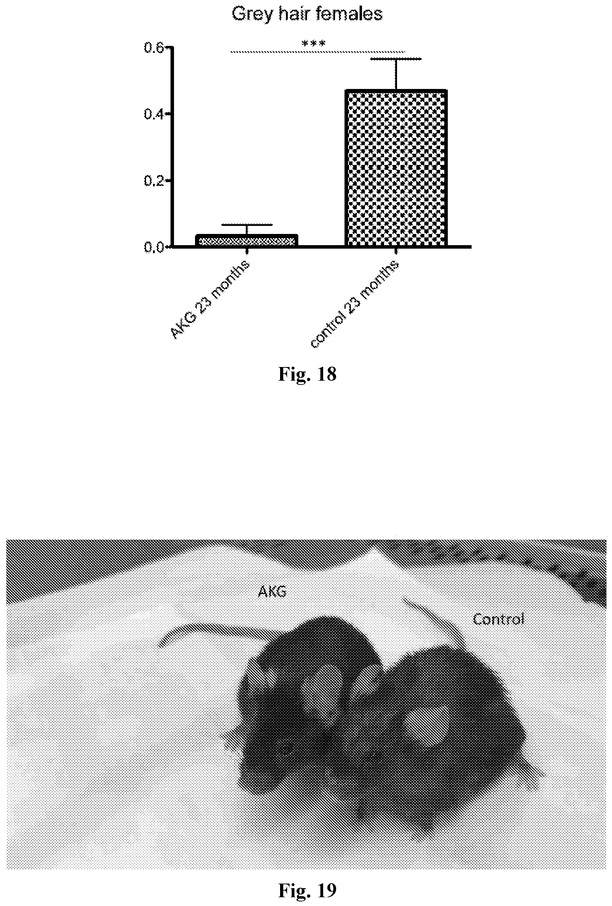

**Fig. 18** shows data demonstrating a reduction in grey hair in female mice upon treatment of Ca-AKG relative to control has measure at 23 months of life. (Cohort 1)
**Fig. 19** shows a picture of a mouse treated with Ca-AKG (left) next to a control mouse. As shown in the picture the mouse treated with Ca-AKG has less grey hair than the control mouse. (Cohort 1)

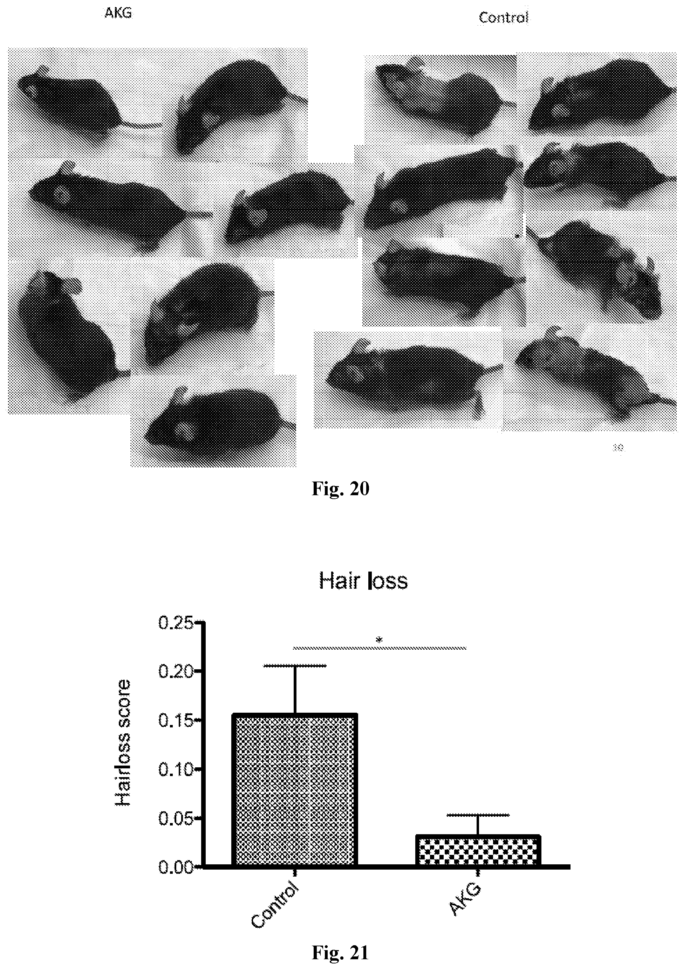

**Fig. 20** shows pictures of mice treated with Ca-AKG (left) next to pictures of control mice. As shown in the picture the mice treated with Ca-AKG have less gray hair, and reduced hair loss than the control mice. (Cohort 1)
**Fig. 21** shows data demonstrating a reduction in hair loss in mice upon treatment of Ca-AKG relative to control mice. (Cohort 1)

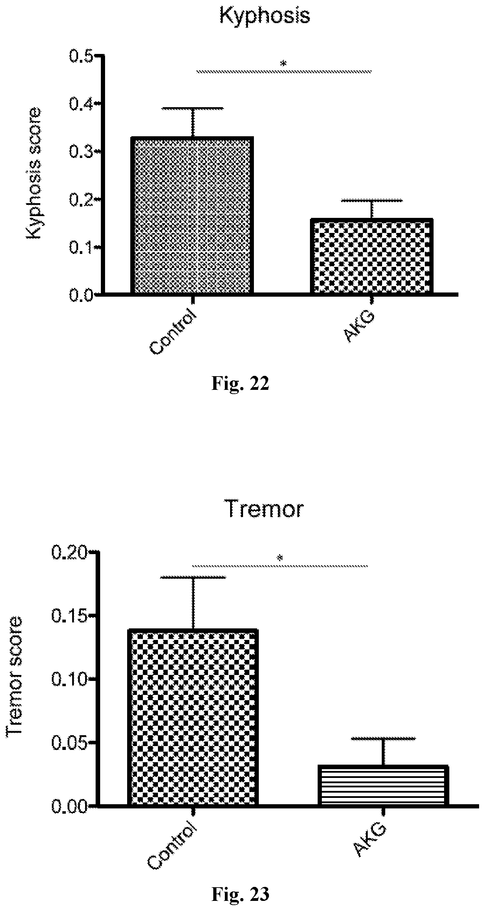

**Fig. 22** shows data demonstrating a reduction in kyphosis in mice upon treatment of Ca-AKG relative to control mice. (Cohort 1)
**Fig. 23** shows data demonstrating a reduction in tremors in mice upon treatment of Ca-AKG relative to control mice. (Cohort 1)

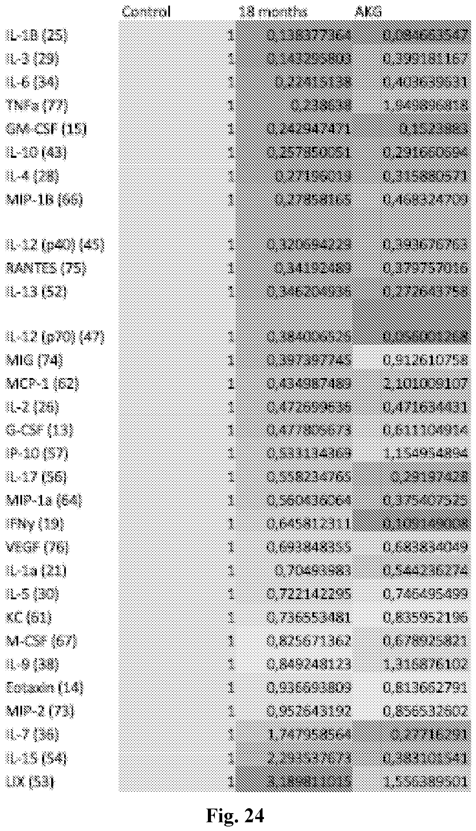

**Fig. 24** shows that a three month treatment of mice starting at 18 months of age is sufficient to dramatically reduce levels of inflammatory cytokines. Each horizontal line represents relative levels of one protein. The first column represents levels of cytokines three months after treatment and these values were normalized to 1. Middle column represent levels of cytokines for animals at 18 months. Notably, most numbers are <1, indicating that the levels of these inflammatory factors are increasing between 18 and 21 months of age. The column on the right represents Ca-AKG treated animals at 21 months of age, showing that Ca-AKG (almost exclusively) prevents, or in some cases even reverses, the increase in levels of inflammatory cytokines. (Cohort 2)

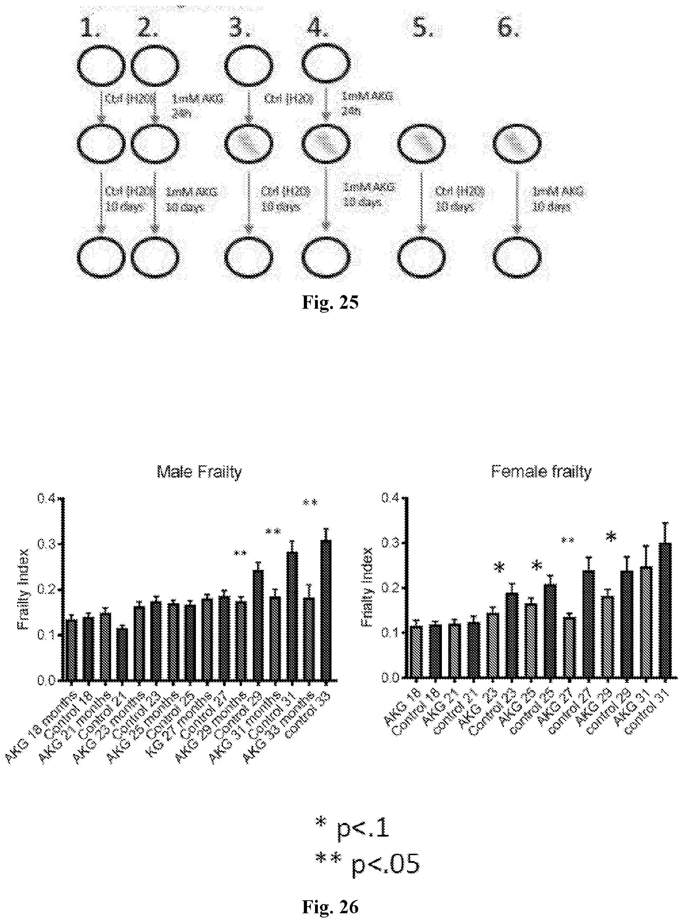

**Fig. 25** shows an exemplary schematic for studying cell senescence on irradiated cells that are either pre-treated or post-treated with Ca-AKG.
**Fig. 26** shows the effect of Ca-AKG on frailty scores in male mice compared to control male mice (left). Also shown is the effect of Ca-AKG on frailty scores in female mice compared to control female mice (right). (Cohort 1)

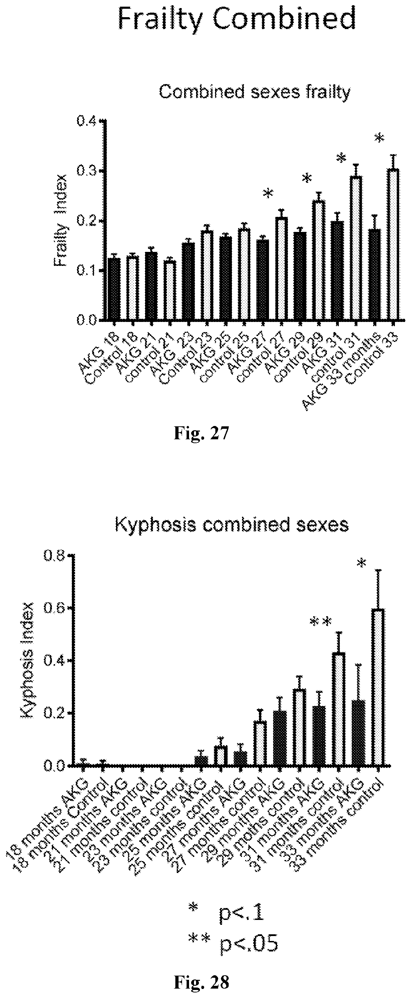

**Fig. 27** shows the effect of Ca-AKG on frailty scores relative to control in combined female and male mice. (Cohort 1)
**Fig. 28** shows the effect of Ca-AKG has on kyphosis relative to control in combined female and male mice. (Cohort 1)

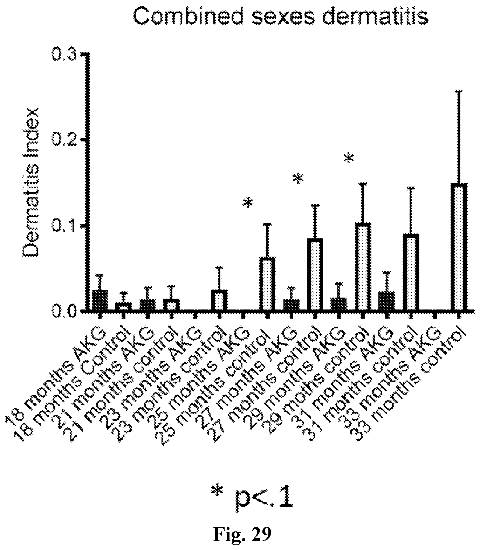

**Fig. 29** shows the effect of Ca-AKG has on dermatitis relative to control in combined female and male mice. (Cohort 1)

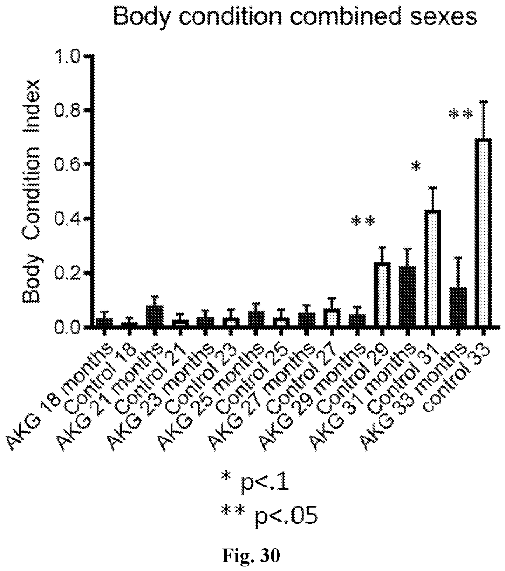

**Fig. 30** shows the effect of Ca-AKG has on body condition relative to control in combined female and male mice. Body condition score involves visual scoring and/or palpitation to assess the state of health of the animal. Signs of frailness result in higher frailty scores. (Cohort 1)

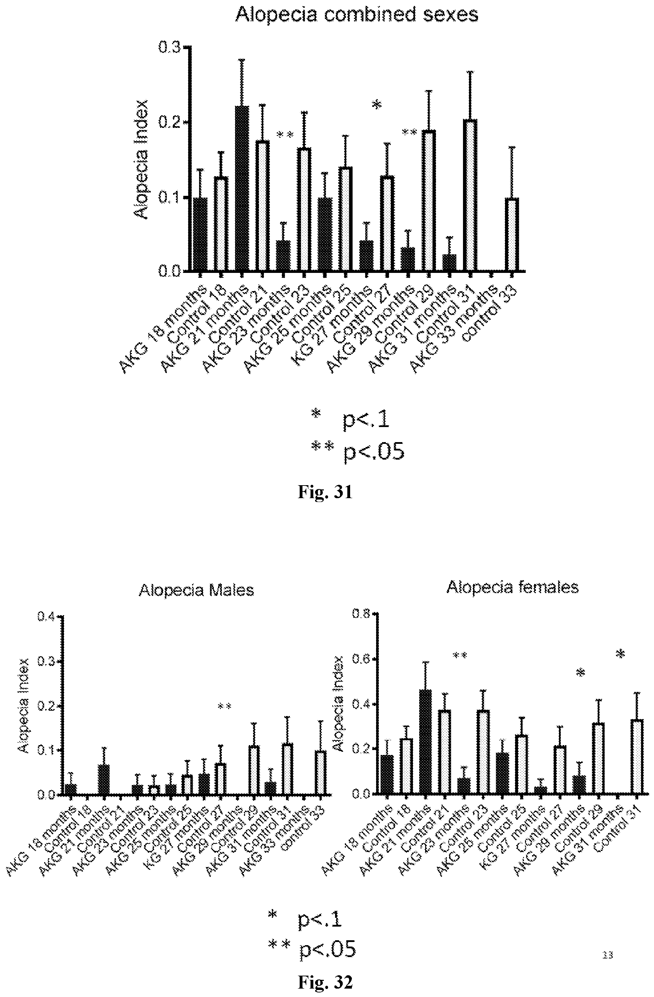

**Fig. 31** shows the effect of Ca-AKG has on alopecia relative to control in combined female and male mice. (Cohort 1)
**Fig. 32** shows the effect of Ca-AKG has on alopecia scores in male mice compared to control male mice (left). Also shown is the effect of Ca-AKG has on alopecia scores in female mice compared to control female mice (right). (Cohort 1)

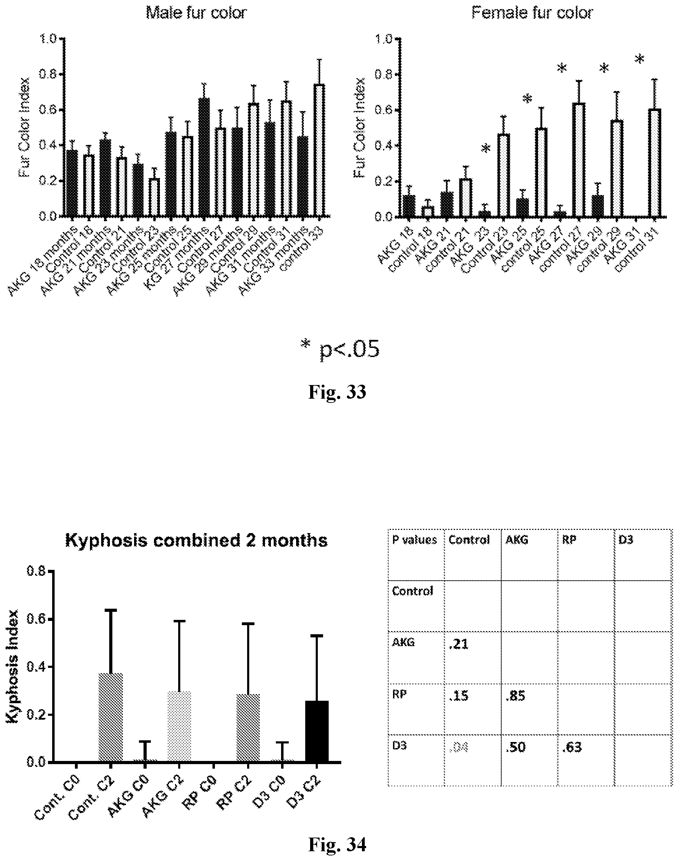

**Fig. 33** shows the effect of Ca-AKG has on fur color (hair greying) in male mice compared to control male mice (left). Also shown is the effect of Ca-AKG has on fur color in female mice compared to control female mice (right). (Cohort 1)
**Fig. 34** shows kyphosis scores at 2 months. Starting at 18 months of age, mice were treated with the indicated compounds. After treatment for two months vitamin D3 was found to reduce the kyphosis index significantly (p<.05). In this study male and female mice were combined, with over 40 mice per cohort balanced between sexes.

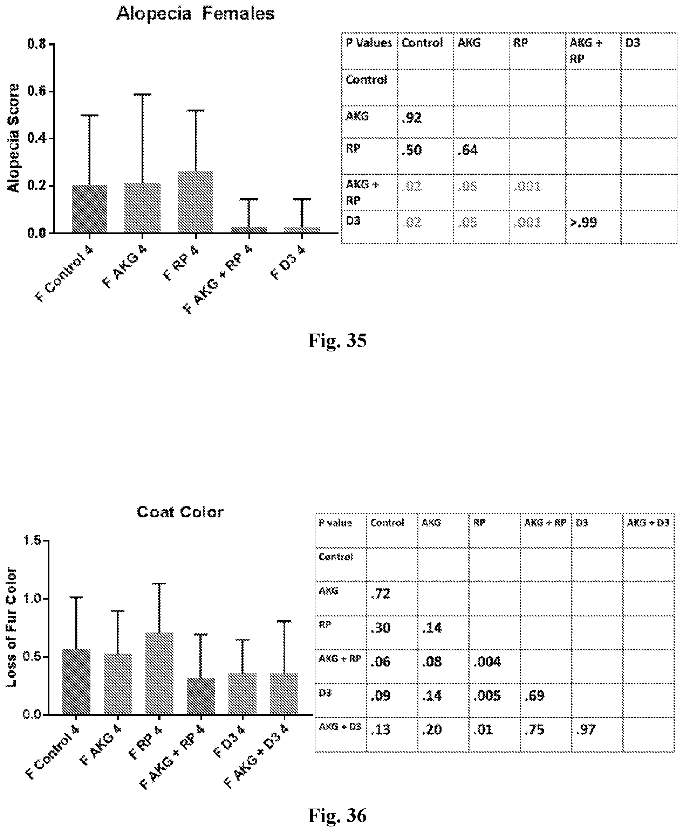

**Fig. 35** shows the effect of Ca-AKG, RP, Vitamin D3 and combination of Ca-AKG + RP on alopecia scores in female mice compared to control female mice after four month treatment. Alopecia was scored in female mice after four month treatment with the indicated compounds, starting at 18 months of age. While neither Ca-AKG nor RP had significant effects on their own, the combination dramatically reduced hair loss. In contrast vitamin D3 on its own was sufficient to reduce alopecia. P value matrix is indicated on the right, with both Ca-AKG + RP and Vitamin D3 showing significant reductions at p< .05.
**Fig. 36** shows the effect of Ca-AKG, RP, Vitamin D3 and combination of Ca-AKG + RP and Ca-AKG + Vitamin D3 on fur color. Several combinations of compounds either prevent or reverse changes in coat color with age. Female mice were treated with indicated compounds for four months, starting at 18 months of age. Ca-AKG + RP showed synergistic effects, and vitamin D3 treated mice had younger coat color whether or not Ca-AKG was added.

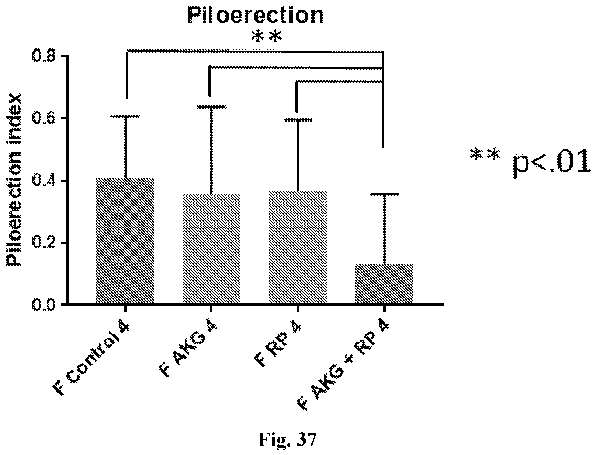

**Fig. 37** shows the effect of Ca-AKG, RP and combination of Ca-AKG + RP on piloerection in female mice. Four month treatment of Ca-AKG + RP results in a reduction in piloerection in female mice (>20 per cohort). Neither compound alone had an effect, and neither did vitamin D3 (data not shown). Piloerection is defined as involuntary hair bristling and can be thought of similarly to "goose bumps." This increases with age and can be triggered by coldness, reflecting an inability of the mice to maintain body temperature. While the Ca-AKG + RP mice did have increased body temperature relative to controls, it did not reach statistical significance (data not shown). Piloerection can also be triggered by fear or shock.

### DETAILED DESCRIPTION OF THE INVENTION

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

### Certain Terminology

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable excipient" or "pharmaceutically acceptable carrier" as used herein means a pharmaceutically acceptable material, composition or vehicle, such as a liquid or solid filler, diluent, excipient, solvent or encapsulating material. Each carrier must be "acceptable" in the sense of being compatible with the other ingredients of the formulation and not injurious to the patient. Some examples of materials which can serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

In certain embodiments, the term "prevent" or "preventing" as related to a disease or disorder may refer to a compound that, in a statistical sample, reduces the occurrence of the disorder or condition in the treated sample relative to an untreated control sample, or delays the onset or reduces the severity of one or more symptoms of the disorder or condition relative to the untreated control sample.

The terms "treat," "treating" or "treatment," as used herein, may include alleviating, abating or ameliorating a disease or condition symptoms, ameliorating the underlying causes of symptoms, inhibiting the disease or condition, e.g., arresting the development of the disease or condition, relieving the disease or condition, causing regression of the disease or condition, relieving a condition caused by the disease or condition, or stopping the symptoms of the disease or condition either prophylactically and/or therapeutically.

In certain embodiments, the term "modulator" as related to a protein or cellular process may refer to a compound that directly or indirectly changes the level of activity associated with the protein or cellular process.

In certain embodiments, the term "delay" or "delaying" as related to a disease or disorder may refer to a compound that, in a statistical sample, delays or postpones the occurrence of the disorder or condition in the treated sample relative to an untreated control sample, or delays the onset or reduces the severity of one or more symptoms of the disorder or condition relative to the untreated control sample.

As used herein, "α-ketoglutarate" or AKG comprises derivatives of α-ketoglutarate (e.g., the derivatives set forth in MacKenzie, et al. (2007) Mol Cell Biol 27(9):3282-3289)), analogues of α-ketoglutarate (e.g., phosphonate analogues (e.g., those recited in Bunik, et al. (2005) Biochemistry 44(31):10552-61), esters of α-ketoglutarate (e.g., dimethyl α-ketoglutarate and octyl α-ketoglutarate), and various species specific analogues, e.g., human α-ketoglutarate, porcine α-ketoglutarate, murine α-ketoglutarate, bovine α-ketoglutarate, and the like.

### Compositions

Described herein are methods and compositions for pharmacological treatment of lifespan, healthspan, and aging-related disease. Further disclosed herein, in some aspects, are methods and compositions for delaying onset, or delaying progression of a disorder, reversing an age-related phenotype, extending healthspan, compressing morbidity, and reducing formation of senescent cells

In certain aspects, the disclosure provides compositions that comprise two or more compounds (individually referred to as the "active ingredients") that are available for human consumption without FDA approval or generally recognized as safe (GRAS). In certain aspects, the disclosure provides compositions that comprise berberine, α-ketoglutarate (AKG), and vitamin A. In certain aspects, the disclosure provides compositions that comprise berberine, α-ketoglutarate (AKG), and retinoic acid (RA). In certain aspects, the disclosure provides compositions that comprise berberine, α-ketoglutarate (AKG), and retinyl palmitate (RP). In certain aspects, the disclosure provides compositions that comprise AKG and vitamin A. In certain aspects, the disclosure provides compositions that comprise AKG and retinoic acid (RA). In certain aspects, the disclosure provides compositions that comprise AKG and retinyl palmitate (RP). In certain aspects, the disclosure provides compositions that comprise AKG and vitamin D. In certain aspects, the disclosure provides compositions that comprise AKG and vitamin D3. In certain aspects, the disclosure provides compositions that comprise AKG and nicotinamide mononucleotide (NMN). In some instances, the AKG in the compositions is a salt of AKG. In some instances, the AKG provided in the compositions is a calcium salt of AKG (Ca-AKG). In certain aspects, the disclosure further provides compositions that comprise a compound (e.g. AKG) that is available for human consumption without FDA approval or generally recognized as safe (GRAS). In some embodiments, AKG is provided as a salt. In some embodiments, AKG is provided as a calcium salt (Ca-AKG). Such compounds may be so classified because they are: a) present in the FDA SCOGS database and are generally recognized as safe by the U.S. Food and Drug Administration; or b) are derived from plants (for e.g. fruits, vegetables, herbs) present in traditional diets and so are recognized by the scientific community as safe for consumption. In some embodiments GRAS compounds are those compounds which are available for human consumption without FDA approval.

The active ingredients can be in free-acid or free-base forms, or in a pharmaceutically acceptable salt forms. In addition, the methods and pharmaceutical compositions described herein include the use of N-oxides, crystalline forms (also known as polymorphs), as well as active metabolites of these compounds having the same type of activity. All tautomers of the compounds described herein are included within the scope of the compounds presented herein. Additionally, the compounds described herein encompass unsolvated as well as solvated forms with pharmaceutically acceptable solvents such as water, ethanol, and the like. The solvated forms of the compounds presented herein are also considered to be disclosed herein.

Disclosed herein, in some embodiments, are compositions comprising an aging process modulator. In some embodiments, the compound in the composition (e.g. AKG or Ca-AKG) modulates two or more aging processes. In some embodiments, the aging process modulator is an agent which modulates the mTOR pathway, the insulin/insulin-like growth factor (IGF) pathway, inflammatory pathways, senescence pathways, the dietary restriction pathway, and mitochondrial bioenergetics. In some embodiments, the aging process modulator modulates aging processes associated with caloric restriction. In some embodiments, the aging process modulator modulates aging processes associated with fasting. In some embodiments, the aging process modulator modulates mitochondrial biosynthesis, mitochondrial repair, and mitophagy. In some embodiments, the aging process modulator modulates protein homeostasis and oxidative damage. In some embodiments, the aging process modulator enhances adult stem cell function. In some embodiments, the aging process modulator blocks the effects of senescent cells.

Further described herein are compositions comprising at least two aging process modulators. In some embodiments, the same compound modulates two or more aging processes. In some embodiments, the aging process modulators are agents which modulate the mTOR pathway, the insulin/insulin-like growth factor (IGF) pathway, inflammatory pathways, senescence pathways, the dietary restriction pathway, and mitochondrial bioenergetics. In some embodiments, the aging process modulators modulate aging processes associated with caloric restriction. In some embodiments, the aging process modulators modulate aging processes associated with fasting. In some embodiments the aging process modulators modulate mitochondrial biosynthesis, mitochondrial repair, and mitophagy. In some embodiments, the aging process modulators modulate protein homeostasis and oxidative damage. In some embodiments, the aging process modulators enhance adult stem cell function. In some embodiments, the aging process modulators block the effects of senescent cells.

In some embodiments, the composition comprises a therapeutically effective amount of an agent that modulates mTOR signaling and a therapeutically effective amount of an agent that modulates IGF signaling. In some embodiments, the composition comprises a therapeutically effective amount of an agent that modulates mTOR signaling and a therapeutically effective amount of an agent that modulates AMPK signaling. In some embodiments, the composition comprises a therapeutically effective amount of an agent that modulates mTOR signaling and a therapeutically effective amount of an agent that modulates mitochondrial bioenergetics.

In some embodiments, the composition comprises at least three aging process modulators. In some embodiments, the composition comprises a therapeutically effective amount of an agent that modulates mTOR signaling, a therapeutically effective amount of an agent that modulates IGF signaling, and a therapeutically effective amount of an agent that modulates AMPK signaling. In some embodiments, the composition comprises a therapeutically effective amount of an agent that modulates mTOR, a therapeutically effective amount of an agent that modulates IGF signaling, and a therapeutically effective amount of an agent that modulates mitochondrial bioenergetics. In some embodiments, the composition comprises a therapeutically effective amount of an agent that modulates mTOR signaling, a therapeutically effective amount of an agent that modulates AMPK signaling, and a therapeutically effective amount of an agent that modulates mitochondrial bioenergetics.

### Active agents

Examples of active agents that act in the mTOR pathway include, but are not limited to, vitamin A (e.g. as an activator, see Chen et al. Stem Cells 2010:57-63(2010) and Berry et al. Proc Natl Acad Sci U S A 108:4340-5(2011) ), berberine (e.g. as an inhibitor, see for Ming et al. PLoS One 9:e114573(2014) and Liu et al. Mol Cell Endocrinol 317:148-53(2010)), α-ketoglutarate (e.g. as an inhibitor, see Chin et al. Nature 510:397-401(2014)), resveratrol (e.g. as an inhibitor, see Park et al. Sci Rep. 6:21772(2016)), caffeine (e.g . as an inhibitor, see Saiki et al. Autophagy 7:176-87(2011)), aspirin (e.g. as an inhibitor, see Gao et al. J Biol Chem 278:24944-50(2003)), 5-aminoimidazole-4-carboxamide-1β-d-ribonucleoside (AICAR, e.g. as an inhibitor, see Bolster et al. J Biol Chem 277:23977-23980(2002)), and rapamycin.

Examples of active agents that act in the IGF pathway include, but are not limited to, vitamin A (e.g. as an activator, see Chen et al. Stem Cells 2010:57-63(2010) and Berry et al. Proc Natl Acad Sci U S A 108:4340-5(2011) ), aspirin (e.g. as an inhibitor, see Gao et al. J Biol Chem 278:24944-50(2003)), 2-deoxyglucose (e.g. as an activator, see Zhong et al. J Biol Chem 284: 23225-23233(2009)), apigenin (e.g. as an inhibitor, see Shukla et al. Pharm Res 29:1506-17(2012)), and berberine (e.g. as an inhibitor, see for Ming et al. PLoS One 9:e114573(2014) and Liu et al. Mol Cell Endocrinol 317:148-53(2010)).

Examples of active agents that act in the AMPK pathway include, but are not limited to, α-ketoglutarate (e.g. as an activator, see Chin et al. Nature 510:397-401(2014)), metformin (e.g. as an activator, see Onken et al. PLoS One 5: e8758(2010)), rosaglitazone (e.g. as an activator, see LeBrasseur et al. Am J Physiol Endocrinol Metab 291:E175-E181(2006)), quercetin (e.g. as an activator, see LeBrasseur et al. Am J Physiol Endocrinol Metab 291:E175-E181(2006)), and AICAR (e.g. as an activator, see Bolster et al. J Biol Chem 277:23977-23980(2002)).

Examples of active agents that act on the inflammation pathway include, but are not limited to, berberine (e.g. as an anti-inflammatory, see Kuo et al. Cancer Lett 203:127-37(2004)) and α-ketoglutarate (e.g. as an anti-inflammatory, see Wang et al. Amino Acids 47:1309-18(2015) and Hou et al. Amino Acids 39:555-64(2010)), and aminoguanidine (e.g. as an anti-inflammatory, see Calixto et al. PloS One 8:e76786(2013).

Examples of active agents that act in the dietary restriction pathway include, but are not limited to, berberine (e.g. as a SIRT1 activator, see Gomes et al. Biochim Biophys Acta 1822:185-95(2012)) and alpha-ketoglutarate (e.g. as a dietary-restriction mimetic, see Chin et al. Nature 510:397-401(2014)), reservatrol, metformin, and rapamycin.

Examples of active agents that act on mitochondrial bioenergetics include, but are not limited to, vitamin A (e.g. as an activator of mitochondrial oxygen consumption, see Actin-perez et al. FASEB J 24:627-636(2010)), vitamin D3 (e.g. as an inhibitor of mitochondrial permeability transition pore opening, see Uberti et al. J Clin Endocrinol Metab 99:1367-1374(2014)), nicotinamide mononucleotide (NMN, e.g. as an activator of mitochondrial respiration, see Long et al. BMC Neurology 15:19(2015)), quercetin (e.g. as a scavenger of O2 radicals, see Gibellini et al. Evid Based Complement Alternat Med. 2015:527209(2015)), resveratrol (e.g. as a scavenger of H2O2, see Gibellini et al. Evid Based Complement Alternat Med. 2015:527209(2015)), and curcumin (e.g. via upregulation of antioxidant enzymes, see Gibellini et al. Evid Based Complement Alternat Med. 2015:527209(2015)), and metformin.

**In** some aspects, the combination of active agents comprises two or more active agents selected from the group consisting of: alpha-ketoglutarate, berberine, vitamin A, vitamin D and nicotinamide mononucleotide (NMN). In certain aspects, the disclosure provides compositions that comprise berberine, α-ketoglutarate (AKG), and vitamin A. In certain aspects, the disclosure provides compositions that comprise berberine, α-ketoglutarate (AKG), and retinoic acid (RA). In certain aspects, the disclosure provides compositions that comprise berberine, α-ketoglutarate (AKG), and retinyl palmitate (RP). In certain aspects, the disclosure provides compositions that comprise AKG and vitamin A. In certain aspects, the disclosure provides compositions that comprise AKG and retinoic acid (RA). In certain aspects, the disclosure provides compositions that comprise AKG and retinyl palmitate (RP). In certain aspects, the disclosure provides compositions that comprise AKG and vitamin D. In certain aspects, the disclosure provides compositions that comprise AKG and vitamin D3. In certain aspects, the disclosure provides compositions that comprise AKG and nicotinamide mononucleotide (NMN). In some instances, the AKG in the compositions is a salt of AKG. In some instances, the AKG provided in the compositions is a calcium salt of AKG (Ca-AKG). In specific embodiments, the two or more compounds comprise alpha-ketoglutarate and berberine. In yet other embodiments, the two or more compounds comprise berberine and vitamin A. In some aspects, disclosure further provides compositions comprising an active agent selected from the group consisting of: alpha-ketoglutarate, berberine, vitamin A, vitamin D and nicotinamide mononucleotide (NMN). In certain aspects, the compound is AKG. In some embodiments, AKG is provided as a salt. In some embodiments, AKG is provided as a calcium salt (Ca-AKG).

Any of the compositions according to the disclosure herein can further comprise a pharmaceutically acceptable excipient.

In some embodiments, compositions according to the invention comprise two or more compounds selected from the group consisting of alpha-ketoglutarate, berberine, vitamin A, vitamin D and nicotinamide mononucleotide (NMN) wherein the composition does not comprise a pharmaceutically acceptable excipient. In some embodiments, the composition comprises a pharmaceutically acceptable excipient. In some embodiments, compositions according to the disclosure comprise a compound selected from the group consisting of alpha-ketoglutarate, berberine, vitamin A, vitamin D and nicotinamide mononucleotide (NMN) wherein the composition does not comprise a pharmaceutically acceptable excipient. In some embodiments, the composition comprises a pharmaceutically acceptable excipient. In certain aspects, the compound is AKG. In some embodiments, AKG is provided as a salt. In some embodiments, AKG is provided as a calcium salt (Ca-AKG).

Active agents according to the invention have combinatorial effects on the phenotypes treated or evaluated herein. In some embodiments, the active agents have additive effects on lifespan, healthspan, frailty and components thereof, or age-related disease. In some embodiments, the active agents have synergistic effects on lifespan, healthspan, frailty and components thereof, or age-related disease. In some embodiments the active agents do not have negative synergistic effects on lifespan, healthspan, frailty and components thereof, or age-related disease.

### Berberine

In some embodiments, the compositions disclosed herein comprise berberine. In some embodiments, the compositions disclosed herein comprise berberine in combination with one or more of the active agents disclosed herein (e.g. AKG or Ca-AKG, vitamin A or retinoic acid or retinyl palmitate, vitamin D, NMN).

Berberine (also known as umbellatine or 5,6-dihydro-9,10-dimethoxybenzo[g]-1,3-benzodioxolo[5,6-a]quinolizinium, **Formula I**) is a benzoisoquinoline alkaloid found in such plants as *Berberis vulgaris* (barberry), *Berberis aristata* (tree turmeric), *Mahonia aquifolium* (Oregon grape), *Hydrastis canadensis* (goldenseal), *Xanthorhiza simplicissima* (yellowroot), *Phellodendron amurense* (Amur cork tree), *Coptis chinensis* (Chinese goldthread), *Tinospora cordifolia, Argemone mexicana* (prickly poppy), and *Eschscholzia californica* (Californian poppy).

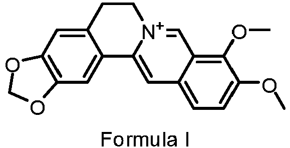

In some embodiments, berberine is provided in a pure synthetic form. Pure synthetic forms of berberine are available from commercial providers (e.g. Sigma-Aldrich). Methods for synthesis of berberine and related benzoisoquinoline analogs from commercially available derivatives of phenylethylamine and benzaldehyde have been described and can be found, for example in Wang et al. Bioorganic & Medicinal Chemistry 20: 6552-6558 (2012).

In other embodiments, berberine is provided in a form extracted from any of the plant species described above. Methods for high-purity extraction of berberine from plant material are known and can be found, for example in Liu et al. Journal of Pharmaceutical and Biomedical Analysis 41:1056-1060(2006) which describes extraction from *Coptis chinensis Franch* rhizomes using supercritical fluid extraction.

Both embodiments described for provision of berberine are capable of yielding berberine of high purity. In some embodiments, the purity is in the range of 80% to 99.9%, or in the range of 85% to 99.9%, or in the range of 90% to 99.9%, or in the range of 95% to 99.9%. In some embodiments, the purity is in the range of about 80% to about 99.9%, or in the range of about 85% to about 99.9%, or in the range of about 90% to about 99.9%, or in the range of about 95% to about 99.9%. In some embodiments, the purity is more than 80%, or more than 85%, or more than 90%, or more than 91%, or more than 92%, or more than 93%, or more than 94%, or more than 95%, or more than 96%, or more than 97%, or more than 98%, or more than 99%, or more than 99.5%, or more than 99.9%.

In some embodiments, berberine is provided as the chloride salt. In other embodiments, berberine is provided as a salt with other anions described in the U.S. FDA *Orange Book.* Such anions include acetate, benzoate, besylate, bromide, camsylate, carbonate, citrate, edisylate, estolate, fumarate, gluceptate, gluconate, glucuronate, hippurate, iodide, isethionate, lactate, lactobionate, malate, maleate, mesylate, methylsulfate, napsylate, nitrate, oxalate, pamoate, phosphate, stearate, succinate, sulfate, tartrate/bitartrate, and tosylate.

In some embodiments, a therapeutically effective amount of berberine is from about 1,000 mg to about 5,000 mg. In some embodiments, the therapeutically effective amount is at least 1,000, at least 1,500, at least 2,000, at least 2,500, at least 3,000, at least 3,500 or at least 4,000 mg. In other embodiments, the therapeutically effective amount is less than 5,000, less than 5,500, less than 4,000, less than 3,500, less than 3,000, or less than 2,000 mg.

### Alpha-Ketoglutarate (AKG)

In some embodiments, the compositions disclosed herein comprise AKG. In some embodiments, the compositions disclosed herein comprise AKG in combination with one or more of the active agents disclosed herein (e.g. berberine, vitamin A or retinoic acid or retinyl palmitate, vitamin D, NMN).

α-ketoglutarate (**Formula 2,** is also known as 2-oxopentanedioic acid, 2-ketoglutaric acid, 2-oxoglutaric acid, and oxoglutaric acid. At physiological pH, α-ketoglutarate exists in the deprotonated form depicted as **Formula 3**). α-ketoglutarate is an intermediate in the Krebs cycle of eukaryotic organisms, and is biosynthesized from isocitrate (in the Krebs cycle process) or L-glutamate (via alanine transaminase) in such organisms. Both α-ketoglutarate and its corresponding salts are commercially available, either via preparation from fermentation cultures (for example see US 2,776,926) or chemical synthesis from closely related compounds.

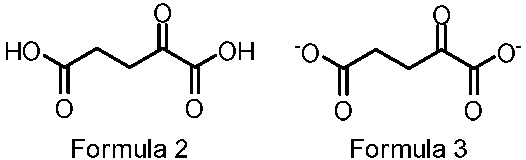

Consistent with its role in energy generation via the Krebs cycle, α-ketoglutarate is an important regulator of bioenergetics in cells and is implicated as an inhibitor of ATP synthase subunit β and an indirect inhibitor of the kinase mTOR, a consequence of partial inhibition of the mitochondrial electron transport chain.

In some embodiments, α-ketoglutarate is provided as the free acid (α-ketoglutaric acid). In other embodiments, α-ketoglutarate is provided as a monosodium salt, a disodium salt, or a monopotassium salt. In further embodiments, disodium salts of α-ketoglutarate are provided as anhydrous salts, monohydrates, or dihydrates. In yet further embodiments, α-ketoglutarate is provided as a mono- or divalent salt with other cations described in the U.S. FDA *Orange Book.* Such cations include calcium, diolamine, lithium, lysine, magnesium, meglumine, olamine, tromethamine, and zinc.

Further disclosed herein, in certain aspects, are compositions that comprise α-ketoglutarate salt. In some embodiments, α-ketoglutarate is provided as a calcium salt. In some embodiments, calcium α-ketoglutarate can be a hydrate calcium α-ketoglutarate. In some embodiments, calcium α-ketoglutarate can be a mono-hydrate calcium α-ketoglutarate. In some embodiments, calcium α-ketoglutarate can be hemi-hydrate calcium α-ketoglutarate. In some embodiments, calcium α-ketoglutarate can be anhydrous calcium α-ketoglutarate.

In some embodiments, a therapeutically effective amount of α-ketoglutarate is from about 350 mg to about 2000 mg. In some embodiments, the therapeutically effective amount is at least 350, at least 400, at least 450, at least 500, at least 550, at least 600, at least 650, at least 700, at least 750, at least 800, at least 850, at least 900, at least 950, at least 1000, at least 1050, at least 1100, at least 1150, at least 1200, at least 1250, at least 1300, at least 1350, at least 1400, at least 1450, at least 1500, at least 1550, at least 1600, at least 1650, at least 1700, at least 1750, at least 1800, at least 1850, at least 1900, or at least 1950 mg. In other embodiments, the therapeutically effective amount is less than 2000, less than 1950, less than 1900, less than 1850, less than 1800, less than 1750, less than 1700, less than 1650, less than 1600, less than 1550, less than 1500, less than 1450, less than 1400, less than 1350, less than 1300, less than 1250, less than 1200, less than 1150, less than 1100, less than 1050, less than 1000, less than 950, less than 900, less than 850, less than 800, less than 750, less than 700, less than 650, less than 600, less than 550, less than 500, less than 450, or less than 400 mg.

### Vitamin A

In some embodiments, the compositions disclosed herein comprise vitamin A. In some embodiments, the compositions disclosed herein comprise vitamin A in combination with one or more of the active agents disclosed herein (e.g. berberine, AKG or Ca-AKG, vitamin D, NMN). As disclosed herein, vitamin A can also be provided as retinoic acid (RA) or retinyl palmitate (RP).

Vitamin A is an essential organic nutrient in animals, which is converted between a variety of chemical forms in cells. These forms include retinol esters **(Formula 4),** retinol **(Formula 5),** retinal **(Formula 6),** and retinoic acid **(Formula 7).** As retinal, vitamin A is essential for the formation of rhodopsin in the eye. As retinoic acid, vitamin A plays an important role as a hormone-like growth factor for epithelial and immune cells. Relevant to age-related indications, current studies indicate that vitamin A in the form of retinoic acid activates AMPK and stimulates glucose uptake in cells (see for example Yun et al. J Biol Chem 283:33969-33974 (2008)), thus acting as an anti-diabetic agent.

Vitamin A is also present in plants in a variety of pro-vitamin A forms that are converted into retinol in animals. Such forms include alpha-carotene **(Formula 8),** beta-carotene **(Formula 9),** and gamma-carotene **(Formula 10).** Because of the diverse forms vitamin A is present in, nutritional amounts of vitamin A are defined in International Units (IU), which is agnostic to the chemical form of vitamin A. For example, 1 IU is the biological equivalent of 0.3 mg retinol but 0.6 mg beta-carotene. Information regarding the IU equivalency of different forms of vitamin A is known in the art.

In some embodiments, vitamin A is provided as a retinol ester, retinol, retinal, retinoic acid, or retinoic acid salts with pharmaceutically acceptable cations as described previously. In other embodiments, vitamin A is provided as the alpha-, beta-, or gamma-carotene forms represented in formulas 8-10.

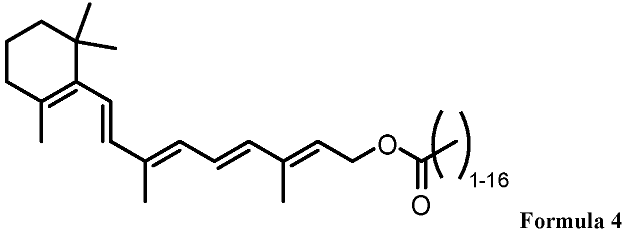

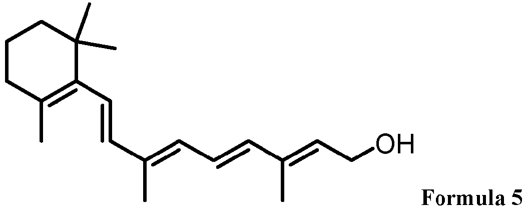

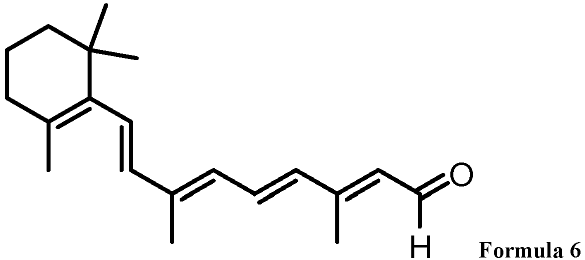

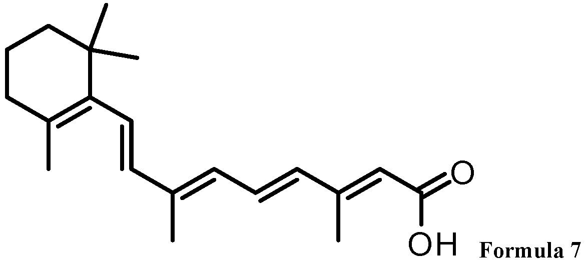

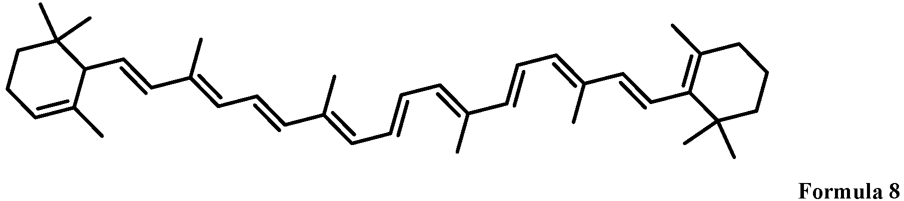

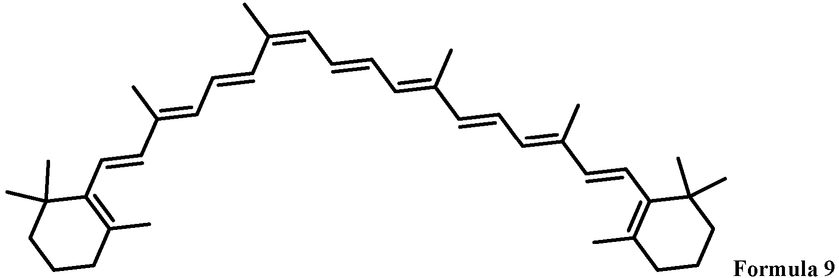

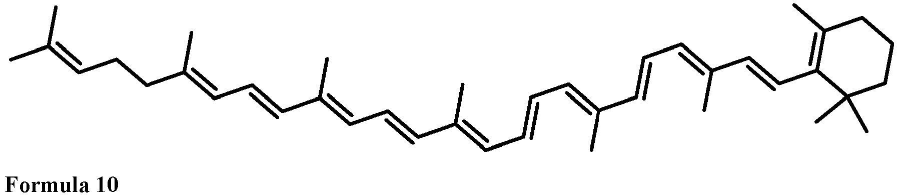

A therapeutically effective amount of vitamin A or any of its derivatives herein, such as **Formulas 4-10** is from about 10,000 IU to about 20,000 IU. In some embodiments, the therapeutically effective amount is at least 10,000, at least 12,500, at least 15,000, or at least 17,500 IU. In other embodiments, the therapeutically effective amount is less than 20,000, less than 17,500, less than 15,000, or less than 12,500 IU.

In some embodiments optimal doses of vitamin A are determined using experimental model systems (e.g. *C*. *elegans, D. melanogaster,* murine models). In other embodiments optimal doses of berberine are determined using clinical trials. The optimal dose may depend upon the body mass, weight, or blood volume of the subject.

### Methods of Use

Described herein are methods for treating, delaying onset, or delaying progression of a disorder in a subject in need thereof by administering the active agents compounds or compositions thereof disclosed herein. In some embodiments, the present disclosure provides for methods for extending lifespan or survival, delaying onset, or delaying progression of a disorder, reversing an age-related phenotype, extending healthspan, compressing morbidity, and reducing formation of senescent cells in a subject in need thereof by administering compositions comprising the active agents of the disclosure.

In some aspects, the composition comprises two or more active agents selected from the group consisting of: alpha-ketoglutarate, berberine, vitamin A, vitamin D and nicotinamide mononucleotide (NMN). In certain aspects, the composition comprises berberine, α-ketoglutarate (AKG), and vitamin A. In certain aspects, the composition comprises berberine, α-ketoglutarate (AKG), and retinoic acid (RA). In certain aspects, the composition comprises berberine, α-ketoglutarate (AKG), and retinyl palmitate (RP). In certain aspects, the composition comprises AKG and vitamin A. In certain aspects, the composition comprises AKG and retinoic acid (RA). In certain aspects, the composition comprises AKG and retinyl palmitate (RP). In certain aspects, the composition comprises AKG and vitamin D. In certain aspects, the composition comprises AKG and vitamin D3. In certain aspects, the composition comprises AKG and nicotinamide mononucleotide (NMN). In some instances, the AKG in the compositions is a salt of AKG. In some instances, the AKG provided in the compositions is a calcium salt of AKG (Ca-AKG). In some embodiments, the two or more compounds comprise alpha-ketoglutarate and berberine. In yet other embodiments, the two or more compounds comprise berberine and vitamin A. In some aspects, disclosure further provides compositions comprising an active agent selected from the group consisting of: alpha-ketoglutarate, berberine, vitamin A and vitamin D. In certain aspects, the compound is AKG. In some embodiments, AKG is provided as a salt. In some embodiments, AKG is provided as a calcium salt (Ca-AKG).

In some aspects, the present disclosure provides for methods for delaying onset, or delaying progression of a disorder, reversing an age-related phenotype, extending healthspan, compressing morbidity, and reducing formation of senescent cells in a subject in need thereof by administering compositions comprising calcium salt of α-ketoglutarate. In some embodiments, calcium α-ketoglutarate can be a hydrate calcium α-ketoglutarate. In some embodiments, calcium α-ketoglutarate can be a mono-hydrate calcium α-ketoglutarate. In some embodiments, calcium α-ketoglutarate can be hemi-hydrate calcium α-ketoglutarate. In some embodiments, calcium α-ketoglutarate can be anhydrous calcium α-ketoglutarate. In other aspects, the present disclosure provides for methods for delaying onset, or delaying progression of a disorder, reversing an age-related phenotype, extending healthspan and compressing morbidity in a subject in need thereof by administering compositions consisting essentially of calcium salt of α-ketoglutarate. In some embodiments, calcium α-ketoglutarate can be a hydrate calcium α-ketoglutarate. In some embodiments, calcium α-ketoglutarate can be a mono-hydrate calcium α-ketoglutarate. In some embodiments, calcium α-ketoglutarate can be hemi-hydrate calcium α-ketoglutarate. In some embodiments, calcium α-ketoglutarate can be anhydrous calcium α-ketoglutarate.

### Lifespan

In an aspect, the disclosure provides methods for extending lifespan by administering the active agents or compositions thereof described herein. In many embodiments, extension of lifespan is assessed in a convenient non-human test subject with a lifespan lower than that of humans. Examples of non-human animals used for assessment of lifespan-extension inverventions include *C*. *elegans, D. melanogaster,* and *M. musculus.* Use of the *C*. *elegans* platform to evaluate lifespan or healthspan interventions along with appropriate methods is exemplified in **Example 1.** Use of *D. melanogaster* or *M. musculus* to evaluate lifespan or healthspan interventions are found in, for e.g. Bauer et al. Proc Natl Acad Sci U S A. 101:12980-5 (2004) and Selman et al. FASEB J 22:807-18 (2008).

In some embodiments, the disclosure provides methods for extending lifespan by administering the active agents or compositions thereof described herein. In some aspects, the composition comprises two or more active agents selected from the group consisting of: alpha-ketoglutarate, berberine, vitamin A, vitamin D and nicotinamide mononucleotide (NMN). In certain aspects, the composition comprises berberine, α-ketoglutarate (AKG), and vitamin A. In certain aspects, the composition comprises berberine, α-ketoglutarate (AKG), and retinoic acid (RA). In certain aspects, the composition comprises berberine, α-ketoglutarate (AKG), and retinyl palmitate (RP). In certain aspects, the composition comprises AKG and vitamin A. In certain aspects, the composition comprises AKG and retinoic acid (RA). In certain aspects, the composition comprises AKG and retinyl palmitate (RP). In certain aspects, the composition comprises AKG and vitamin D. In certain aspects, the composition comprises AKG and vitamin D3. In certain aspects, the composition comprises AKG and nicotinamide mononucleotide (NMN). In some instances, the AKG in the compositions is a salt of AKG. In some instances, the AKG provided in the compositions is a calcium salt of AKG (Ca-AKG). In some embodiments, the two or more compounds comprise alpha-ketoglutarate and berberine. In yet other embodiments, the two or more compounds comprise berberine and vitamin A. In some aspects, disclosure further provides compositions comprising an active agent selected from the group consisting of: alpha-ketoglutarate, berberine, vitamin A and vitamin D. In certain aspects, the compound is AKG. In some embodiments, AKG is provided as a salt. In some embodiments, AKG is provided as a calcium salt (Ca-AKG).

**In** certain aspects, the disclosure provides methods extending lifespan in a subject in need thereof comprising administering to the subject a composition comprising a calcium salt of alpha-ketoglutarate. In another aspect, the disclosure provides methods for extending lifespan in a subject in need thereof comprising administering to the subject a composition consisting essentially of a calcium salt of alpha-ketoglutarate. In some embodiments, calcium α-ketoglutarate can be a hydrate calcium α-ketoglutarate. In some embodiments, calcium α-ketoglutarate can be a mono-hydrate calcium α-ketoglutarate. In some embodiments, calcium α-ketoglutarate can be hemi-hydrate calcium α-ketoglutarate. In some embodiments, calcium α-ketoglutarate can be anhydrous calcium α-ketoglutarate.

In some embodiments, the Ca-AKG is administered at a dose of at least 350 mg and no greater than 2000 mg. In some instances, the Ca-AKG is administered at a dose of about 350 mg to about 2000 mg. In some instances, the Ca-AKG is administered at a dose of about 400 mg to about 2000 mg. In some instances, the Ca-AKG is administered at a dose of about 450 mg to about 2000 mg. In some instances, the Ca-AKG is administered at a dose of about 500 mg to about 2000 mg. In some instances, the Ca-AKG is administered at a dose of about 550 mg to about 2000 mg. In some instances, the Ca-AKG is administered at a dose of about 600 mg to about 2000 mg. In some instances, the Ca-AKG is administered at a dose of about 650 mg to about 2000 mg. In some instances, the Ca-AKG is administered at a dose of about 700 mg to about 2000 mg. In some instances, the Ca-AKG is administered at a dose of about 800 mg to about 2000 mg. In some instances, the Ca-AKG is administered at a dose of about 900 mg to about 2000 mg. In some instances, the Ca-AKG is administered at a dose of about 1000 mg to about 2000 mg. In some instances, the Ca-AKG is administered at a dose of about 1100 mg to about 2000 mg. In some instances, the Ca-AKG is administered at a dose of about 1200 mg to about 2000 mg. In some instances, the Ca-AKG is administered at a dose of about 1300 mg to about 2000 mg. In some instances, the Ca-AKG is administered at a dose of about 1400 mg to about 2000 mg. In some instances, the Ca-AKG is administered at a dose of about 1500 mg to about 2000 mg. In some instances, the Ca-AKG is administered at a dose of about 1600 mg to about 2000 mg. In some instances, the Ca-AKG is administered at a dose of about 1700 mg to about 2000 mg. In some instances, the Ca-AKG is administered at a dose of about 1800 mg to about 2000 mg. In some instances, the Ca-AKG is administered at a dose of about 1900 mg to about 2000 mg. In some instances, the Ca-AKG is administered at a dose of about 350 mg to about 1900 mg. In some instances, the Ca-AKG is administered at a dose of about 350 mg to about 1800 mg. In some instances, the Ca-AKG is administered at a dose of about 350 mg to about 1700 mg. In some instances, the Ca-AKG is administered at a dose of about 350 mg to about 1600 mg. In some instances, the Ca-AKG is administered at a dose of about 350 mg to about 1500 mg. In some instances, the Ca-AKG is administered at a dose of about 350 mg to about 1400 mg. In some instances, the Ca-AKG is administered at a dose of about 350 mg to about 1300 mg. In some instances, the Ca-AKG is administered at a dose of about 350 mg to about 1200 mg. In some instances, the Ca-AKG is administered at a dose of about 350 mg to about 1100 mg. In some instances, the Ca-AKG is administered at a dose of about 350 mg to about 1000 mg. In some instances, the Ca-AKG is administered at a dose of about 350 mg to about 900 mg. In some instances, the Ca-AKG is administered at a dose of about 350 mg to about 800 mg. In some instances, the Ca-AKG is administered at a dose of about 350 mg to about 700 mg. In some instances, the Ca-AKG is administered at a dose of about 350 mg to about 650 mg. In some instances, the Ca-AKG is administered at a dose of about 350 mg to about 600 mg. In some instances, the Ca-AKG is administered at a dose of about 350 mg to about 550 mg. In some instances, the Ca-AKG is administered at a dose of about 350 mg to about 500 mg. In some instances, the Ca-AKG is administered at a dose of about 350 mg to about 450 mg. In some instances, the Ca-AKG is administered at a dose of about 350 mg to about 400 mg. In some instances, the Ca-AKG is administered at a dose of at least about 350 mg. In some instances, the Ca-AKG is administered at a dose of at least about 400 mg. In some instances, the Ca-AKG is administered at a dose of at least about 450 mg. In some instances, the Ca-AKG is administered at a dose of at least about 500 mg. In some instances, the Ca-AKG is administered at a dose of at least about 550 mg. In some instances, the Ca-AKG is administered at a dose of at least about 600 mg. In some instances, the Ca-AKG is administered at a dose of at least about 650 mg. In some instances, the Ca-AKG is administered at a dose of at least about 700 mg. In some instances, the Ca-AKG is administered at a dose of at least about 750 mg. In some instances, the Ca-AKG is administered at a dose of at least about 800 mg. In some instances, the Ca-AKG is administered at a dose of at least about 900 mg. In some instances, the Ca-AKG is administered at a dose of at least about 1000 mg. In some instances, the Ca-AKG is administered at a dose of at least about 1100 mg. In some instances, the Ca-AKG is administered at a dose of at least about 1200 mg. In some instances, the Ca-AKG is administered at a dose of at least about 1300 mg. In some instances, the Ca-AKG is administered at a dose of at least about 1400 mg. In some instances, the Ca-AKG is administered at a dose of at least about 1500 mg. In some instances, the Ca-AKG is administered at a dose of at least about 1600 mg. In some instances, the Ca-AKG is administered at a dose of at least about 1700 mg. In some instances, the Ca-AKG is administered at a dose of at least about 1800 mg. In some instances, the Ca-AKG is administered at a dose of at least about 1900 mg. In some instances, the Ca-AKG is administered at a dose of at least about 2000 mg.

In some embodiments, the hydrate Ca-AKG is administered at a dose of at least 350 mg and no greater than 2000 mg. In some instances, the hydrate Ca-AKG is administered at a dose of about 350 mg to about 2000 mg. In some instances, the hydrate Ca-AKG is administered at a dose of about 400 mg to about 2000 mg. In some instances, the hydrate Ca-AKG is administered at a dose of about 450 mg to about 2000 mg. In some instances, the hydrate Ca-AKG is administered at a dose of about 500 mg to about 2000 mg. In some instances, the hydrate Ca-AKG is administered at a dose of about 550 mg to about 2000 mg. In some instances, the hydrate Ca-AKG is administered at a dose of about 600 mg to about 2000 mg. In some instances, the hydrate Ca-AKG is administered at a dose of about 650 mg to about 2000 mg. In some instances, the hydrate Ca-AKG is administered at a dose of about 700 mg to about 2000 mg. In some instances, the hydrate Ca-AKG is administered at a dose of about 800 mg to about 2000 mg. In some instances, the hydrate Ca-AKG is administered at a dose of about 900 mg to about 2000 mg. In some instances, the hydrate Ca-AKG is administered at a dose of about 1000 mg to about 2000 mg. In some instances, the hydrate Ca-AKG is administered at a dose of about 1100 mg to about 2000 mg. In some instances, the hydrate Ca-AKG is administered at a dose of about 1200 mg to about 2000 mg. In some instances, the hydrate Ca-AKG is administered at a dose of about 1300 mg to about 2000 mg. In some instances, the hydrate Ca-AKG is administered at a dose of about 1400 mg to about 2000 mg. In some instances, the hydrate Ca-AKG is administered at a dose of about 1500 mg to about 2000 mg. In some instances, the hydrate Ca-AKG is administered at a dose of about 1600 mg to about 2000 mg. In some instances, the hydrate Ca-AKG is administered at a dose of about 1700 mg to about 2000 mg. In some instances, the hydrate Ca-AKG is administered at a dose of about 1800 mg to about 2000 mg. In some instances, the hydrate Ca-AKG is administered at a dose of about 1900 mg to about 2000 mg. In some instances, the hydrate Ca-AKG is administered at a dose of about 350 mg to about 1900 mg. In some instances, the hydrate Ca-AKG is administered at a dose of about 350 mg to about 1800 mg. In some instances, the hydrate Ca-AKG is administered at a dose of about 350 mg to about 1700 mg. In some instances, the hydrate Ca-AKG is administered at a dose of about 350 mg to about 1600 mg. In some instances, the hydrate Ca-AKG is administered at a dose of about 350 mg to about 1500 mg. In some instances, the hydrate Ca-AKG is administered at a dose of about 350 mg to about 1400 mg. In some instances, the hydrate Ca-AKG is administered at a dose of about 350 mg to about 1300 mg. In some instances, the hydrate Ca-AKG is administered at a dose of about 350 mg to about 1200 mg. In some instances, the hydrate Ca-AKG is administered at a dose of about 350 mg to about 1100 mg. In some instances, the hydrate Ca-AKG is administered at a dose of about 350 mg to about 1000 mg. In some instances, the hydrate Ca-AKG is administered at a dose of about 350 mg to about 900 mg. In some instances, the hydrate Ca-AKG is administered at a dose of about 350 mg to about 800 mg. In some instances, the hydrate Ca-AKG is administered at a dose of about 350 mg to about 700 mg. In some instances, the hydrate Ca-AKG is administered at a dose of about 350 mg to about 650 mg. In some instances, the hydrate Ca-AKG is administered at a dose of about 350 mg to about 600 mg. In some instances, the hydrate Ca-AKG is administered at a dose of about 350 mg to about 550 mg. In some instances, the hydrate Ca-AKG is administered at a dose of about 350 mg to about 500 mg. In some instances, the hydrate Ca-AKG is administered at a dose of about 350 mg to about 450 mg. In some instances, the hydrate Ca-AKG is administered at a dose of about 350 mg to about 400 mg. In some instances, the hydrate Ca-AKG is administered at a dose of at least about 350 mg. In some instances, the hydrate Ca-AKG is administered at a dose of at least about 400 mg. In some instances, the hydrate Ca-AKG is administered at a dose of at least about 450 mg. In some instances, the hydrate Ca-AKG is administered at a dose of at least about 500 mg. In some instances, the hydrate Ca-AKG is administered at a dose of at least about 550 mg. In some instances, the hydrate Ca-AKG is administered at a dose of at least about 600 mg. In some instances, the hydrate Ca-AKG is administered at a dose of at least about 650 mg. In some instances, the hydrate Ca-AKG is administered at a dose of at least about 700 mg. In some instances, the hydrate Ca-AKG is administered at a dose of at least about 750 mg. In some instances, the hydrate Ca-AKG is administered at a dose of at least about 800 mg. In some instances, the hydrate Ca-AKG is administered at a dose of at least about 900 mg. In some instances, the hydrate Ca-AKG is administered at a dose of at least about 1000 mg. In some instances, the hydrate Ca-AKG is administered at a dose of at least about 1100 mg. In some instances, the hydrate Ca-AKG is administered at a dose of at least about 1200 mg. In some instances, the hydrate Ca-AKG is administered at a dose of at least about 1300 mg. In some instances, the hydrate Ca-AKG is administered at a dose of at least about 1400 mg. In some instances, the hydrate Ca-AKG is administered at a dose of at least about 1500 mg. In some instances, the hydrate Ca-AKG is administered at a dose of at least about 1600 mg. In some instances, the hydrate Ca-AKG is administered at a dose of at least about 1700 mg. In some instances, the hydrate Ca-AKG is administered at a dose of at least about 1800 mg. In some instances, the hydrate Ca-AKG is administered at a dose of at least about 1900 mg. In some instances, the hydrate Ca-AKG is administered at a dose of at least about 2000 mg.

In some embodiments, the mono-hydrate Ca-AKG is administered at a dose of at least 350 mg and no greater than 2000 mg. In some instances, the mono-hydrate Ca-AKG is administered at a dose of about 350 mg to about 2000 mg. In some instances, the mono-hydrate Ca-AKG is administered at a dose of about 400 mg to about 2000 mg. In some instances, the mono-hydrate Ca-AKG is administered at a dose of about 450 mg to about 2000 mg. In some instances, the mono-hydrate Ca-AKG is administered at a dose of about 500 mg to about 2000 mg. In some instances, the mono-hydrate Ca-AKG is administered at a dose of about 550 mg to about 2000 mg. In some instances, the mono-hydrate Ca-AKG is administered at a dose of about 600 mg to about 2000 mg. In some instances, the mono-hydrate Ca-AKG is administered at a dose of about 650 mg to about 2000 mg. In some instances, the mono-hydrate Ca-AKG is administered at a dose of about 700 mg to about 2000 mg. In some instances, the mono-hydrate Ca-AKG is administered at a dose of about 800 mg to about 2000 mg. In some instances, the mono-hydrate Ca-AKG is administered at a dose of about 900 mg to about 2000 mg. In some instances, the mono-hydrate Ca-AKG is administered at a dose of about 1000 mg to about 2000 mg. In some instances, the mono-hydrate Ca-AKG is administered at a dose of about 1100 mg to about 2000 mg. In some instances, the mono-hydrate Ca-AKG is administered at a dose of about 1200 mg to about 2000 mg. In some instances, the mono-hydrate Ca-AKG is administered at a dose of about 1300 mg to about 2000 mg. In some instances, the mono-hydrate Ca-AKG is administered at a dose of about 1400 mg to about 2000 mg. In some instances, the mono-hydrate Ca-AKG is administered at a dose of about 1500 mg to about 2000 mg. In some instances, the mono-hydrate Ca-AKG is administered at a dose of about 1600 mg to about 2000 mg. In some instances, the mono-hydrate Ca-AKG is administered at a dose of about 1700 mg to about 2000 mg. In some instances, the mono-hydrate Ca-AKG is administered at a dose of about 1800 mg to about 2000 mg. In some instances, the mono-hydrate Ca-AKG is administered at a dose of about 1900 mg to about 2000 mg. In some instances, the mono-hydrate Ca-AKG is administered at a dose of about 350 mg to about 1900 mg. In some instances, the mono-hydrate Ca-AKG is administered at a dose of about 350 mg to about 1800 mg. In some instances, the mono-hydrate Ca-AKG is administered at a dose of about 350 mg to about 1700 mg. In some instances, the mono-hydrate Ca-AKG is administered at a dose of about 350 mg to about 1600 mg. In some instances, the mono-hydrate Ca-AKG is administered at a dose of about 350 mg to about 1500 mg. In some instances, the mono-hydrate Ca-AKG is administered at a dose of about 350 mg to about 1400 mg. In some instances, the mono-hydrate Ca-AKG is administered at a dose of about 350 mg to about 1300 mg. In some instances, the mono-hydrate Ca-AKG is administered at a dose of about 350 mg to about 1200 mg. In some instances, the mono-hydrate Ca-AKG is administered at a dose of about 350 mg to about 1100 mg. In some instances, the mono-hydrate Ca-AKG is administered at a dose of about 350 mg to about 1000 mg. In some instances, the mono-hydrate Ca-AKG is administered at a dose of about 350 mg to about 900 mg. In some instances, the mono-hydrate Ca-AKG is administered at a dose of about 350 mg to about 800 mg. In some instances, the mono-hydrate Ca-AKG is administered at a dose of about 350 mg to about 700 mg. In some instances, the mono-hydrate Ca-AKG is administered at a dose of about 350 mg to about 650 mg. In some instances, the mono-hydrate Ca-AKG is administered at a dose of about 350 mg to about 600 mg. In some instances, the mono-hydrate Ca-AKG is administered at a dose of about 350 mg to about 550 mg. In some instances, the mono-hydrate Ca-AKG is administered at a dose of about 350 mg to about 500 mg. In some instances, the mono-hydrate Ca-AKG is administered at a dose of about 350 mg to about 450 mg. In some instances, the mono-hydrate Ca-AKG is administered at a dose of about 350 mg to about 400 mg. In some instances, the mono-hydrate Ca-AKG is administered at a dose of at least about 350 mg. In some instances, the mono-hydrate Ca-AKG is administered at a dose of at least about 400 mg. In some instances, the mono-hydrate Ca-AKG is administered at a dose of at least about 450 mg. In some instances, the mono-hydrate Ca-AKG is administered at a dose of at least about 500 mg. In some instances, the mono-hydrate Ca-AKG is administered at a dose of at least about 550 mg. In some instances, the mono-hydrate Ca-AKG is administered at a dose of at least about 600 mg. In some instances, the mono-hydrate Ca-AKG is administered at a dose of at least about 650 mg. In some instances, the mono-hydrate Ca-AKG is administered at a dose of at least about 700 mg. In some instances, the mono-hydrate Ca-AKG is administered at a dose of at least about 750 mg. In some instances, the mono-hydrate Ca-AKG is administered at a dose of at least about 800 mg. In some instances, the mono-hydrate Ca-AKG is administered at a dose of at least about 900 mg. In some instances, the mono-hydrate Ca-AKG is administered at a dose of at least about 1000 mg. In some instances, the mono-hydrate Ca-AKG is administered at a dose of at least about 1100 mg. In some instances, the mono-hydrate Ca-AKG is administered at a dose of at least about 1200 mg. In some instances, the mono-hydrate Ca-AKG is administered at a dose of at least about 1300 mg. In some instances, the mono-hydrate Ca-AKG is administered at a dose of at least about 1400 mg. In some instances, the mono-hydrate Ca-AKG is administered at a dose of at least about 1500 mg. In some instances, the mono-hydrate Ca-AKG is administered at a dose of at least about 1600 mg. In some instances, the mono-hydrate Ca-AKG is administered at a dose of at least about 1700 mg. In some instances, the mono-hydrate Ca-AKG is administered at a dose of at least about 1800 mg. In some instances, the mono-hydrate Ca-AKG is administered at a dose of at least about 1900 mg. In some instances, the mono-hydrate Ca-AKG is administered at a dose of at least about 2000 mg.

In some embodiments, the hemi-hydrate Ca-AKG is administered at a dose of at least 350 mg and no greater than 2000 mg. In some instances, the hemi-hydrate Ca-AKG is administered at a dose of about 350 mg to about 2000 mg. In some instances, the hemi-hydrate Ca-AKG is administered at a dose of about 400 mg to about 2000 mg. In some instances, the hemi-hydrate Ca-AKG is administered at a dose of about 450 mg to about 2000 mg. In some instances, the hemi-hydrate Ca-AKG is administered at a dose of about 500 mg to about 2000 mg. In some instances, the hemi-hydrate Ca-AKG is administered at a dose of about 550 mg to about 2000 mg. In some instances, the hemi-hydrate Ca-AKG is administered at a dose of about 600 mg to about 2000 mg. In some instances, the hemi-hydrate Ca-AKG is administered at a dose of about 650 mg to about 2000 mg. In some instances, the hemi-hydrate Ca-AKG is administered at a dose of about 700 mg to about 2000 mg. In some instances, the hemi-hydrate Ca-AKG is administered at a dose of about 800 mg to about 2000 mg. In some instances, the hemi-hydrate Ca-AKG is administered at a dose of about 900 mg to about 2000 mg. In some instances, the hemi-hydrate Ca-AKG is administered at a dose of about 1000 mg to about 2000 mg. In some instances, the hemi-hydrate Ca-AKG is administered at a dose of about 1100 mg to about 2000 mg. In some instances, the hemi-hydrate Ca-AKG is administered at a dose of about 1200 mg to about 2000 mg. In some instances, the hemi-hydrate Ca-AKG is administered at a dose of about 1300 mg to about 2000 mg. In some instances, the hemi-hydrate Ca-AKG is administered at a dose of about 1400 mg to about 2000 mg. In some instances, the hemi-hydrate Ca-AKG is administered at a dose of about 1500 mg to about 2000 mg. In some instances, the hemi-hydrate Ca-AKG is administered at a dose of about 1600 mg to about 2000 mg. In some instances, the hemi-hydrate Ca-AKG is administered at a dose of about 1700 mg to about 2000 mg. In some instances, the hemi-hydrate Ca-AKG is administered at a dose of about 1800 mg to about 2000 mg. In some instances, the hemi-hydrate Ca-AKG is administered at a dose of about 1900 mg to about 2000 mg. In some instances, the hemi-hydrate Ca-AKG is administered at a dose of about 350 mg to about 1900 mg. In some instances, the hemi-hydrate Ca-AKG is administered at a dose of about 350 mg to about 1800 mg. In some instances, the hemi-hydrate Ca-AKG is administered at a dose of about 350 mg to about 1700 mg. In some instances, the hemi-hydrate Ca-AKG is administered at a dose of about 350 mg to about 1600 mg. In some instances, the hemi-hydrate Ca-AKG is administered at a dose of about 350 mg to about 1500 mg. In some instances, the hemi-hydrate Ca-AKG is administered at a dose of about 350 mg to about 1400 mg. In some instances, the hemi-hydrate Ca-AKG is administered at a dose of about 350 mg to about 1300 mg. In some instances, the hemi-hydrate Ca-AKG is administered at a dose of about 350 mg to about 1200 mg. In some instances, the hemi-hydrate Ca-AKG is administered at a dose of about 350 mg to about 1100 mg. In some instances, the hemi-hydrate Ca-AKG is administered at a dose of about 350 mg to about 1000 mg. In some instances, the hemi-hydrate Ca-AKG is administered at a dose of about 350 mg to about 900 mg. In some instances, the hemi-hydrate Ca-AKG is administered at a dose of about 350 mg to about 800 mg. In some instances, the hemi-hydrate Ca-AKG is administered at a dose of about 350 mg to about 700 mg. In some instances, the hemi-hydrate Ca-AKG is administered at a dose of about 350 mg to about 650 mg. In some instances, the hemi-hydrate Ca-AKG is administered at a dose of about 350 mg to about 600 mg. In some instances, the hemi-hydrate Ca-AKG is administered at a dose of about 350 mg to about 550 mg. In some instances, the hemi-hydrate Ca-AKG is administered at a dose of about 350 mg to about 500 mg. In some instances, the hemi-hydrate Ca-AKG is administered at a dose of about 350 mg to about 450 mg. In some instances, the hemi-hydrate Ca-AKG is administered at a dose of about 350 mg to about 400 mg. In some instances, the hemi-hydrate Ca-AKG is administered at a dose of at least about 350 mg. In some instances, the hemi-hydrate Ca-AKG is administered at a dose of at least about 400 mg. In some instances, the hemi-hydrate Ca-AKG is administered at a dose of at least about 450 mg. In some instances, the hemi-hydrate Ca-AKG is administered at a dose of at least about 500 mg. In some instances, the hemi-hydrate Ca-AKG is administered at a dose of at least about 550 mg. In some instances, the hemi-hydrate Ca-AKG is administered at a dose of at least about 600 mg. In some instances, the hemi-hydrate Ca-AKG is administered at a dose of at least about 650 mg. In some instances, the hemi-hydrate Ca-AKG is administered at a dose of at least about 700 mg. In some instances, the hemi-hydrate Ca-AKG is administered at a dose of at least about 750 mg. In some instances, the hemi-hydrate Ca-AKG is administered at a dose of at least about 800 mg. In some instances, the hemi-hydrate Ca-AKG is administered at a dose of at least about 900 mg. In some instances, the hemi-hydrate Ca-AKG is administered at a dose of at least about 1000 mg. In some instances, the hemi-hydrate Ca-AKG is administered at a dose of at least about 1100 mg. In some instances, the hemi-hydrate Ca-AKG is administered at a dose of at least about 1200 mg. In some instances, the hemi-hydrate Ca-AKG is administered at a dose of at least about 1300 mg. In some instances, the hemi-hydrate Ca-AKG is administered at a dose of at least about 1400 mg. In some instances, the hemi-hydrate Ca-AKG is administered at a dose of at least about 1500 mg. In some instances, the hemi-hydrate Ca-AKG is administered at a dose of at least about 1600 mg. In some instances, the hemi-hydrate Ca-AKG is administered at a dose of at least about 1700 mg. In some instances, the hemi-hydrate Ca-AKG is administered at a dose of at least about 1800 mg. In some instances, the hemi-hydrate Ca-AKG is administered at a dose of at least about 1900 mg. In some instances, the hemi-hydrate Ca-AKG is administered at a dose of at least about 2000 mg.

In some embodiments, the anhydrous Ca-AKG is administered at a dose of at least 350 mg and no greater than 2000 mg. In some instances, the anhydrous Ca-AKG is administered at a dose of about 350 mg to about 2000 mg. In some instances, the anhydrous Ca-AKG is administered at a dose of about 400 mg to about 2000 mg. In some instances, the anhydrous Ca-AKG is administered at a dose of about 450 mg to about 2000 mg. In some instances, the anhydrous Ca-AKG is administered at a dose of about 500 mg to about 2000 mg. In some instances, the anhydrous Ca-AKG is administered at a dose of about 550 mg to about 2000 mg. In some instances, the anhydrous Ca-AKG is administered at a dose of about 600 mg to about 2000 mg. In some instances, the anhydrous Ca-AKG is administered at a dose of about 650 mg to about 2000 mg. In some instances, the anhydrous Ca-AKG is administered at a dose of about 700 mg to about 2000 mg. In some instances, the anhydrous Ca-AKG is administered at a dose of about 800 mg to about 2000 mg. In some instances, the anhydrous Ca-AKG is administered at a dose of about 900 mg to about 2000 mg. In some instances, the anhydrous Ca-AKG is administered at a dose of about 1000 mg to about 2000 mg. In some instances, the anhydrous Ca-AKG is administered at a dose of about 1100 mg to about 2000 mg. In some instances, the anhydrous Ca-AKG is administered at a dose of about 1200 mg to about 2000 mg. In some instances, the anhydrous Ca-AKG is administered at a dose of about 1300 mg to about 2000 mg. In some instances, the anhydrous Ca-AKG is administered at a dose of about 1400 mg to about 2000 mg. In some instances, the anhydrous Ca-AKG is administered at a dose of about 1500 mg to about 2000 mg. In some instances, the anhydrous Ca-AKG is administered at a dose of about 1600 mg to about 2000 mg. In some instances, the anhydrous Ca-AKG is administered at a dose of about 1700 mg to about 2000 mg. In some instances, the anhydrous Ca-AKG is administered at a dose of about 1800 mg to about 2000 mg. In some instances, the anhydrous Ca-AKG is administered at a dose of about 1900 mg to about 2000 mg. In some instances, the anhydrous Ca-AKG is administered at a dose of about 350 mg to about 1900 mg. In some instances, the anhydrous Ca-AKG is administered at a dose of about 350 mg to about 1800 mg. In some instances, the anhydrous Ca-AKG is administered at a dose of about 350 mg to about 1700 mg. In some instances, the anhydrous Ca-AKG is administered at a dose of about 350 mg to about 1600 mg. In some instances, the anhydrous Ca-AKG is administered at a dose of about 350 mg to about 1500 mg. In some instances, the anhydrous Ca-AKG is administered at a dose of about 350 mg to about 1400 mg. In some instances, the anhydrous Ca-AKG is administered at a dose of about 350 mg to about 1300 mg. In some instances, the anhydrous Ca-AKG is administered at a dose of about 350 mg to about 1200 mg. In some instances, the anhydrous Ca-AKG is administered at a dose of about 350 mg to about 1100 mg. In some instances, the anhydrous Ca-AKG is administered at a dose of about 350 mg to about 1000 mg. In some instances, the anhydrous Ca-AKG is administered at a dose of about 350 mg to about 900 mg. In some instances, the anhydrous Ca-AKG is administered at a dose of about 350 mg to about 800 mg. In some instances, the anhydrous Ca-AKG is administered at a dose of about 350 mg to about 700 mg. In some instances, the anhydrous Ca-AKG is administered at a dose of about 350 mg to about 650 mg. In some instances, the anhydrous Ca-AKG is administered at a dose of about 350 mg to about 600 mg. In some instances, the anhydrous Ca-AKG is administered at a dose of about 350 mg to about 550 mg. In some instances, the anhydrous Ca-AKG is administered at a dose of about 350 mg to about 500 mg. In some instances, the anhydrous Ca-AKG is administered at a dose of about 350 mg to about 450 mg. In some instances, the anhydrous Ca-AKG is administered at a dose of about 350 mg to about 400 mg. In some instances, the anhydrous Ca-AKG is administered at a dose of at least about 350 mg. In some instances, the anhydrous Ca-AKG is administered at a dose of at least about 400 mg. In some instances, the anhydrous Ca-AKG is administered at a dose of at least about 450 mg. In some instances, the anhydrous Ca-AKG is administered at a dose of at least about 500 mg. In some instances, the anhydrous Ca-AKG is administered at a dose of at least about 550 mg. In some instances, the anhydrous Ca-AKG is administered at a dose of at least about 600 mg. In some instances, the anhydrous Ca-AKG is administered at a dose of at least about 650 mg. In some instances, the anhydrous Ca-AKG is administered at a dose of at least about 700 mg. In some instances, the anhydrous Ca-AKG is administered at a dose of at least about 750 mg. In some instances, the anhydrous Ca-AKG is administered at a dose of at least about 800 mg. In some instances, the anhydrous Ca-AKG is administered at a dose of at least about 900 mg. In some instances, the anhydrous Ca-AKG is administered at a dose of at least about 1000 mg. In some instances, the anhydrous Ca-AKG is administered at a dose of at least about 1100 mg. In some instances, the anhydrous Ca-AKG is administered at a dose of at least about 1200 mg. In some instances, the anhydrous Ca-AKG is administered at a dose of at least about 1300 mg. In some instances, the anhydrous Ca-AKG is administered at a dose of at least about 1400 mg. In some instances, the anhydrous Ca-AKG is administered at a dose of at least about 1500 mg. In some instances, the anhydrous Ca-AKG is administered at a dose of at least about 1600 mg. In some instances, the anhydrous Ca-AKG is administered at a dose of at least about 1700 mg. In some instances, the anhydrous Ca-AKG is administered at a dose of at least about 1800 mg. In some instances, the anhydrous Ca-AKG is administered at a dose of at least about 1900 mg. In some instances, the anhydrous Ca-AKG is administered at a dose of at least about 2000 mg.

In some embodiments, administration of compositions comprising AKG extends lifespan or survival in a subject in need thereof. In some embodiments, administration of compositions comprising berberine, AKG, and vitamin A extends lifespan or survival in a subject in need thereof. In some embodiments, administration of compositions comprising AKG and vitamin A extends lifespan or survival in a subject in need thereof. In some embodiments, administration of compositions comprising AKG and vitamin D extends lifespan or survival in a subject in need thereof. In some embodiments, administration of compositions comprising AKG and NMN extends lifespan or survival in a subject in need thereof.

In some embodiments, administration of the active agents or compositions thereof extend the lifespan of a non-human test subject relative to a control non-human test subject. In particular embodiments, the administration extends lifespan of a non-human test subject by at least 5%, at least 10%, at least 15%, at least 20%, or at least 25% relative to the lifespan of a control non-human test subject. In some embodiments the lifespan comparison is performed on an individual-to-individual basis, in which lifespan of individual non-human test subjects is correlated to a dose series or assayed biomarker levels and compared to those parameters assessed in a control population. In other embodiments, the comparison is performed by assessing the average lifespan in test and control groups of non-human subjects and comparing them.

In some embodiments, administration of the active agents or compositions thereof slow the rate of progression or reverse changes in biomarkers of non-human or human aging. Biomarker strategies to measure aging are currently being developed and can be employed to test the effects of aging interventions. The most prominent is the epigenetic clock, which likely measures biologic age in cells from humans (see for e.g. Chen et al. Aging, 8:1844-1865(2016), PMID 27690265), dogs (see for e.g. Thompson et al. Aging 9:1055-1068(2017), PMID 28373601), and mice (see for e.g. Petkovich et al. Cell Metab 25:954-960(2017), PMID 28380383). Other biomarker strategies include, but are not limited to, inflammatory cytokine levels, p16INK4A protein levels in specific cell populations, telomere length and levels of specific metabolites.

In some embodiments, the subject is a mammal. In some embodiments, the mammal is a human. In some embodiments, the mammal is a dog. In some embodiments, the mammal is livestock. In some embodiments, the livestock is selected from the group consisting of cattle, sheep, goats, swine, poultry, and equine animals.

In some embodiments, the composition is formulated into an orally administered form. In further embodiments, the orally administered form comprises a tablet, a powder, a suspension, a serum, an emulsion, a capsule, a granule, a pill, a gel, a solution, or a syrup. In some instances, the orally administered form is a sustained release dosage form. In some instances, the orally administered form is formulated into animal feed. In some instances, the orally administered form is the gel. In some embodiments, the composition is formulated into an injectable administered form. In further embodiments, the injectable administered form comprises a liquid, a suspension, or a solution. In some instances, the injectable administered form is a sustained release dosage form. In some embodiments, the composition is formulated into a topically administered form. In further embodiments, the topically administered form is a cream, a foam, a gel, a lotion, an ointment, or a serum. In some embodiments, the composition is formulated into a hair care product. In further embodiments, the hair care product is a shampoo, a conditioner, hair spray, or a moisturizer.

In some embodiments, the composition further comprises a pharmaceutically acceptable excipient. In further embodiments, the pharmaceutically acceptable excipient comprises an antiadherent, a binder, a coating, a color, disintegrant, a flavor, a glidant, a lubricant, a preservative, a sorbent, or a vehicle. In some instances, the composition further comprises silymarin.

In some embodiments, the composition is administered for a particular time period. In some embodiments, the composition is administered for a chronic treatment period, which is, for an extended period of time, including throughout the duration of the subject's life in order to ameliorate or otherwise control or limit the symptoms of the subject's disease or condition. Within the treatment period, the composition is administered on a particular time schedule. In further embodiments, the composition is administered one, two, three, or four times daily. In some embodiments, the composition is administered once daily. In some embodiments, the composition is administered twice daily. In some embodiments, the composition is administered in the morning and evening. In some embodiments, the composition is administered one, two, three, or four times weekly. In some embodiments, the composition is administered once a week. In some embodiments the composition is administered one, two, three, or four times monthly. In some embodiments, the composition is administered once a month. In some embodiments, the composition is administered for at least three months of every one year. In some embodiments, the composition is administered one month of every six months.

### Frailty

In an aspect, the disclosure provides methods for treating, delaying onset, or delaying progression of frailty using the active agents or compositions thereof described herein. The term "frailty" refers to a biological syndrome of decreased reserve and resistance to stressors due to decline in multiple physiological systems. Subjects suffering from frailty have improved likelihood of adverse health outcomes to events that stress one or more of their physiological systems. In humans, frailty frequently presents via non-specific symptoms, falls, delirium, fluctuating disability, or a combination thereof. Non-specific symptoms include extreme fatigue, unexplained weight loss, and frequent infections. Falls include hot falls (minor illness reducing postural balance below a threshold to maintain stability) or spontaneous falls (vital postural systems declining as a result of declines in vision, balance, and strength). Delirium refers to rapid onset of fluctuating confusion and impaired awareness. Fluctuating disability refers to day-to-day instability in the ability of a patient to function independently.

Various clinical scoring and evaluation systems for frailty are known to those skilled in the art and are suitable for assessing the effects of treatment on frailty. In some embodiments, frailty is evaluated in humans using the 70-item CSHA Frailty Index (see, for e.g. Theou et al. Age Ageing 42: 614-619 (2013)). A brief description of how the index is employed follows:

Items including the presence and/or severity of current diseases, ability in daily living and physical signs from the clinical and neurological examinations (see items in **Table 1** below) are evaluated. Each deficit is dichotomized or trichotomized and mapped to the interval 0-1 (i.e. individual items had scores of 0, 0.33, 0.50, 0.67 or 1.0), representing the occurrence and severity of the problem. For each person, a 70-dimentional vector is constructed, such that a person with 5 deficits would have a score of 5/70=0.071.

**Table 1. List of measures used to construct the Frailty Index**

| Self-rated health | Long-term illness | Limitations with Activities |
|---|---|---|
| In hospital last 12 months | Heart attack | High blood pressure |
| High blood cholesterol | Stroke or cerebral vascular Disease | Diabetes or high blood sugar |
| Chronic lung disease | Asthma | Arthritis |
| Osteoporosis | Cancer | Stomach or duodenal ulcer |
| Parkinson disease | Cataracts | Hip or femoral fracture |
| Pain in any joint | Heart trouble or angina | Breathlessness |
| Persistent cough | Swollen legs | Sleeping problems |
| Falling down | Fear of falling down | Dizziness |
| Stomach or intestine Problems | Incontinence | Dentures |
| Biting on hard foods | Eyesight | Hearing |
| Walking 100 meters | Sitting for about two hours | Getting up from a chair after prolonged sitting |
| Climbing several flights of Stairs | Stooping, kneeling, or Crouching | Reaching or extending arm above shoulder level |
| Pulling or pushing large Objects | Lifting or carrying weights over 10 pounds/5 kilos | Picking up a small coin from a table |
| Dressing | Walking across a room | Bathing or showering |
| Eating | Getting in or out of bed | Using the toilet |
| Using a map to figure out how to get around | Preparing a hot meal | Shopping for groceries |
| Making telephone calls | Taking medications | Doing work around the house or garden |
| Managing money | Vigorous activities | Moderate Activities |
| Orientation | Mathematical performance | Verbal fluency score |
| Delayed recall test | Depression | Pessimism |
| Suicidality | Sleep | Interest |
| Appetite | Fatigue | Concentration |

Further detail on application of the CSHA frailty index (e.g. calculation of scores for individual measures) can be found in other publications in the field, for e.g. in Searle et al. A standard procedure for creating a frailty index. BMC Geriatrics 8:24 (2008). An example of the use of the CSHA frailty index in humans can be found in **Example 5,** where selection of pre-frail individuals for treatment and evaluation of pharmacological treatment of frailty is described.

In other embodiments, frailty is evaluated in non-human animals, such as mice. Recognized signs of frailty in mice correspond to many of those in humans, and involve metabolic (e.g. body temperature, body weight), integumental (e.g. alopecia, loss of fur color, dermatitis, loss of whiskers, grooming), physical/musculoskeletal (e.g. tumors, distended abdomen, kyphosis, tail stiffening, gait disorder, tremor, decreased forelimb grip strength, body condition/muscle wasting/obesity), vestibulocochlear/auditory (e.g. vestibular disturbance, hearing loss), ocular/nasal (e.g. cataracts, corneal opacity, eye discharge, microphthalmia, vision loss, increased menace reflex, nasal discharge), digestive/urogenital (e.g. malocclusions, rectal prolapse, vaginal/uterine/penile prolapse, diarrhea), respiratory (e.g. abnormal breathing rate or depth), and discomfort symptoms (e.g. increased mouse grimace scale, piloerection).

In some embodiments, frailty is assessed in mice via a 31-item clinical frailty index encompassing the 31 example phenotypes recited above as described in, for e.g. Whitehead et al. J Gerontol A Biol Sci Med Sci 69:621-632 (2014). Clinical examinations are performed at approximately the same time every day, and involve body weight and surface temperature measurement by abdominal infrared, followed by a clinical exam to evaluate the 31 frailty phenotypes. The severity of each deficit is rated on a scale, with 0 given for no sign of a deficit, 0.5 for a mild deficit, and 1 for a severe deficit. Deficits in body weight (g) and body surface temperature (°C) are scored in quantiles between 0 and 1 based on number of standard deviations from reference values in young adult animals (0.25, 0.5, 0.75, and 1.0) according to how many standard deviations the score varies from the mean (1 is >3SD). The sum of the scores for each parameter produce the final 31-item frailty index, which can be compared between individual mice according to standard statistical techniques to assess frailty.

In some embodiments, frailty is assessed in mice via an abbreviated with an eight-item functional frailty index as described in, for e.g. Whitehead et al. J Gerontol A Biol Sci Med Sci 69:621-632 (2014) and Parks et al. J Gerontol A Biol Sci Med Sci. 67:217-227 (2012). In this method, 7 performance parameters based on open-field behavior of mouse subjects are assessed: 1) total distance moved in 10 minutes; 2) maximal distance moved between bouts of inactivity; 3) total duration of movement (seconds); 4) percent of total time spent moving; 5) the change in direction per unit distance moved, called meander (degrees/cm; from 0° to 180°); 6) the average velocity of movement over 10 minutes (cm/s); and 7) rearing frequency (number of occurrences/10 min). An eighth non-movement parameter, weight, is additionally assessed. Open-field assessments are performed between 10 am and noon each day. Mice are weighed and activity was recorded with automated video tracking software for 10 minutes in an open-field arena. Videos are digitized with an analog-to-digital converter and analyzed with video tracking analysis software to obtain values for the parameters used to create the eight-item frailty index. Mean and standard deviation for each of these parameters are calculated and assigned to a score quantile between 0 and 1 (0.25, 0.5, 0.75, and 1.0) according to how many standard deviations the score varies from the mean (1 is >3SD). The parameters are added, and divided by eight to receive a frailty index score between 0 and 1 for the mouse subjects. Higher scores correspond to increasingly frail mice.

In some embodiments, the disclosure provides methods for treating, delaying onset, or delaying progression of frailty using the compositions disclosed herein. In some aspects, the composition comprises two or more active agents selected from the group consisting of: alpha-ketoglutarate, berberine, vitamin A, vitamin D and nicotinamide mononucleotide (NMN). In certain aspects, the composition comprises berberine, α-ketoglutarate (AKG), and vitamin A. In certain aspects, the composition comprises berberine, α-ketoglutarate (AKG), and retinoic acid (RA). In certain aspects, the composition comprises berberine, α-ketoglutarate (AKG), and retinyl palmitate (RP). In certain aspects, the composition comprises AKG and vitamin A. In certain aspects, the composition comprises AKG and retinoic acid (RA). In certain aspects, the composition comprises AKG and retinyl palmitate (RP). In certain aspects, the composition comprises AKG and vitamin D. In certain aspects, the composition comprises AKG and vitamin D3. In certain aspects, the composition comprises AKG and nicotinamide mononucleotide (NMN). In some instances, the AKG in the compositions is a salt of AKG. In some instances, the AKG provided in the compositions is a calcium salt of AKG (Ca-AKG). In some embodiments, the two or more compounds comprise alpha-ketoglutarate and berberine. In yet other embodiments, the two or more compounds comprise berberine and vitamin A. In some aspects, disclosure further provides compositions comprising an active agent selected from the group consisting of: alpha-ketoglutarate, berberine, vitamin A and vitamin D. In certain aspects, the compound is AKG. In some embodiments, AKG is provided as a salt. In some embodiments, AKG is provided as a calcium salt (Ca-AKG).

**In** certain aspects, the disclosure provides methods for treating, delaying onset, or delaying progression of frailty in a subject in need thereof comprising administering to the subject a composition comprising a calcium salt of alpha-ketoglutarate. In another aspect, the disclosure provides methods for treating, delaying onset, or delaying progression of frailty in a subject in need thereof comprising administering to the subject a composition consisting essentially of a calcium salt of alpha-ketoglutarate. In some embodiments, calcium α-ketoglutarate can be a hydrate calcium α-ketoglutarate. In some embodiments, calcium α-ketoglutarate can be a mono-hydrate calcium α-ketoglutarate. In some embodiments, calcium α-ketoglutarate can be hemi-hydrate calcium α-ketoglutarate. In some embodiments, calcium α-ketoglutarate can be anhydrous calcium α-ketoglutarate.

**In** some embodiments, the Ca-AKG is administered at a dose of at least 350 mg and no greater than 2000 mg. In some instances, the Ca-AKG is administered at a dose of about 350 mg to about 2000 mg. In some instances, the Ca-AKG is administered at a dose of about 400 mg to about 2000 mg. In some instances, the Ca-AKG is administered at a dose of about 450 mg to about 2000 mg. In some instances, the Ca-AKG is administered at a dose of about 500 mg to about 2000 mg. In some instances, the Ca-AKG is administered at a dose of about 550 mg to about 2000 mg. In some instances, the Ca-AKG is administered at a dose of about 600 mg to about 2000 mg. In some instances, the Ca-AKG is administered at a dose of about 650 mg to about 2000 mg. In some instances, the Ca-AKG is administered at a dose of about 700 mg to about 2000 mg. In some instances, the Ca-AKG is administered at a dose of about 800 mg to about 2000 mg. In some instances, the Ca-AKG is administered at a dose of about 900 mg to about 2000 mg. In some instances, the Ca-AKG is administered at a dose of about 1000 mg to about 2000 mg. In some instances, the Ca-AKG is administered at a dose of about 1100 mg to about 2000 mg. In some instances, the Ca-AKG is administered at a dose of about 1200 mg to about 2000 mg. In some instances, the Ca-AKG is administered at a dose of about 1300 mg to about 2000 mg. In some instances, the Ca-AKG is administered at a dose of about 1400 mg to about 2000 mg. In some instances, the Ca-AKG is administered at a dose of about 1500 mg to about 2000 mg. In some instances, the Ca-AKG is administered at a dose of about 1600 mg to about 2000 mg. In some instances, the Ca-AKG is administered at a dose of about 1700 mg to about 2000 mg. In some instances, the Ca-AKG is administered at a dose of about 1800 mg to about 2000 mg. In some instances, the Ca-AKG is administered at a dose of about 1900 mg to about 2000 mg. In some instances, the Ca-AKG is administered at a dose of about 350 mg to about 1900 mg. In some instances, the Ca-AKG is administered at a dose of about 350 mg to about 1800 mg. In some instances, the Ca-AKG is administered at a dose of about 350 mg to about 1700 mg. In some instances, the Ca-AKG is administered at a dose of about 350 mg to about 1600 mg. In some instances, the Ca-AKG is administered at a dose of about 350 mg to about 1500 mg. In some instances, the Ca-AKG is administered at a dose of about 350 mg to about 1400 mg. In some instances, the Ca-AKG is administered at a dose of about 350 mg to about 1300 mg. In some instances, the Ca-AKG is administered at a dose of about 350 mg to about 1200 mg. In some instances, the Ca-AKG is administered at a dose of about 350 mg to about 1100 mg. In some instances, the Ca-AKG is administered at a dose of about 350 mg to about 1000 mg. In some instances, the Ca-AKG is administered at a dose of about 350 mg to about 900 mg. In some instances, the Ca-AKG is administered at a dose of about 350 mg to about 800 mg. In some instances, the Ca-AKG is administered at a dose of about 350 mg to about 700 mg. In some instances, the Ca-AKG is administered at a dose of about 350 mg to about 650 mg. In some instances, the Ca-AKG is administered at a dose of about 350 mg to about 600 mg. In some instances, the Ca-AKG is administered at a dose of about 350 mg to about 550 mg. In some instances, the Ca-AKG is administered at a dose of about 350 mg to about 500 mg. In some instances, the Ca-AKG is administered at a dose of about 350 mg to about 450 mg. In some instances, the Ca-AKG is administered at a dose of about 350 mg to about 400 mg. In some instances, the Ca-AKG is administered at a dose of at least about 350 mg. In some instances, the Ca-AKG is administered at a dose of at least about 400 mg. In some instances, the Ca-AKG is administered at a dose of at least about 450 mg. In some instances, the Ca-AKG is administered at a dose of at least about 500 mg. In some instances, the Ca-AKG is administered at a dose of at least about 550 mg. In some instances, the Ca-AKG is administered at a dose of at least about 600 mg. In some instances, the Ca-AKG is administered at a dose of at least about 650 mg. In some instances, the Ca-AKG is administered at a dose of at least about 700 mg. In some instances, the Ca-AKG is administered at a dose of at least about 750 mg. In some instances, the Ca-AKG is administered at a dose of at least about 800 mg. In some instances, the Ca-AKG is administered at a dose of at least about 900 mg. In some instances, the Ca-AKG is administered at a dose of at least about 1000 mg. In some instances, the Ca-AKG is administered at a dose of at least about 1100 mg. In some instances, the Ca-AKG is administered at a dose of at least about 1200 mg. In some instances, the Ca-AKG is administered at a dose of at least about 1300 mg. In some instances, the Ca-AKG is administered at a dose of at least about 1400 mg. In some instances, the Ca-AKG is administered at a dose of at least about 1500 mg. In some instances, the Ca-AKG is administered at a dose of at least about 1600 mg. In some instances, the Ca-AKG is administered at a dose of at least about 1700 mg. In some instances, the Ca-AKG is administered at a dose of at least about 1800 mg. In some instances, the Ca-AKG is administered at a dose of at least about 1900 mg. In some instances, the Ca-AKG is administered at a dose of at least about 2000 mg.

**In** some embodiments, the hydrate Ca-AKG is administered at a dose of at least 350 mg and no greater than 2000 mg. In some instances, the hydrate Ca-AKG is administered at a dose of about 350 mg to about 2000 mg. In some instances, the hydrate Ca-AKG is administered at a dose of about 400 mg to about 2000 mg. In some instances, the hydrate Ca-AKG is administered at a dose of about 450 mg to about 2000 mg. In some instances, the hydrate Ca-AKG is administered at a dose of about 500 mg to about 2000 mg. In some instances, the hydrate Ca-AKG is administered at a dose of about 550 mg to about 2000 mg. In some instances, the hydrate Ca-AKG is administered at a dose of about 600 mg to about 2000 mg. In some instances, the hydrate Ca-AKG is administered at a dose of about 650 mg to about 2000 mg. In some instances, the hydrate Ca-AKG is administered at a dose of about 700 mg to about 2000 mg. In some instances, the hydrate Ca-AKG is administered at a dose of about 800 mg to about 2000 mg. In some instances, the hydrate Ca-AKG is administered at a dose of about 900 mg to about 2000 mg. In some instances, the hydrate Ca-AKG is administered at a dose of about 1000 mg to about 2000 mg. In some instances, the hydrate Ca-AKG is administered at a dose of about 1100 mg to about 2000 mg. In some instances, the hydrate Ca-AKG is administered at a dose of about 1200 mg to about 2000 mg. In some instances, the hydrate Ca-AKG is administered at a dose of about 1300 mg to about 2000 mg. In some instances, the hydrate Ca-AKG is administered at a dose of about 1400 mg to about 2000 mg. In some instances, the hydrate Ca-AKG is administered at a dose of about 1500 mg to about 2000 mg. In some instances, the hydrate Ca-AKG is administered at a dose of about 1600 mg to about 2000 mg. In some instances, the hydrate Ca-AKG is administered at a dose of about 1700 mg to about 2000 mg. In some instances, the hydrate Ca-AKG is administered at a dose of about 1800 mg to about 2000 mg. In some instances, the hydrate Ca-AKG is administered at a dose of about 1900 mg to about 2000 mg. In some instances, the hydrate Ca-AKG is administered at a dose of about 350 mg to about 1900 mg. In some instances, the hydrate Ca-AKG is administered at a dose of about 350 mg to about 1800 mg. In some instances, the hydrate Ca-AKG is administered at a dose of about 350 mg to about 1700 mg. In some instances, the hydrate Ca-AKG is administered at a dose of about 350 mg to about 1600 mg. In some instances, the hydrate Ca-AKG is administered at a dose of about 350 mg to about 1500 mg. In some instances, the hydrate Ca-AKG is administered at a dose of about 350 mg to about 1400 mg. In some instances, the hydrate Ca-AKG is administered at a dose of about 350 mg to about 1300 mg. In some instances, the hydrate Ca-AKG is administered at a dose of about 350 mg to about 1200 mg. In some instances, the hydrate Ca-AKG is administered at a dose of about 350 mg to about 1100 mg. In some instances, the hydrate Ca-AKG is administered at a dose of about 350 mg to about 1000 mg. In some instances, the hydrate Ca-AKG is administered at a dose of about 350 mg to about 900 mg. In some instances, the hydrate Ca-AKG is administered at a dose of about 350 mg to about 800 mg. In some instances, the hydrate Ca-AKG is administered at a dose of about 350 mg to about 700 mg. In some instances, the hydrate Ca-AKG is administered at a dose of about 350 mg to about 650 mg. In some instances, the hydrate Ca-AKG is administered at a dose of about 350 mg to about 600 mg. In some instances, the hydrate Ca-AKG is administered at a dose of about 350 mg to about 550 mg. In some instances, the hydrate Ca-AKG is administered at a dose of about 350 mg to about 500 mg. In some instances, the hydrate Ca-AKG is administered at a dose of about 350 mg to about 450 mg. In some instances, the hydrate Ca-AKG is administered at a dose of about 350 mg to about 400 mg. In some instances, the hydrate Ca-AKG is administered at a dose of at least about 350 mg. In some instances, the hydrate Ca-AKG is administered at a dose of at least about 400 mg. In some instances, the hydrate Ca-AKG is administered at a dose of at least about 450 mg. In some instances, the hydrate Ca-AKG is administered at a dose of at least about 500 mg. In some instances, the hydrate Ca-AKG is administered at a dose of at least about 550 mg. In some instances, the hydrate Ca-AKG is administered at a dose of at least about 600 mg. In some instances, the hydrate Ca-AKG is administered at a dose of at least about 650 mg. In some instances, the hydrate Ca-AKG is administered at a dose of at least about 700 mg. In some instances, the hydrate Ca-AKG is administered at a dose of at least about 750 mg. In some instances, the hydrate Ca-AKG is administered at a dose of at least about 800 mg. In some instances, the hydrate Ca-AKG is administered at a dose of at least about 900 mg. In some instances, the hydrate Ca-AKG is administered at a dose of at least about 1000 mg. In some instances, the hydrate Ca-AKG is administered at a dose of at least about 1100 mg. In some instances, the hydrate Ca-AKG is administered at a dose of at least about 1200 mg. In some instances, the hydrate Ca-AKG is administered at a dose of at least about 1300 mg. In some instances, the hydrate Ca-AKG is administered at a dose of at least about 1400 mg. In some instances, the hydrate Ca-AKG is administered at a dose of at least about 1500 mg. In some instances, the hydrate Ca-AKG is administered at a dose of at least about 1600 mg. In some instances, the hydrate Ca-AKG is administered at a dose of at least about 1700 mg. In some instances, the hydrate Ca-AKG is administered at a dose of at least about 1800 mg. In some instances, the hydrate Ca-AKG is administered at a dose of at least about 1900 mg. In some instances, the hydrate Ca-AKG is administered at a dose of at least about 2000 mg.

In some embodiments, the mono-hydrate Ca-AKG is administered at a dose of at least 350 mg and no greater than 2000 mg. In some instances, the mono-hydrate Ca-AKG is administered at a dose of about 350 mg to about 2000 mg. In some instances, the mono-hydrate Ca-AKG is administered at a dose of about 400 mg to about 2000 mg. In some instances, the mono-hydrate Ca-AKG is administered at a dose of about 450 mg to about 2000 mg. In some instances, the mono-hydrate Ca-AKG is administered at a dose of about 500 mg to about 2000 mg. In some instances, the mono-hydrate Ca-AKG is administered at a dose of about 550 mg to about 2000 mg. In some instances, the mono-hydrate Ca-AKG is administered at a dose of about 600 mg to about 2000 mg. In some instances, the mono-hydrate Ca-AKG is administered at a dose of about 650 mg to about 2000 mg. In some instances, the mono-hydrate Ca-AKG is administered at a dose of about 700 mg to about 2000 mg. In some instances, the mono-hydrate Ca-AKG is administered at a dose of about 800 mg to about 2000 mg. In some instances, the mono-hydrate Ca-AKG is administered at a dose of about 900 mg to about 2000 mg. In some instances, the mono-hydrate Ca-AKG is administered at a dose of about 1000 mg to about 2000 mg. In some instances, the mono-hydrate Ca-AKG is administered at a dose of about 1100 mg to about 2000 mg. In some instances, the mono-hydrate Ca-AKG is administered at a dose of about 1200 mg to about 2000 mg. In some instances, the mono-hydrate Ca-AKG is administered at a dose of about 1300 mg to about 2000 mg. In some instances, the mono-hydrate Ca-AKG is administered at a dose of about 1400 mg to about 2000 mg. In some instances, the mono-hydrate Ca-AKG is administered at a dose of about 1500 mg to about 2000 mg. In some instances, the mono-hydrate Ca-AKG is administered at a dose of about 1600 mg to about 2000 mg. In some instances, the mono-hydrate Ca-AKG is administered at a dose of about 1700 mg to about 2000 mg. In some instances, the mono-hydrate Ca-AKG is administered at a dose of about 1800 mg to about 2000 mg. In some instances, the mono-hydrate Ca-AKG is administered at a dose of about 1900 mg to about 2000 mg. In some instances, the mono-hydrate Ca-AKG is administered at a dose of about 350 mg to about 1900 mg. In some instances, the mono-hydrate Ca-AKG is administered at a dose of about 350 mg to about 1800 mg. In some instances, the mono-hydrate Ca-AKG is administered at a dose of about 350 mg to about 1700 mg. In some instances, the mono-hydrate Ca-AKG is administered at a dose of about 350 mg to about 1600 mg. In some instances, the mono-hydrate Ca-AKG is administered at a dose of about 350 mg to about 1500 mg. In some instances, the mono-hydrate Ca-AKG is administered at a dose of about 350 mg to about 1400 mg. In some instances, the mono-hydrate Ca-AKG is administered at a dose of about 350 mg to about 1300 mg. In some instances, the mono-hydrate Ca-AKG is administered at a dose of about 350 mg to about 1200 mg. In some instances, the mono-hydrate Ca-AKG is administered at a dose of about 350 mg to about 1100 mg. In some instances, the mono-hydrate Ca-AKG is administered at a dose of about 350 mg to about 1000 mg. In some instances, the mono-hydrate Ca-AKG is administered at a dose of about 350 mg to about 900 mg. In some instances, the mono-hydrate Ca-AKG is administered at a dose of about 350 mg to about 800 mg. In some instances, the mono-hydrate Ca-AKG is administered at a dose of about 350 mg to about 700 mg. In some instances, the mono-hydrate Ca-AKG is administered at a dose of about 350 mg to about 650 mg. In some instances, the mono-hydrate Ca-AKG is administered at a dose of about 350 mg to about 600 mg. In some instances, the mono-hydrate Ca-AKG is administered at a dose of about 350 mg to about 550 mg. In some instances, the mono-hydrate Ca-AKG is administered at a dose of about 350 mg to about 500 mg. In some instances, the mono-hydrate Ca-AKG is administered at a dose of about 350 mg to about 450 mg. In some instances, the mono-hydrate Ca-AKG is administered at a dose of about 350 mg to about 400 mg. In some instances, the mono-hydrate Ca-AKG is administered at a dose of at least about 350 mg. In some instances, the mono-hydrate Ca-AKG is administered at a dose of at least about 400 mg. In some instances, the mono-hydrate Ca-AKG is administered at a dose of at least about 450 mg. In some instances, the mono-hydrate Ca-AKG is administered at a dose of at least about 500 mg. In some instances, the mono-hydrate Ca-AKG is administered at a dose of at least about 550 mg. In some instances, the mono-hydrate Ca-AKG is administered at a dose of at least about 600 mg. In some instances, the mono-hydrate Ca-AKG is administered at a dose of at least about 650 mg. In some instances, the mono-hydrate Ca-AKG is administered at a dose of at least about 700 mg. In some instances, the mono-hydrate Ca-AKG is administered at a dose of at least about 750 mg. In some instances, the mono-hydrate Ca-AKG is administered at a dose of at least about 800 mg. In some instances, the mono-hydrate Ca-AKG is administered at a dose of at least about 900 mg. In some instances, the mono-hydrate Ca-AKG is administered at a dose of at least about 1000 mg. In some instances, the mono-hydrate Ca-AKG is administered at a dose of at least about 1100 mg. In some instances, the mono-hydrate Ca-AKG is administered at a dose of at least about 1200 mg. In some instances, the mono-hydrate Ca-AKG is administered at a dose of at least about 1300 mg. In some instances, the mono-hydrate Ca-AKG is administered at a dose of at least about 1400 mg. In some instances, the mono-hydrate Ca-AKG is administered at a dose of at least about 1500 mg. In some instances, the mono-hydrate Ca-AKG is administered at a dose of at least about 1600 mg. In some instances, the mono-hydrate Ca-AKG is administered at a dose of at least about 1700 mg. In some instances, the mono-hydrate Ca-AKG is administered at a dose of at least about 1800 mg. In some instances, the mono-hydrate Ca-AKG is administered at a dose of at least about 1900 mg. In some instances, the mono-hydrate Ca-AKG is administered at a dose of at least about 2000 mg.

In some embodiments, the hemi-hydrate Ca-AKG is administered at a dose of at least 350 mg and no greater than 2000 mg. In some instances, the hemi-hydrate Ca-AKG is administered at a dose of about 350 mg to about 2000 mg. In some instances, the hemi-hydrate Ca-AKG is administered at a dose of about 400 mg to about 2000 mg. In some instances, the hemi-hydrate Ca-AKG is administered at a dose of about 450 mg to about 2000 mg. In some instances, the hemi-hydrate Ca-AKG is administered at a dose of about 500 mg to about 2000 mg. In some instances, the hemi-hydrate Ca-AKG is administered at a dose of about 550 mg to about 2000 mg. In some instances, the hemi-hydrate Ca-AKG is administered at a dose of about 600 mg to about 2000 mg. In some instances, the hemi-hydrate Ca-AKG is administered at a dose of about 650 mg to about 2000 mg. In some instances, the hemi-hydrate Ca-AKG is administered at a dose of about 700 mg to about 2000 mg. In some instances, the hemi-hydrate Ca-AKG is administered at a dose of about 800 mg to about 2000 mg. In some instances, the hemi-hydrate Ca-AKG is administered at a dose of about 900 mg to about 2000 mg. In some instances, the hemi-hydrate Ca-AKG is administered at a dose of about 1000 mg to about 2000 mg. In some instances, the hemi-hydrate Ca-AKG is administered at a dose of about 1100 mg to about 2000 mg. In some instances, the hemi-hydrate Ca-AKG is administered at a dose of about 1200 mg to about 2000 mg. In some instances, the hemi-hydrate Ca-AKG is administered at a dose of about 1300 mg to about 2000 mg. In some instances, the hemi-hydrate Ca-AKG is administered at a dose of about 1400 mg to about 2000 mg. In some instances, the hemi-hydrate Ca-AKG is administered at a dose of about 1500 mg to about 2000 mg. In some instances, the hemi-hydrate Ca-AKG is administered at a dose of about 1600 mg to about 2000 mg. In some instances, the hemi-hydrate Ca-AKG is administered at a dose of about 1700 mg to about 2000 mg. In some instances, the hemi-hydrate Ca-AKG is administered at a dose of about 1800 mg to about 2000 mg. In some instances, the hemi-hydrate Ca-AKG is administered at a dose of about 1900 mg to about 2000 mg. In some instances, the hemi-hydrate Ca-AKG is administered at a dose of about 350 mg to about 1900 mg. In some instances, the hemi-hydrate Ca-AKG is administered at a dose of about 350 mg to about 1800 mg. In some instances, the hemi-hydrate Ca-AKG is administered at a dose of about 350 mg to about 1700 mg. In some instances, the hemi-hydrate Ca-AKG is administered at a dose of about 350 mg to about 1600 mg. In some instances, the hemi-hydrate Ca-AKG is administered at a dose of about 350 mg to about 1500 mg. In some instances, the hemi-hydrate Ca-AKG is administered at a dose of about 350 mg to about 1400 mg. In some instances, the hemi-hydrate Ca-AKG is administered at a dose of about 350 mg to about 1300 mg. In some instances, the hemi-hydrate Ca-AKG is administered at a dose of about 350 mg to about 1200 mg. In some instances, the hemi-hydrate Ca-AKG is administered at a dose of about 350 mg to about 1100 mg. In some instances, the hemi-hydrate Ca-AKG is administered at a dose of about 350 mg to about 1000 mg. In some instances, the hemi-hydrate Ca-AKG is administered at a dose of about 350 mg to about 900 mg. In some instances, the hemi-hydrate Ca-AKG is administered at a dose of about 350 mg to about 800 mg. In some instances, the hemi-hydrate Ca-AKG is administered at a dose of about 350 mg to about 700 mg. In some instances, the hemi-hydrate Ca-AKG is administered at a dose of about 350 mg to about 650 mg. In some instances, the hemi-hydrate Ca-AKG is administered at a dose of about 350 mg to about 600 mg. In some instances, the hemi-hydrate Ca-AKG is administered at a dose of about 350 mg to about 550 mg. In some instances, the hemi-hydrate Ca-AKG is administered at a dose of about 350 mg to about 500 mg. In some instances, the hemi-hydrate Ca-AKG is administered at a dose of about 350 mg to about 450 mg. In some instances, the hemi-hydrate Ca-AKG is administered at a dose of about 350 mg to about 400 mg. In some instances, the hemi-hydrate Ca-AKG is administered at a dose of at least about 350 mg. In some instances, the hemi-hydrate Ca-AKG is administered at a dose of at least about 400 mg. In some instances, the hemi-hydrate Ca-AKG is administered at a dose of at least about 450 mg. In some instances, the hemi-hydrate Ca-AKG is administered at a dose of at least about 500 mg. In some instances, the hemi-hydrate Ca-AKG is administered at a dose of at least about 550 mg. In some instances, the hemi-hydrate Ca-AKG is administered at a dose of at least about 600 mg. In some instances, the hemi-hydrate Ca-AKG is administered at a dose of at least about 650 mg. In some instances, the hemi-hydrate Ca-AKG is administered at a dose of at least about 700 mg. In some instances, the hemi-hydrate Ca-AKG is administered at a dose of at least about 750 mg. In some instances, the hemi-hydrate Ca-AKG is administered at a dose of at least about 800 mg. In some instances, the hemi-hydrate Ca-AKG is administered at a dose of at least about 900 mg. In some instances, the hemi-hydrate Ca-AKG is administered at a dose of at least about 1000 mg. In some instances, the hemi-hydrate Ca-AKG is administered at a dose of at least about 1100 mg. In some instances, the hemi-hydrate Ca-AKG is administered at a dose of at least about 1200 mg. In some instances, the hemi-hydrate Ca-AKG is administered at a dose of at least about 1300 mg. In some instances, the hemi-hydrate Ca-AKG is administered at a dose of at least about 1400 mg. In some instances, the hemi-hydrate Ca-AKG is administered at a dose of at least about 1500 mg. In some instances, the hemi-hydrate Ca-AKG is administered at a dose of at least about 1600 mg. In some instances, the hemi-hydrate Ca-AKG is administered at a dose of at least about 1700 mg. In some instances, the hemi-hydrate Ca-AKG is administered at a dose of at least about 1800 mg. In some instances, the hemi-hydrate Ca-AKG is administered at a dose of at least about 1900 mg. In some instances, the hemi-hydrate Ca-AKG is administered at a dose of at least about 2000 mg.

In some embodiments, the anhydrous Ca-AKG is administered at a dose of at least 350 mg and no greater than 2000 mg. In some instances, the anhydrous Ca-AKG is administered at a dose of about 350 mg to about 2000 mg. In some instances, the anhydrous Ca-AKG is administered at a dose of about 400 mg to about 2000 mg. In some instances, the anhydrous Ca-AKG is administered at a dose of about 450 mg to about 2000 mg. In some instances, the anhydrous Ca-AKG is administered at a dose of about 500 mg to about 2000 mg. In some instances, the anhydrous Ca-AKG is administered at a dose of about 550 mg to about 2000 mg. In some instances, the anhydrous Ca-AKG is administered at a dose of about 600 mg to about 2000 mg. In some instances, the anhydrous Ca-AKG is administered at a dose of about 650 mg to about 2000 mg. In some instances, the anhydrous Ca-AKG is administered at a dose of about 700 mg to about 2000 mg. In some instances, the anhydrous Ca-AKG is administered at a dose of about 800 mg to about 2000 mg. In some instances, the anhydrous Ca-AKG is administered at a dose of about 900 mg to about 2000 mg. In some instances, the anhydrous Ca-AKG is administered at a dose of about 1000 mg to about 2000 mg. In some instances, the anhydrous Ca-AKG is administered at a dose of about 1100 mg to about 2000 mg. In some instances, the anhydrous Ca-AKG is administered at a dose of about 1200 mg to about 2000 mg. In some instances, the anhydrous Ca-AKG is administered at a dose of about 1300 mg to about 2000 mg. In some instances, the anhydrous Ca-AKG is administered at a dose of about 1400 mg to about 2000 mg. In some instances, the anhydrous Ca-AKG is administered at a dose of about 1500 mg to about 2000 mg. In some instances, the anhydrous Ca-AKG is administered at a dose of about 1600 mg to about 2000 mg. In some instances, the anhydrous Ca-AKG is administered at a dose of about 1700 mg to about 2000 mg. In some instances, the anhydrous Ca-AKG is administered at a dose of about 1800 mg to about 2000 mg. In some instances, the anhydrous Ca-AKG is administered at a dose of about 1900 mg to about 2000 mg. In some instances, the anhydrous Ca-AKG is administered at a dose of about 350 mg to about 1900 mg. In some instances, the anhydrous Ca-AKG is administered at a dose of about 350 mg to about 1800 mg. In some instances, the anhydrous Ca-AKG is administered at a dose of about 350 mg to about 1700 mg. In some instances, the anhydrous Ca-AKG is administered at a dose of about 350 mg to about 1600 mg. In some instances, the anhydrous Ca-AKG is administered at a dose of about 350 mg to about 1500 mg. In some instances, the anhydrous Ca-AKG is administered at a dose of about 350 mg to about 1400 mg. In some instances, the anhydrous Ca-AKG is administered at a dose of about 350 mg to about 1300 mg. In some instances, the anhydrous Ca-AKG is administered at a dose of about 350 mg to about 1200 mg. In some instances, the anhydrous Ca-AKG is administered at a dose of about 350 mg to about 1100 mg. In some instances, the anhydrous Ca-AKG is administered at a dose of about 350 mg to about 1000 mg. In some instances, the anhydrous Ca-AKG is administered at a dose of about 350 mg to about 900 mg. In some instances, the anhydrous Ca-AKG is administered at a dose of about 350 mg to about 800 mg. In some instances, the anhydrous Ca-AKG is administered at a dose of about 350 mg to about 700 mg. In some instances, the anhydrous Ca-AKG is administered at a dose of about 350 mg to about 650 mg. In some instances, the anhydrous Ca-AKG is administered at a dose of about 350 mg to about 600 mg. In some instances, the anhydrous Ca-AKG is administered at a dose of about 350 mg to about 550 mg. In some instances, the anhydrous Ca-AKG is administered at a dose of about 350 mg to about 500 mg. In some instances, the anhydrous Ca-AKG is administered at a dose of about 350 mg to about 450 mg. In some instances, the anhydrous Ca-AKG is administered at a dose of about 350 mg to about 400 mg. In some instances, the anhydrous Ca-AKG is administered at a dose of at least about 350 mg. In some instances, the anhydrous Ca-AKG is administered at a dose of at least about 400 mg. In some instances, the anhydrous Ca-AKG is administered at a dose of at least about 450 mg. In some instances, the anhydrous Ca-AKG is administered at a dose of at least about 500 mg. In some instances, the anhydrous Ca-AKG is administered at a dose of at least about 550 mg. In some instances, the anhydrous Ca-AKG is administered at a dose of at least about 600 mg. In some instances, the anhydrous Ca-AKG is administered at a dose of at least about 650 mg. In some instances, the anhydrous Ca-AKG is administered at a dose of at least about 700 mg. In some instances, the anhydrous Ca-AKG is administered at a dose of at least about 750 mg. In some instances, the anhydrous Ca-AKG is administered at a dose of at least about 800 mg. In some instances, the anhydrous Ca-AKG is administered at a dose of at least about 900 mg. In some instances, the anhydrous Ca-AKG is administered at a dose of at least about 1000 mg. In some instances, the anhydrous Ca-AKG is administered at a dose of at least about 1100 mg. In some instances, the anhydrous Ca-AKG is administered at a dose of at least about 1200 mg. In some instances, the anhydrous Ca-AKG is administered at a dose of at least about 1300 mg. In some instances, the anhydrous Ca-AKG is administered at a dose of at least about 1400 mg. In some instances, the anhydrous Ca-AKG is administered at a dose of at least about 1500 mg. In some instances, the anhydrous Ca-AKG is administered at a dose of at least about 1600 mg. In some instances, the anhydrous Ca-AKG is administered at a dose of at least about 1700 mg. In some instances, the anhydrous Ca-AKG is administered at a dose of at least about 1800 mg. In some instances, the anhydrous Ca-AKG is administered at a dose of at least about 1900 mg. In some instances, the anhydrous Ca-AKG is administered at a dose of at least about 2000 mg.

In some embodiments, administration of compositions comprising AKG delays onset or delays progression of a frailty phenotype in a subject in need thereof. In some embodiments, administration of compositions comprising berberine, AKG, and vitamin A delays onset or delays progression of a frailty phenotype in a subject in need thereof. In some embodiments, administration of compositions comprising AKG and vitamin A delays onset or delays progression of a frailty phenotype in a subject in need thereof. In some embodiments, administration of compositions comprising AKG and vitamin D delays onset or delays progression of a frailty phenotype in a subject in need thereof. In some embodiments, administration of compositions comprising AKG and NMN delays onset or delays progression of a frailty phenotype in a subject in need thereof.

In some embodiments, the delaying onset or delaying progression of frailty comprises delaying onset or delaying progression of a frailty phenotype. In some embodiments, the frailty phenotype is selected from the group consisting of hair loss, dermatitis, kyphosis, grip strength, a gait disorder, hearing loss, cataracts, corneal opacity, eye discharge, vision loss, nasal discharge, age-related fat loss and tremors. In some embodiments, the frailty phenotype is hair loss. In some embodiments, the frailty phenotype is dermatitis. In some embodiments, the frailty phenotype is kyphosis. In some embodiments, the kyphosis is not caused by osteoporosis. In some embodiments, the kyphosis is caused by disk degeneration. In some embodiments, the frailty phenotype is grip strength. In some embodiments, the frailty phenotype is the gait disorder. In some embodiments, the frailty phenotype is hearing loss. In some embodiments, the frailty phenotype is cataracts. In some embodiments, the frailty phenotype is corneal opacity. In some embodiments, the frailty phenotype is eye discharge. In some embodiments, the frailty phenotype is vision loss. In some embodiments, the frailty phenotype is nasal discharge. In some embodiments, the frailty phenotype is age-related fat loss. In some embodiments, the frailty phenotype is tremors.

**In** some embodiments, administration of compositions comprising AKG delays onset or delays progression of hair loss in a subject in need thereof. In some embodiments, administration of compositions comprising berberine, AKG, and vitamin A delays onset or delays progression of hair loss in a subject in need thereof. In some embodiments, administration of compositions comprising AKG and vitamin A delays onset or delays progression of hair loss in a subject in need thereof. In some embodiments, administration of compositions comprising AKG and vitamin D delays onset or delays progression of hair loss in a subject in need thereof. In some embodiments, administration of compositions comprising AKG and NMN delays onset or delays progression of hair loss in a subject in need thereof.

**In** some embodiments, administration of compositions comprising AKG delays onset or delays progression of dermatitis in a subject in need thereof. In some embodiments, administration of compositions comprising berberine, AKG, and vitamin A delays onset or delays progression of dermatitis in a subject in need thereof. In some embodiments, administration of compositions comprising AKG and vitamin A delays onset or delays progression of dermatitis in a subject in need thereof. In some embodiments, administration of compositions comprising AKG and vitamin D delays onset or delays progression of dermatitis in a subject in need thereof. In some embodiments, administration of compositions comprising AKG and NMN delays onset or delays progression of dermatitis in a subject in need thereof.

**In** some embodiments, administration of compositions comprising AKG delays onset or delays progression of kyphosis in a subject in need thereof. In some embodiments, administration of compositions comprising berberine, AKG, and vitamin A delays onset or delays progression of kyphosis in a subject in need thereof. In some embodiments, administration of compositions comprising AKG and vitamin A delays onset or delays progression of kyphosis in a subject in need thereof. In some embodiments, administration of compositions comprising AKG and vitamin D delays onset or delays progression of kyphosis in a subject in need thereof. In some embodiments, administration of compositions comprising AKG and NMN delays onset or delays progression of kyphosis in a subject in need thereof.

**In** some embodiments, administration of compositions comprising AKG delays onset or delays progression of tremors in a subject in need thereof. In some embodiments, administration of compositions comprising berberine, AKG, and vitamin A delays onset or delays progression of tremors in a subject in need thereof. In some embodiments, administration of compositions comprising AKG and vitamin A delays onset or delays progression of tremors in a subject in need thereof. In some embodiments, administration of compositions comprising AKG and vitamin D delays onset or delays progression of tremors in a subject in need thereof. In some embodiments, administration of compositions comprising AKG and NMN delays onset or delays progression of tremors in a subject in need thereof.

In some embodiments, administration of compositions comprising AKG delays onset or delays progression of alopecia in a subject in need thereof. In some embodiments, administration of compositions comprising berberine, AKG, and vitamin A delays onset or delays progression of alopecia in a subject in need thereof. In some embodiments, administration of compositions comprising AKG and vitamin A delays onset or delays progression of alopecia in a subject in need thereof. In some embodiments, administration of compositions comprising AKG and vitamin D delays onset or delays progression of alopecia in a subject in need thereof. In some embodiments, administration of compositions comprising AKG and NMN delays onset or delays progression of alopecia in a subject in need thereof.

In some embodiments, administration of compositions comprising AKG delays onset or delays progression of loss of fur color in a subject in need thereof. In some embodiments, administration of compositions comprising berberine, AKG, and vitamin A delays onset or delays progression of loss of fur color in a subject in need thereof. In some embodiments, administration of compositions comprising AKG and vitamin A delays onset or delays progression of loss of fur color in a subject in need thereof. In some embodiments, administration of compositions comprising AKG and vitamin D delays onset or delays progression of loss of fur color in a subject in need thereof. In some embodiments, administration of compositions comprising AKG and NMN delays onset or delays progression of loss of fur color in a subject in need thereof.

In some embodiments, administration of compositions comprising AKG delays onset or delays progression of decreased body condition in a subject in need thereof. In some embodiments, administration of compositions comprising berberine, AKG, and vitamin A delays onset or delays progression of decreased body condition in a subject in need thereof. In some embodiments, administration of compositions comprising AKG and vitamin A delays onset or delays progression of decreased body condition in a subject in need thereof. In some embodiments, administration of compositions comprising AKG and vitamin D delays onset or delays progression of decreased body condition in a subject in need thereof. In some embodiments, administration of compositions comprising AKG and NMN delays onset or delays progression of decreased body condition in a subject in need thereof.

In some embodiments, administration of compositions comprising AKG delays onset or delays progression of piloerection in a subject in need thereof. In some embodiments, administration of compositions comprising berberine, AKG, and vitamin A delays onset or delays progression of piloerection in a subject in need thereof. In some embodiments, administration of compositions comprising AKG and vitamin A delays onset or delays progression of piloerection in a subject in need thereof. In some embodiments, administration of compositions comprising AKG and vitamin D delays onset or delays progression of piloerection in a subject in need thereof. In some embodiments, administration of compositions comprising AKG and NMN delays onset or delays progression of piloerection in a subject in need thereof.

In some embodiments, the progression of the frailty phenotype is delayed for at least 1 month after administration of the composition to the subjects. In some embodiments, the progression of the frailty phenotype is delayed for at least 2 months after administration of the composition to the subjects. In some embodiments, the progression of the frailty phenotype is delayed for at least 3 months after administration of the composition to the subjects. In some embodiments, the progression of the frailty phenotype is delayed for at least 4 months after administration of the composition to the subjects. In some embodiments, the progression of the frailty phenotype is delayed for at least 5 months after administration of the composition to the subjects. In some embodiments, the progression of the frailty phenotype is delayed for at least 6 months after administration of the composition to the subjects. In some embodiments, the progression of the frailty phenotype is delayed for at least 7 months after administration of the composition to the subjects. In some embodiments, the progression of the frailty phenotype is delayed for at least 8 months after administration of the composition to the subjects. In some embodiments, the progression of the frailty phenotype is delayed for at least 9 months after administration of the composition to the subjects. In some embodiments, the progression of the frailty phenotype is delayed for at least 10 months after administration of the composition to the subjects. In some embodiments, the progression of the frailty phenotype is delayed for at least 11 months after administration of the composition to the subjects. In some embodiments, the progression of the frailty phenotype is delayed for at least 12 months after administration of the composition to the subjects. In some embodiments, the progression of the frailty phenotype is delayed for at least 18 months after administration of the composition to the subjects. In some embodiments, the progression of the frailty phenotype is delayed for at least 24 months after administration of the composition to the subjects. In some embodiments, the progression of the frailty phenotype is delayed for at least 30 months after administration of the composition to the subjects. In some embodiments, the progression of the frailty phenotype is delayed for at least 36 months after administration of the composition to the subjects. In other embodiments, treatment of frailty comprises at reversal in one or more of the frailty phenotypes described above.

In some embodiments, the composition decreases the frailty phenotype relative to a pretreatment value of the frailty phenotype. In some embodiments, the frailty phenotype is decreased at least 5%, 10%, 15%, 20%, 25%, 33%, 40%, 45%, 50%, 66%, 75%, or 100% relative to the pretreatment value.

In some embodiments, administration of the compositions slows the rate of progression or reverse changes in biomarkers of non-human or human aging. Biomarker strategies to measure aging are currently being developed and can be employed to test the effects of aging interventions. The most prominent is the epigenetic clock, which likely measures biologic age in cells from humans (see for e.g. Chen et al. Aging, 8:1844-1865(2016), PMID 27690265), dogs (see for e.g. Thompson et al. Aging 9:1055-1068(2017), PMID 28373601), and mice (see for e.g. Petkovich et al. Cell Metab 25:954-960(2017), PMID 28380383). Other biomarker strategies include, but are not limited to, inflammatory cytokine levels, p16INK4A protein levels in specific cell populations, telomere length and levels of specific metabolites. In some embodiments, the composition modulates a biomarker of DNA methylation.

In some embodiments, the subject is a mammal. In some embodiments, the mammal is a human. In some embodiments, the mammal is a dog. In some embodiments, the mammal is livestock. In some embodiments, the livestock is selected from the group consisting of cattle, sheep, goats, swine, poultry, and equine animals.

In some embodiments, the composition is formulated into an orally administered form. In further embodiments, the orally administered form comprises a tablet, a powder, a suspension, a serum, an emulsion, a capsule, a granule, a pill, a gel, a solution, or a syrup. In some instances, the orally administered form is a sustained release dosage form. In some instances, the orally administered form is formulated into animal feed. In some instances, the orally administered form is the gel. In some embodiments, the composition is formulated into an injectable administered form. In further embodiments, the injectable administered form comprises a liquid, a suspension, or a solution. In some instances, the injectable administered form is a sustained release dosage form. In some embodiments, the composition is formulated into a topically administered form. In further embodiments, the topically administered form is a cream, a foam, a gel, a lotion, an ointment, or a serum. In some embodiments, the composition is formulated into a hair care product. In further embodiments, the hair care product is a shampoo, a conditioner, hair spray, or a moisturizer.

In some embodiments, the composition further comprises a pharmaceutically acceptable excipient. In further embodiments, the pharmaceutically acceptable excipient comprises an antiadherent, a binder, a coating, a color, disintegrant, a flavor, a glidant, a lubricant, a preservative, a sorbent, or a vehicle. In some instances, the composition further comprises silymarin.

In some embodiments, the composition is administered for a particular time period. In some embodiments, the composition is administered for a chronic treatment period, which is, for an extended period of time, including throughout the duration of the subject's life in order to ameliorate or otherwise control or limit the symptoms of the subject's disease or condition. Within the treatment period, the composition is administered on a particular time schedule. In further embodiments, the composition is administered one, two, three, or four times daily. In some embodiments, the composition is administered once daily. In some embodiments, the composition is administered twice daily. In some embodiments, the composition is administered in the morning and evening. In some embodiments, the composition is administered one, two, three, or four times weekly. In some embodiments, the composition is administered once a week. In some embodiments the composition is administered one, two, three, or four times monthly. In some embodiments, the composition is administered once a month. In some embodiments, the composition is administered for at least three months of every one year. In some embodiments, the composition is administered one month of every six months.

### Aging Phenotype

**In** certain aspects, the disclosure provides methods of treatment for reversing an age-related phenotype using the active agents or compositions thereof disclosed herein. In some aspects, the composition comprises two or more active agents selected from the group consisting of: alpha-ketoglutarate, berberine, vitamin A, vitamin D and nicotinamide mononucleotide (NMN). In certain aspects, the composition comprises berberine, α-ketoglutarate (AKG), and vitamin A. In certain aspects, the composition comprises berberine, α-ketoglutarate (AKG), and retinoic acid (RA). In certain aspects, the composition comprises berberine, α-ketoglutarate (AKG), and retinyl palmitate (RP). In certain aspects, the composition comprises AKG and vitamin A. In certain aspects, the composition comprises AKG and retinoic acid (RA). In certain aspects, the composition comprises AKG and retinyl palmitate (RP). In certain aspects, the composition comprises AKG and vitamin D. In certain aspects, the composition comprises AKG and vitamin D3. In certain aspects, the composition comprises AKG and nicotinamide mononucleotide (NMN). In some instances, the AKG in the compositions is a salt of AKG. In some instances, the AKG provided in the compositions is a calcium salt of AKG (Ca-AKG). In some embodiments, the two or more compounds comprise alpha-ketoglutarate and berberine. In yet other embodiments, the two or more compounds comprise berberine and vitamin A. In some aspects, disclosure further provides compositions comprising an active agent selected from the group consisting of: alpha-ketoglutarate, berberine, vitamin A and vitamin D. In certain aspects, the compound is AKG. In some embodiments, AKG is provided as a salt. In some embodiments, AKG is provided as a calcium salt (Ca-AKG).

In certain aspects, the disclosure provides methods for reversing an age-related phenotype in a subject in need thereof comprising administering to the subject a composition comprising a calcium salt of alpha-ketoglutarate (Ca-AKG). In another aspect, the disclosure provides methods for reversing an age-related phenotype in a subject in need thereof comprising administering to the subject a composition consisting essentially of a calcium salt of alpha-ketoglutarate (Ca-AKG). In some embodiments, calcium α-ketoglutarate can be a hydrate calcium α-ketoglutarate. In some embodiments, calcium α-ketoglutarate can be a mono-hydrate calcium α-ketoglutarate. In some embodiments, calcium α-ketoglutarate can be hemi-hydrate calcium α-ketoglutarate. In some embodiments, calcium α-ketoglutarate can be anhydrous calcium α-ketoglutarate.

In some embodiments, the Ca-AKG is administered at a dose of at least 350 mg and no greater than 2000 mg. In some instances, the Ca-AKG is administered at a dose of about 350 mg to about 2000 mg. In some instances, the Ca-AKG is administered at a dose of about 400 mg to about 2000 mg. In some instances, the Ca-AKG is administered at a dose of about 450 mg to about 2000 mg. In some instances, the Ca-AKG is administered at a dose of about 500 mg to about 2000 mg. In some instances, the Ca-AKG is administered at a dose of about 550 mg to about 2000 mg. In some instances, the Ca-AKG is administered at a dose of about 600 mg to about 2000 mg. In some instances, the Ca-AKG is administered at a dose of about 650 mg to about 2000 mg. In some instances, the Ca-AKG is administered at a dose of about 700 mg to about 2000 mg. In some instances, the Ca-AKG is administered at a dose of about 800 mg to about 2000 mg. In some instances, the Ca-AKG is administered at a dose of about 900 mg to about 2000 mg. In some instances, the Ca-AKG is administered at a dose of about 1000 mg to about 2000 mg. In some instances, the Ca-AKG is administered at a dose of about 1100 mg to about 2000 mg. In some instances, the Ca-AKG is administered at a dose of about 1200 mg to about 2000 mg. In some instances, the Ca-AKG is administered at a dose of about 1300 mg to about 2000 mg. In some instances, the Ca-AKG is administered at a dose of about 1400 mg to about 2000 mg. In some instances, the Ca-AKG is administered at a dose of about 1500 mg to about 2000 mg. In some instances, the Ca-AKG is administered at a dose of about 1600 mg to about 2000 mg. In some instances, the Ca-AKG is administered at a dose of about 1700 mg to about 2000 mg. In some instances, the Ca-AKG is administered at a dose of about 1800 mg to about 2000 mg. In some instances, the Ca-AKG is administered at a dose of about 1900 mg to about 2000 mg. In some instances, the Ca-AKG is administered at a dose of about 350 mg to about 1900 mg. In some instances, the Ca-AKG is administered at a dose of about 350 mg to about 1800 mg. In some instances, the Ca-AKG is administered at a dose of about 350 mg to about 1700 mg. In some instances, the Ca-AKG is administered at a dose of about 350 mg to about 1600 mg. In some instances, the Ca-AKG is administered at a dose of about 350 mg to about 1500 mg. In some instances, the Ca-AKG is administered at a dose of about 350 mg to about 1400 mg. In some instances, the Ca-AKG is administered at a dose of about 350 mg to about 1300 mg. In some instances, the Ca-AKG is administered at a dose of about 350 mg to about 1200 mg. In some instances, the Ca-AKG is administered at a dose of about 350 mg to about 1100 mg. In some instances, the Ca-AKG is administered at a dose of about 350 mg to about 1000 mg. In some instances, the Ca-AKG is administered at a dose of about 350 mg to about 900 mg. In some instances, the Ca-AKG is administered at a dose of about 350 mg to about 800 mg. In some instances, the Ca-AKG is administered at a dose of about 350 mg to about 700 mg. In some instances, the Ca-AKG is administered at a dose of about 350 mg to about 650 mg. In some instances, the Ca-AKG is administered at a dose of about 350 mg to about 600 mg. In some instances, the Ca-AKG is administered at a dose of about 350 mg to about 550 mg. In some instances, the Ca-AKG is administered at a dose of about 350 mg to about 500 mg. In some instances, the Ca-AKG is administered at a dose of about 350 mg to about 450 mg. In some instances, the Ca-AKG is administered at a dose of about 350 mg to about 400 mg. In some instances, the Ca-AKG is administered at a dose of at least about 350 mg. In some instances, the Ca-AKG is administered at a dose of at least about 400 mg. In some instances, the Ca-AKG is administered at a dose of at least about 450 mg. In some instances, the Ca-AKG is administered at a dose of at least about 500 mg. In some instances, the Ca-AKG is administered at a dose of at least about 550 mg. In some instances, the Ca-AKG is administered at a dose of at least about 600 mg. In some instances, the Ca-AKG is administered at a dose of at least about 650 mg. In some instances, the Ca-AKG is administered at a dose of at least about 700 mg. In some instances, the Ca-AKG is administered at a dose of at least about 750 mg. In some instances, the Ca-AKG is administered at a dose of at least about 800 mg. In some instances, the Ca-AKG is administered at a dose of at least about 900 mg. In some instances, the Ca-AKG is administered at a dose of at least about 1000 mg. In some instances, the Ca-AKG is administered at a dose of at least about 1100 mg. In some instances, the Ca-AKG is administered at a dose of at least about 1200 mg. In some instances, the Ca-AKG is administered at a dose of at least about 1300 mg. In some instances, the Ca-AKG is administered at a dose of at least about 1400 mg. In some instances, the Ca-AKG is administered at a dose of at least about 1500 mg. In some instances, the Ca-AKG is administered at a dose of at least about 1600 mg. In some instances, the Ca-AKG is administered at a dose of at least about 1700 mg. In some instances, the Ca-AKG is administered at a dose of at least about 1800 mg. In some instances, the Ca-AKG is administered at a dose of at least about 1900 mg. In some instances, the Ca-AKG is administered at a dose of at least about 2000 mg.

In some embodiments, the hydrate Ca-AKG is administered at a dose of at least 350 mg and no greater than 2000 mg. In some instances, the hydrate Ca-AKG is administered at a dose of about 350 mg to about 2000 mg. In some instances, the hydrate Ca-AKG is administered at a dose of about 400 mg to about 2000 mg. In some instances, the hydrate Ca-AKG is administered at a dose of about 450 mg to about 2000 mg. In some instances, the hydrate Ca-AKG is administered at a dose of about 500 mg to about 2000 mg. In some instances, the hydrate Ca-AKG is administered at a dose of about 550 mg to about 2000 mg. In some instances, the hydrate Ca-AKG is administered at a dose of about 600 mg to about 2000 mg. In some instances, the hydrate Ca-AKG is administered at a dose of about 650 mg to about 2000 mg. In some instances, the hydrate Ca-AKG is administered at a dose of about 700 mg to about 2000 mg. In some instances, the hydrate Ca-AKG is administered at a dose of about 800 mg to about 2000 mg. In some instances, the hydrate Ca-AKG is administered at a dose of about 900 mg to about 2000 mg. In some instances, the hydrate Ca-AKG is administered at a dose of about 1000 mg to about 2000 mg. In some instances, the hydrate Ca-AKG is administered at a dose of about 1100 mg to about 2000 mg. In some instances, the hydrate Ca-AKG is administered at a dose of about 1200 mg to about 2000 mg. In some instances, the hydrate Ca-AKG is administered at a dose of about 1300 mg to about 2000 mg. In some instances, the hydrate Ca-AKG is administered at a dose of about 1400 mg to about 2000 mg. In some instances, the hydrate Ca-AKG is administered at a dose of about 1500 mg to about 2000 mg. In some instances, the hydrate Ca-AKG is administered at a dose of about 1600 mg to about 2000 mg. In some instances, the hydrate Ca-AKG is administered at a dose of about 1700 mg to about 2000 mg. In some instances, the hydrate Ca-AKG is administered at a dose of about 1800 mg to about 2000 mg. In some instances, the hydrate Ca-AKG is administered at a dose of about 1900 mg to about 2000 mg. In some instances, the hydrate Ca-AKG is administered at a dose of about 350 mg to about 1900 mg. In some instances, the hydrate Ca-AKG is administered at a dose of about 350 mg to about 1800 mg. In some instances, the hydrate Ca-AKG is administered at a dose of about 350 mg to about 1700 mg. In some instances, the hydrate Ca-AKG is administered at a dose of about 350 mg to about 1600 mg. In some instances, the hydrate Ca-AKG is administered at a dose of about 350 mg to about 1500 mg. In some instances, the hydrate Ca-AKG is administered at a dose of about 350 mg to about 1400 mg. In some instances, the hydrate Ca-AKG is administered at a dose of about 350 mg to about 1300 mg. In some instances, the hydrate Ca-AKG is administered at a dose of about 350 mg to about 1200 mg. In some instances, the hydrate Ca-AKG is administered at a dose of about 350 mg to about 1100 mg. In some instances, the hydrate Ca-AKG is administered at a dose of about 350 mg to about 1000 mg. In some instances, the hydrate Ca-AKG is administered at a dose of about 350 mg to about 900 mg. In some instances, the hydrate Ca-AKG is administered at a dose of about 350 mg to about 800 mg. In some instances, the hydrate Ca-AKG is administered at a dose of about 350 mg to about 700 mg. In some instances, the hydrate Ca-AKG is administered at a dose of about 350 mg to about 650 mg. In some instances, the hydrate Ca-AKG is administered at a dose of about 350 mg to about 600 mg. In some instances, the hydrate Ca-AKG is administered at a dose of about 350 mg to about 550 mg. In some instances, the hydrate Ca-AKG is administered at a dose of about 350 mg to about 500 mg. In some instances, the hydrate Ca-AKG is administered at a dose of about 350 mg to about 450 mg. In some instances, the hydrate Ca-AKG is administered at a dose of about 350 mg to about 400 mg. In some instances, the hydrate Ca-AKG is administered at a dose of at least about 350 mg. In some instances, the hydrate Ca-AKG is administered at a dose of at least about 400 mg. In some instances, the hydrate Ca-AKG is administered at a dose of at least about 450 mg. In some instances, the hydrate Ca-AKG is administered at a dose of at least about 500 mg. In some instances, the hydrate Ca-AKG is administered at a dose of at least about 550 mg. In some instances, the hydrate Ca-AKG is administered at a dose of at least about 600 mg. In some instances, the hydrate Ca-AKG is administered at a dose of at least about 650 mg. In some instances, the hydrate Ca-AKG is administered at a dose of at least about 700 mg. In some instances, the hydrate Ca-AKG is administered at a dose of at least about 750 mg. In some instances, the hydrate Ca-AKG is administered at a dose of at least about 800 mg. In some instances, the hydrate Ca-AKG is administered at a dose of at least about 900 mg. In some instances, the hydrate Ca-AKG is administered at a dose of at least about 1000 mg. In some instances, the hydrate Ca-AKG is administered at a dose of at least about 1100 mg. In some instances, the hydrate Ca-AKG is administered at a dose of at least about 1200 mg. In some instances, the hydrate Ca-AKG is administered at a dose of at least about 1300 mg. In some instances, the hydrate Ca-AKG is administered at a dose of at least about 1400 mg. In some instances, the hydrate Ca-AKG is administered at a dose of at least about 1500 mg. In some instances, the hydrate Ca-AKG is administered at a dose of at least about 1600 mg. In some instances, the hydrate Ca-AKG is administered at a dose of at least about 1700 mg. In some instances, the hydrate Ca-AKG is administered at a dose of at least about 1800 mg. In some instances, the hydrate Ca-AKG is administered at a dose of at least about 1900 mg. In some instances, the hydrate Ca-AKG is administered at a dose of at least about 2000 mg.

In some embodiments, the mono-hydrate Ca-AKG is administered at a dose of at least 350 mg and no greater than 2000 mg. In some instances, the mono-hydrate Ca-AKG is administered at a dose of about 350 mg to about 2000 mg. In some instances, the mono-hydrate Ca-AKG is administered at a dose of about 400 mg to about 2000 mg. In some instances, the mono-hydrate Ca-AKG is administered at a dose of about 450 mg to about 2000 mg. In some instances, the mono-hydrate Ca-AKG is administered at a dose of about 500 mg to about 2000 mg. In some instances, the mono-hydrate Ca-AKG is administered at a dose of about 550 mg to about 2000 mg. In some instances, the mono-hydrate Ca-AKG is administered at a dose of about 600 mg to about 2000 mg. In some instances, the mono-hydrate Ca-AKG is administered at a dose of about 650 mg to about 2000 mg. In some instances, the mono-hydrate Ca-AKG is administered at a dose of about 700 mg to about 2000 mg. In some instances, the mono-hydrate Ca-AKG is administered at a dose of about 800 mg to about 2000 mg. In some instances, the mono-hydrate Ca-AKG is administered at a dose of about 900 mg to about 2000 mg. In some instances, the mono-hydrate Ca-AKG is administered at a dose of about 1000 mg to about 2000 mg. In some instances, the mono-hydrate Ca-AKG is administered at a dose of about 1100 mg to about 2000 mg. In some instances, the mono-hydrate Ca-AKG is administered at a dose of about 1200 mg to about 2000 mg. In some instances, the mono-hydrate Ca-AKG is administered at a dose of about 1300 mg to about 2000 mg. In some instances, the mono-hydrate Ca-AKG is administered at a dose of about 1400 mg to about 2000 mg. In some instances, the mono-hydrate Ca-AKG is administered at a dose of about 1500 mg to about 2000 mg. In some instances, the mono-hydrate Ca-AKG is administered at a dose of about 1600 mg to about 2000 mg. In some instances, the mono-hydrate Ca-AKG is administered at a dose of about 1700 mg to about 2000 mg. In some instances, the mono-hydrate Ca-AKG is administered at a dose of about 1800 mg to about 2000 mg. In some instances, the mono-hydrate Ca-AKG is administered at a dose of about 1900 mg to about 2000 mg. In some instances, the mono-hydrate Ca-AKG is administered at a dose of about 350 mg to about 1900 mg. In some instances, the mono-hydrate Ca-AKG is administered at a dose of about 350 mg to about 1800 mg. In some instances, the mono-hydrate Ca-AKG is administered at a dose of about 350 mg to about 1700 mg. In some instances, the mono-hydrate Ca-AKG is administered at a dose of about 350 mg to about 1600 mg. In some instances, the mono-hydrate Ca-AKG is administered at a dose of about 350 mg to about 1500 mg. In some instances, the mono-hydrate Ca-AKG is administered at a dose of about 350 mg to about 1400 mg. In some instances, the mono-hydrate Ca-AKG is administered at a dose of about 350 mg to about 1300 mg. In some instances, the mono-hydrate Ca-AKG is administered at a dose of about 350 mg to about 1200 mg. In some instances, the mono-hydrate Ca-AKG is administered at a dose of about 350 mg to about 1100 mg. In some instances, the mono-hydrate Ca-AKG is administered at a dose of about 350 mg to about 1000 mg. In some instances, the mono-hydrate Ca-AKG is administered at a dose of about 350 mg to about 900 mg. In some instances, the mono-hydrate Ca-AKG is administered at a dose of about 350 mg to about 800 mg. In some instances, the mono-hydrate Ca-AKG is administered at a dose of about 350 mg to about 700 mg. In some instances, the mono-hydrate Ca-AKG is administered at a dose of about 350 mg to about 650 mg. In some instances, the mono-hydrate Ca-AKG is administered at a dose of about 350 mg to about 600 mg. In some instances, the mono-hydrate Ca-AKG is administered at a dose of about 350 mg to about 550 mg. In some instances, the mono-hydrate Ca-AKG is administered at a dose of about 350 mg to about 500 mg. In some instances, the mono-hydrate Ca-AKG is administered at a dose of about 350 mg to about 450 mg. In some instances, the mono-hydrate Ca-AKG is administered at a dose of about 350 mg to about 400 mg. In some instances, the mono-hydrate Ca-AKG is administered at a dose of at least about 350 mg. In some instances, the mono-hydrate Ca-AKG is administered at a dose of at least about 400 mg. In some instances, the mono-hydrate Ca-AKG is administered at a dose of at least about 450 mg. In some instances, the mono-hydrate Ca-AKG is administered at a dose of at least about 500 mg. In some instances, the mono-hydrate Ca-AKG is administered at a dose of at least about 550 mg. In some instances, the mono-hydrate Ca-AKG is administered at a dose of at least about 600 mg. In some instances, the mono-hydrate Ca-AKG is administered at a dose of at least about 650 mg. In some instances, the mono-hydrate Ca-AKG is administered at a dose of at least about 700 mg. In some instances, the mono-hydrate Ca-AKG is administered at a dose of at least about 750 mg. In some instances, the mono-hydrate Ca-AKG is administered at a dose of at least about 800 mg. In some instances, the mono-hydrate Ca-AKG is administered at a dose of at least about 900 mg. In some instances, the mono-hydrate Ca-AKG is administered at a dose of at least about 1000 mg. In some instances, the mono-hydrate Ca-AKG is administered at a dose of at least about 1100 mg. In some instances, the mono-hydrate Ca-AKG is administered at a dose of at least about 1200 mg. In some instances, the mono-hydrate Ca-AKG is administered at a dose of at least about 1300 mg. In some instances, the mono-hydrate Ca-AKG is administered at a dose of at least about 1400 mg. In some instances, the mono-hydrate Ca-AKG is administered at a dose of at least about 1500 mg. In some instances, the mono-hydrate Ca-AKG is administered at a dose of at least about 1600 mg. In some instances, the mono-hydrate Ca-AKG is administered at a dose of at least about 1700 mg. In some instances, the mono-hydrate Ca-AKG is administered at a dose of at least about 1800 mg. In some instances, the mono-hydrate Ca-AKG is administered at a dose of at least about 1900 mg. In some instances, the mono-hydrate Ca-AKG is administered at a dose of at least about 2000 mg.

In some embodiments, the hemi-hydrate Ca-AKG is administered at a dose of at least 350 mg and no greater than 2000 mg. In some instances, the hemi-hydrate Ca-AKG is administered at a dose of about 350 mg to about 2000 mg. In some instances, the hemi-hydrate Ca-AKG is administered at a dose of about 400 mg to about 2000 mg. In some instances, the hemi-hydrate Ca-AKG is administered at a dose of about 450 mg to about 2000 mg. In some instances, the hemi-hydrate Ca-AKG is administered at a dose of about 500 mg to about 2000 mg. In some instances, the hemi-hydrate Ca-AKG is administered at a dose of about 550 mg to about 2000 mg. In some instances, the hemi-hydrate Ca-AKG is administered at a dose of about 600 mg to about 2000 mg. In some instances, the hemi-hydrate Ca-AKG is administered at a dose of about 650 mg to about 2000 mg. In some instances, the hemi-hydrate Ca-AKG is administered at a dose of about 700 mg to about 2000 mg. In some instances, the hemi-hydrate Ca-AKG is administered at a dose of about 800 mg to about 2000 mg. In some instances, the hemi-hydrate Ca-AKG is administered at a dose of about 900 mg to about 2000 mg. In some instances, the hemi-hydrate Ca-AKG is administered at a dose of about 1000 mg to about 2000 mg. In some instances, the hemi-hydrate Ca-AKG is administered at a dose of about 1100 mg to about 2000 mg. In some instances, the hemi-hydrate Ca-AKG is administered at a dose of about 1200 mg to about 2000 mg. In some instances, the hemi-hydrate Ca-AKG is administered at a dose of about 1300 mg to about 2000 mg. In some instances, the hemi-hydrate Ca-AKG is administered at a dose of about 1400 mg to about 2000 mg. In some instances, the hemi-hydrate Ca-AKG is administered at a dose of about 1500 mg to about 2000 mg. In some instances, the hemi-hydrate Ca-AKG is administered at a dose of about 1600 mg to about 2000 mg. In some instances, the hemi-hydrate Ca-AKG is administered at a dose of about 1700 mg to about 2000 mg. In some instances, the hemi-hydrate Ca-AKG is administered at a dose of about 1800 mg to about 2000 mg. In some instances, the hemi-hydrate Ca-AKG is administered at a dose of about 1900 mg to about 2000 mg. In some instances, the hemi-hydrate Ca-AKG is administered at a dose of about 350 mg to about 1900 mg. In some instances, the hemi-hydrate Ca-AKG is administered at a dose of about 350 mg to about 1800 mg. In some instances, the hemi-hydrate Ca-AKG is administered at a dose of about 350 mg to about 1700 mg. In some instances, the hemi-hydrate Ca-AKG is administered at a dose of about 350 mg to about 1600 mg. In some instances, the hemi-hydrate Ca-AKG is administered at a dose of about 350 mg to about 1500 mg. In some instances, the hemi-hydrate Ca-AKG is administered at a dose of about 350 mg to about 1400 mg. In some instances, the hemi-hydrate Ca-AKG is administered at a dose of about 350 mg to about 1300 mg. In some instances, the hemi-hydrate Ca-AKG is administered at a dose of about 350 mg to about 1200 mg. In some instances, the hemi-hydrate Ca-AKG is administered at a dose of about 350 mg to about 1100 mg. In some instances, the hemi-hydrate Ca-AKG is administered at a dose of about 350 mg to about 1000 mg. In some instances, the hemi-hydrate Ca-AKG is administered at a dose of about 350 mg to about 900 mg. In some instances, the hemi-hydrate Ca-AKG is administered at a dose of about 350 mg to about 800 mg. In some instances, the hemi-hydrate Ca-AKG is administered at a dose of about 350 mg to about 700 mg. In some instances, the hemi-hydrate Ca-AKG is administered at a dose of about 350 mg to about 650 mg. In some instances, the hemi-hydrate Ca-AKG is administered at a dose of about 350 mg to about 600 mg. In some instances, the hemi-hydrate Ca-AKG is administered at a dose of about 350 mg to about 550 mg. In some instances, the hemi-hydrate Ca-AKG is administered at a dose of about 350 mg to about 500 mg. In some instances, the hemi-hydrate Ca-AKG is administered at a dose of about 350 mg to about 450 mg. In some instances, the hemi-hydrate Ca-AKG is administered at a dose of about 350 mg to about 400 mg. In some instances, the hemi-hydrate Ca-AKG is administered at a dose of at least about 350 mg. In some instances, the hemi-hydrate Ca-AKG is administered at a dose of at least about 400 mg. In some instances, the hemi-hydrate Ca-AKG is administered at a dose of at least about 450 mg. In some instances, the hemi-hydrate Ca-AKG is administered at a dose of at least about 500 mg. In some instances, the hemi-hydrate Ca-AKG is administered at a dose of at least about 550 mg. In some instances, the hemi-hydrate Ca-AKG is administered at a dose of at least about 600 mg. In some instances, the hemi-hydrate Ca-AKG is administered at a dose of at least about 650 mg. In some instances, the hemi-hydrate Ca-AKG is administered at a dose of at least about 700 mg. In some instances, the hemi-hydrate Ca-AKG is administered at a dose of at least about 750 mg. In some instances, the hemi-hydrate Ca-AKG is administered at a dose of at least about 800 mg. In some instances, the hemi-hydrate Ca-AKG is administered at a dose of at least about 900 mg. In some instances, the hemi-hydrate Ca-AKG is administered at a dose of at least about 1000 mg. In some instances, the hemi-hydrate Ca-AKG is administered at a dose of at least about 1100 mg. In some instances, the hemi-hydrate Ca-AKG is administered at a dose of at least about 1200 mg. In some instances, the hemi-hydrate Ca-AKG is administered at a dose of at least about 1300 mg. In some instances, the hemi-hydrate Ca-AKG is administered at a dose of at least about 1400 mg. In some instances, the hemi-hydrate Ca-AKG is administered at a dose of at least about 1500 mg. In some instances, the hemi-hydrate Ca-AKG is administered at a dose of at least about 1600 mg. In some instances, the hemi-hydrate Ca-AKG is administered at a dose of at least about 1700 mg. In some instances, the hemi-hydrate Ca-AKG is administered at a dose of at least about 1800 mg. In some instances, the hemi-hydrate Ca-AKG is administered at a dose of at least about 1900 mg. In some instances, the hemi-hydrate Ca-AKG is administered at a dose of at least about 2000 mg.

In some embodiments, the anhydrous Ca-AKG is administered at a dose of at least 350 mg and no greater than 2000 mg. In some instances, the anhydrous Ca-AKG is administered at a dose of about 350 mg to about 2000 mg. In some instances, the anhydrous Ca-AKG is administered at a dose of about 400 mg to about 2000 mg. In some instances, the anhydrous Ca-AKG is administered at a dose of about 450 mg to about 2000 mg. In some instances, the anhydrous Ca-AKG is administered at a dose of about 500 mg to about 2000 mg. In some instances, the anhydrous Ca-AKG is administered at a dose of about 550 mg to about 2000 mg. In some instances, the anhydrous Ca-AKG is administered at a dose of about 600 mg to about 2000 mg. In some instances, the anhydrous Ca-AKG is administered at a dose of about 650 mg to about 2000 mg. In some instances, the anhydrous Ca-AKG is administered at a dose of about 700 mg to about 2000 mg. In some instances, the anhydrous Ca-AKG is administered at a dose of about 800 mg to about 2000 mg. In some instances, the anhydrous Ca-AKG is administered at a dose of about 900 mg to about 2000 mg. In some instances, the anhydrous Ca-AKG is administered at a dose of about 1000 mg to about 2000 mg. In some instances, the anhydrous Ca-AKG is administered at a dose of about 1100 mg to about 2000 mg. In some instances, the anhydrous Ca-AKG is administered at a dose of about 1200 mg to about 2000 mg. In some instances, the anhydrous Ca-AKG is administered at a dose of about 1300 mg to about 2000 mg. In some instances, the anhydrous Ca-AKG is administered at a dose of about 1400 mg to about 2000 mg. In some instances, the anhydrous Ca-AKG is administered at a dose of about 1500 mg to about 2000 mg. In some instances, the anhydrous Ca-AKG is administered at a dose of about 1600 mg to about 2000 mg. In some instances, the anhydrous Ca-AKG is administered at a dose of about 1700 mg to about 2000 mg. In some instances, the anhydrous Ca-AKG is administered at a dose of about 1800 mg to about 2000 mg. In some instances, the anhydrous Ca-AKG is administered at a dose of about 1900 mg to about 2000 mg. In some instances, the anhydrous Ca-AKG is administered at a dose of about 350 mg to about 1900 mg. In some instances, the anhydrous Ca-AKG is administered at a dose of about 350 mg to about 1800 mg. In some instances, the anhydrous Ca-AKG is administered at a dose of about 350 mg to about 1700 mg. In some instances, the anhydrous Ca-AKG is administered at a dose of about 350 mg to about 1600 mg. In some instances, the anhydrous Ca-AKG is administered at a dose of about 350 mg to about 1500 mg. In some instances, the anhydrous Ca-AKG is administered at a dose of about 350 mg to about 1400 mg. In some instances, the anhydrous Ca-AKG is administered at a dose of about 350 mg to about 1300 mg. In some instances, the anhydrous Ca-AKG is administered at a dose of about 350 mg to about 1200 mg. In some instances, the anhydrous Ca-AKG is administered at a dose of about 350 mg to about 1100 mg. In some instances, the anhydrous Ca-AKG is administered at a dose of about 350 mg to about 1000 mg. In some instances, the anhydrous Ca-AKG is administered at a dose of about 350 mg to about 900 mg. In some instances, the anhydrous Ca-AKG is administered at a dose of about 350 mg to about 800 mg. In some instances, the anhydrous Ca-AKG is administered at a dose of about 350 mg to about 700 mg. In some instances, the anhydrous Ca-AKG is administered at a dose of about 350 mg to about 650 mg. In some instances, the anhydrous Ca-AKG is administered at a dose of about 350 mg to about 600 mg. In some instances, the anhydrous Ca-AKG is administered at a dose of about 350 mg to about 550 mg. In some instances, the anhydrous Ca-AKG is administered at a dose of about 350 mg to about 500 mg. In some instances, the anhydrous Ca-AKG is administered at a dose of about 350 mg to about 450 mg. In some instances, the anhydrous Ca-AKG is administered at a dose of about 350 mg to about 400 mg. In some instances, the anhydrous Ca-AKG is administered at a dose of at least about 350 mg. In some instances, the anhydrous Ca-AKG is administered at a dose of at least about 400 mg. In some instances, the anhydrous Ca-AKG is administered at a dose of at least about 450 mg. In some instances, the anhydrous Ca-AKG is administered at a dose of at least about 500 mg. In some instances, the anhydrous Ca-AKG is administered at a dose of at least about 550 mg. In some instances, the anhydrous Ca-AKG is administered at a dose of at least about 600 mg. In some instances, the anhydrous Ca-AKG is administered at a dose of at least about 650 mg. In some instances, the anhydrous Ca-AKG is administered at a dose of at least about 700 mg. In some instances, the anhydrous Ca-AKG is administered at a dose of at least about 750 mg. In some instances, the anhydrous Ca-AKG is administered at a dose of at least about 800 mg. In some instances, the anhydrous Ca-AKG is administered at a dose of at least about 900 mg. In some instances, the anhydrous Ca-AKG is administered at a dose of at least about 1000 mg. In some instances, the anhydrous Ca-AKG is administered at a dose of at least about 1100 mg. In some instances, the anhydrous Ca-AKG is administered at a dose of at least about 1200 mg. In some instances, the anhydrous Ca-AKG is administered at a dose of at least about 1300 mg. In some instances, the anhydrous Ca-AKG is administered at a dose of at least about 1400 mg. In some instances, the anhydrous Ca-AKG is administered at a dose of at least about 1500 mg. In some instances, the anhydrous Ca-AKG is administered at a dose of at least about 1600 mg. In some instances, the anhydrous Ca-AKG is administered at a dose of at least about 1700 mg. In some instances, the anhydrous Ca-AKG is administered at a dose of at least about 1800 mg. In some instances, the anhydrous Ca-AKG is administered at a dose of at least about 1900 mg. In some instances, the anhydrous Ca-AKG is administered at a dose of at least about 2000 mg.

In some embodiments, administration of compositions comprising AKG reverses an age-related phenotype in a subject in need thereof. In some embodiments, administration of compositions comprising berberine, AKG, and vitamin A reverses an age-related phenotype in a subject in need thereof. In some embodiments, administration of compositions comprising AKG and vitamin A reverses an age-related phenotype in a subject in need thereof. In some embodiments, administration of compositions comprising AKG and vitamin D reverses an age-related phenotype in a subject in need thereof. In some embodiments, administration of compositions comprising AKG and NMN reverses an age-related phenotype in a subject in need thereof.

In some embodiments, the age-related phenotype is selected from the group consisting of decreased stem cell production, increased cell senescence, increased inflammation, elevated cholesterol, elevated A1C levels, reduced mobility, elevated high density lipoprotein, elevated blood pressure, graying hair, hair loss, hair regeneration, increased abdominal adiposity, decreased left ventricular diastolic function, elevated interleukin-6, impaired cognition, and modulation of DNA methylation. In some instances, the age-related phenotype is decreased stem cell production. In some instances, the age-related phenotype is increased cell senescence. In some instances, the age-related phenotype is increased inflammation. In some instances, the age-related phenotype is elevated cholesterol. In some instances, the age-related phenotype is elevated A1C levels. In some instances, the age-related phenotype is reduced mobility. In some instances, the age-related phenotype is elevated high density lipoprotein. In some instances, the age-related phenotype is elevated blood pressure. In some instances, the age-related phenotype is graying hair. In some instances, the age-related phenotype is hair loss. In some instances, the age-related phenotype is increased abdominal adiposity. In some instances, the age-related phenotype is decreased left ventricular diastolic function. In some instances, the age-related phenotype is elevated interleukin-6. In some instances, the age-related phenotype is impaired cognition. In some instances, the age-related phenotype is modulation of DNA methylation.

In some embodiments, administration of compositions comprising AKG delays or reverses cell senescence in a subject in need thereof. In some embodiments, administration of compositions comprising berberine, AKG, and vitamin A delays or reverses cell senescence in a subject in need thereof. In some embodiments, administration of compositions comprising AKG and vitamin A delays or reverses cell senescence in a subject in need thereof. In some embodiments, administration of compositions comprising AKG and vitamin D delays or reverses cell senescence in a subject in need thereof. In some embodiments, administration of compositions comprising AKG and NMN delays or reverses cell senescence in a subject in need thereof.

In some embodiments, administration of compositions comprising AKG delays or reverses graying hair in a subject in need thereof. In some embodiments, administration of compositions comprising berberine, AKG, and vitamin A delays or reverses graying hair in a subject in need thereof. In some embodiments, administration of compositions comprising AKG and vitamin A delays or reverses graying hair in a subject in need thereof. In some embodiments, administration of compositions comprising AKG and vitamin D delays or reverses graying hair in a subject in need thereof. In some embodiments, administration of compositions comprising AKG and NMN delays or reverses graying hair in a subject in need thereof.

In some embodiments, administration of compositions comprising AKG delays or reverses hair loss in a subject in need thereof. In some embodiments, administration of compositions comprising berberine, AKG, and vitamin A delays or reverses hair loss in a subject in need thereof. In some embodiments, administration of compositions comprising AKG and vitamin A delays or reverses hair loss in a subject in need thereof. In some embodiments, administration of compositions comprising AKG and vitamin D delays or reverses hair loss in a subject in need thereof. In some embodiments, administration of compositions comprising AKG and NMN delays or reverses hair loss in a subject in need thereof.

In some embodiments, administration of compositions comprising AKG promotes hair regeneration in a subject in need thereof. In some embodiments, administration of compositions comprising berberine, AKG, and vitamin A promotes hair regeneration in a subject in need thereof. In some embodiments, administration of compositions comprising AKG and vitamin A promotes hair regeneration in a subject in need thereof. In some embodiments, administration of compositions comprising AKG and vitamin D promotes hair regeneration in a subject in need thereof. In some embodiments, administration of compositions comprising AKG and NMN promotes hair regeneration in a subject in need thereof.

In some embodiments, the composition decreases the age-related phenotype relative to a pretreatment value of the age-related phenotype. In some embodiments, the age-related phenotype is decreased at least 5%, 10%, 15%, 20%, 25%, 33%, 40%, 45%, 50%, 66%, 75%, or 100% relative to the pretreatment value.

In some embodiments, administration of the compositions slows the rate of progression or reverse changes in biomarkers of non-human or human aging. Biomarker strategies to measure aging are currently being developed and can be employed to test the effects of aging interventions. The most prominent is the epigenetic clock, which likely measures biologic age in cells from humans (see for e.g. Chen et al. Aging, 8:1844-1865(2016), PMID 27690265), dogs (see for e.g. Thompson et al. Aging 9:1055-1068(2017), PMID 28373601), and mice (see for e.g. Petkovich et al. Cell Metab 25:954-960(2017), PMID 28380383). Other biomarker strategies include, but are not limited to, inflammatory cytokine levels, p16INK4A protein levels in specific cell populations, telomere length and levels of specific metabolites. In some embodiments, the composition modulates a biomarker of DNA methylation.

In some embodiments, the subject is a mammal. In some embodiments, the mammal is a human. In some embodiments, the mammal is a dog. In some embodiments, the mammal is livestock. In some embodiments, the livestock is selected from the group consisting of cattle, sheep, goats, swine, poultry, and equine animals.

In some embodiments, the composition is formulated into an orally administered form. In further embodiments, the orally administered form comprises a tablet, a powder, a suspension, a serum, an emulsion, a capsule, a granule, a pill, a gel, a solution, or a syrup. In some instances, the orally administered form is a sustained release dosage form. In some instances, the orally administered form is formulated into animal feed. In some instances, the orally administered form is the gel. In some embodiments, the composition is formulated into an injectable administered form. In further embodiments, the injectable administered form comprises a liquid, a suspension, or a solution. In some instances, the injectable administered form is a sustained release dosage form. In some embodiments, the composition is formulated into a topically administered form. In further embodiments, the topically administered form is a cream, a foam, a gel, a lotion, an ointment, or a serum. In some embodiments, the composition is formulated into a hair care product. In further embodiments, the hair care product is a shampoo, a conditioner, hair spray, or a moisturizer.

In some embodiments, the composition further comprises a pharmaceutically acceptable excipient. In further embodiments, the pharmaceutically acceptable excipient comprises an antiadherent, a binder, a coating, a color, disintegrant, a flavor, a glidant, a lubricant, a preservative, a sorbent, or a vehicle. In some instances, the composition further comprises silymarin.

In some embodiments, the composition is administered for a particular time period. In some embodiments, the composition is administered for a chronic treatment period, which is, for an extended period of time, including throughout the duration of the subject's life in order to ameliorate or otherwise control or limit the symptoms of the subject's disease or condition. Within the treatment period, the composition is administered on a particular time schedule. In further embodiments, the composition is administered one, two, three, or four times daily. In some embodiments, the composition is administered once daily. In some embodiments, the composition is administered twice daily. In some embodiments, the composition is administered in the morning and evening. In some embodiments, the composition is administered one, two, three, or four times weekly. In some embodiments, the composition is administered once a week. In some embodiments the composition is administered one, two, three, or four times monthly. In some embodiments, the composition is administered once a month. In some embodiments, the composition is administered for at least three months of every one year. In some embodiments, the composition is administered one month of every six months.

### Healthspan

In an aspect, the disclosure provides methods of treatment for extending healthspan using the active agents or compositions thereof disclosed herein. Healthspan refers to the period of time during which an individual meets one or more selected measures of health. An increase in healthspan refers to an extension in the period of health, according to such measures, as compared to the period of health in a control population. Examples of selected measures of health that are evaluated in a human population to assess healthspan include one or more age-related phenotypes such as energetics/metabolism (e.g. elevated insulin, insulin resistance, elevated fasting blood glucose + GTT, elevated Hb A1c, adiponectin, elevated DEXA/abdominal adiposity, increased IGF-I, decreased T3, elevated low-density lipoprotein, decreased high-density lipoprotein, elevated triglycerides), skeletal muscle function (e.g. decreased hand grip strength, decreased mobility), cardiopulmonary function (e.g. decreased VO2max, elevated blood pressure, decreased pulse wave velocity, intima media thickness, decreased left ventricular diastolic function, increased left ventricular diastolic pressure), inflammation and immune function (e.g. decreased lymphocyte number, decreased lymphoid/myeloid ratio, elevated CRP, elevated IL-6, elevated TNF-α), sensory function (e.g. decreased visual acuity, decreased nerve conduction velocity), cognition (e.g. decreased score on cognitive function tests like the MMSE/AMTS/GPAC, impaired activity via fMRIs), cellular senescence (e.g. graying hair), and pathology (e.g. renal, cardiac, pulmonary, breast, or prostate tissue evaluation to evaluate age-related tissue hypertrophy or dysplasia).

Examples of non-human animals used for assessment of lifespan-extension or healthspan interventions include *C*. *elegans, D. melanogaster,* and *M. musculus.* Use of *D. melanogaster* or *M. musculus* to evaluate lifespan or healthspan interventions are found in, for e.g. Bauer et al. Proc Natl Acad Sci U S A. 101:12980-5 (2004) and Selman et al. FASEB J 22:807-18 (2008).

**In** some embodiments, the disclosure provides methods of extending healthspan using the compositions disclosed herein. In some aspects, the composition comprises two or more active agents selected from the group consisting of: alpha-ketoglutarate, berberine, vitamin A, vitamin D and nicotinamide mononucleotide (NMN). In certain aspects, the composition comprises berberine, α-ketoglutarate (AKG), and vitamin A. In certain aspects, the composition comprises berberine, α-ketoglutarate (AKG), and retinoic acid (RA). In certain aspects, the composition comprises berberine, α-ketoglutarate (AKG), and retinyl palmitate (RP). In certain aspects, the composition comprises AKG and vitamin A. In certain aspects, the composition comprises AKG and retinoic acid (RA). In certain aspects, the composition comprises AKG and retinyl palmitate (RP). In certain aspects, the composition comprises AKG and vitamin D. In certain aspects, the composition comprises AKG and vitamin D3. In certain aspects, the composition comprises AKG and nicotinamide mononucleotide (NMN). In some instances, the AKG in the compositions is a salt of AKG. In some instances, the AKG provided in the compositions is a calcium salt of AKG (Ca-AKG). In some embodiments, the two or more compounds comprise alpha-ketoglutarate and berberine. In yet other embodiments, the two or more compounds comprise berberine and vitamin A. In some aspects, disclosure further provides compositions comprising an active agent selected from the group consisting of: alpha-ketoglutarate, berberine, vitamin A and vitamin D. In certain aspects, the compound is AKG. In some embodiments, AKG is provided as a salt. In some embodiments, AKG is provided as a calcium salt (Ca-AKG).

In certain aspects, the disclosure provides methods of extending healthspan in a subject in need thereof comprising administering to the subject a composition comprising a calcium salt of alpha-ketoglutarate (Ca-AKG). In another aspect, the disclosure provides methods of extending healthspan in a subject in need thereof comprising administering to the subject a composition consisting essentially of a calcium salt of alpha-ketoglutarate (Ca-AKG). In some embodiments, calcium α-ketoglutarate can be a hydrate calcium α-ketoglutarate. In some embodiments, calcium α-ketoglutarate can be a mono-hydrate calcium α-ketoglutarate. In some embodiments, calcium α-ketoglutarate can be hemi-hydrate calcium α-ketoglutarate. In some embodiments, calcium α-ketoglutarate can be anhydrous calcium α-ketoglutarate.

In some embodiments, the Ca-AKG is administered at a dose of at least 350 mg and no greater than 2000 mg. In some instances, the Ca-AKG is administered at a dose of about 350 mg to about 2000 mg. In some instances, the Ca-AKG is administered at a dose of about 400 mg to about 2000 mg. In some instances, the Ca-AKG is administered at a dose of about 450 mg to about 2000 mg. In some instances, the Ca-AKG is administered at a dose of about 500 mg to about 2000 mg. In some instances, the Ca-AKG is administered at a dose of about 550 mg to about 2000 mg. In some instances, the Ca-AKG is administered at a dose of about 600 mg to about 2000 mg. In some instances, the Ca-AKG is administered at a dose of about 650 mg to about 2000 mg. In some instances, the Ca-AKG is administered at a dose of about 700 mg to about 2000 mg. In some instances, the Ca-AKG is administered at a dose of about 800 mg to about 2000 mg. In some instances, the Ca-AKG is administered at a dose of about 900 mg to about 2000 mg. In some instances, the Ca-AKG is administered at a dose of about 1000 mg to about 2000 mg. In some instances, the Ca-AKG is administered at a dose of about 1100 mg to about 2000 mg. In some instances, the Ca-AKG is administered at a dose of about 1200 mg to about 2000 mg. In some instances, the Ca-AKG is administered at a dose of about 1300 mg to about 2000 mg. In some instances, the Ca-AKG is administered at a dose of about 1400 mg to about 2000 mg. In some instances, the Ca-AKG is administered at a dose of about 1500 mg to about 2000 mg. In some instances, the Ca-AKG is administered at a dose of about 1600 mg to about 2000 mg. In some instances, the Ca-AKG is administered at a dose of about 1700 mg to about 2000 mg. In some instances, the Ca-AKG is administered at a dose of about 1800 mg to about 2000 mg. In some instances, the Ca-AKG is administered at a dose of about 1900 mg to about 2000 mg. In some instances, the Ca-AKG is administered at a dose of about 350 mg to about 1900 mg. In some instances, the Ca-AKG is administered at a dose of about 350 mg to about 1800 mg. In some instances, the Ca-AKG is administered at a dose of about 350 mg to about 1700 mg. In some instances, the Ca-AKG is administered at a dose of about 350 mg to about 1600 mg. In some instances, the Ca-AKG is administered at a dose of about 350 mg to about 1500 mg. In some instances, the Ca-AKG is administered at a dose of about 350 mg to about 1400 mg. In some instances, the Ca-AKG is administered at a dose of about 350 mg to about 1300 mg. In some instances, the Ca-AKG is administered at a dose of about 350 mg to about 1200 mg. In some instances, the Ca-AKG is administered at a dose of about 350 mg to about 1100 mg. In some instances, the Ca-AKG is administered at a dose of about 350 mg to about 1000 mg. In some instances, the Ca-AKG is administered at a dose of about 350 mg to about 900 mg. In some instances, the Ca-AKG is administered at a dose of about 350 mg to about 800 mg. In some instances, the Ca-AKG is administered at a dose of about 350 mg to about 700 mg. In some instances, the Ca-AKG is administered at a dose of about 350 mg to about 650 mg. In some instances, the Ca-AKG is administered at a dose of about 350 mg to about 600 mg. In some instances, the Ca-AKG is administered at a dose of about 350 mg to about 550 mg. In some instances, the Ca-AKG is administered at a dose of about 350 mg to about 500 mg. In some instances, the Ca-AKG is administered at a dose of about 350 mg to about 450 mg. In some instances, the Ca-AKG is administered at a dose of about 350 mg to about 400 mg. In some instances, the Ca-AKG is administered at a dose of at least about 350 mg. In some instances, the Ca-AKG is administered at a dose of at least about 400 mg. In some instances, the Ca-AKG is administered at a dose of at least about 450 mg. In some instances, the Ca-AKG is administered at a dose of at least about 500 mg. In some instances, the Ca-AKG is administered at a dose of at least about 550 mg. In some instances, the Ca-AKG is administered at a dose of at least about 600 mg. In some instances, the Ca-AKG is administered at a dose of at least about 650 mg. In some instances, the Ca-AKG is administered at a dose of at least about 700 mg. In some instances, the Ca-AKG is administered at a dose of at least about 750 mg. In some instances, the Ca-AKG is administered at a dose of at least about 800 mg. In some instances, the Ca-AKG is administered at a dose of at least about 900 mg. In some instances, the Ca-AKG is administered at a dose of at least about 1000 mg. In some instances, the Ca-AKG is administered at a dose of at least about 1100 mg. In some instances, the Ca-AKG is administered at a dose of at least about 1200 mg. In some instances, the Ca-AKG is administered at a dose of at least about 1300 mg. In some instances, the Ca-AKG is administered at a dose of at least about 1400 mg. In some instances, the Ca-AKG is administered at a dose of at least about 1500 mg. In some instances, the Ca-AKG is administered at a dose of at least about 1600 mg. In some instances, the Ca-AKG is administered at a dose of at least about 1700 mg. In some instances, the Ca-AKG is administered at a dose of at least about 1800 mg. In some instances, the Ca-AKG is administered at a dose of at least about 1900 mg. In some instances, the Ca-AKG is administered at a dose of at least about 2000 mg.

In some embodiments, the hydrate Ca-AKG is administered at a dose of at least 350 mg and no greater than 2000 mg. In some instances, the hydrate Ca-AKG is administered at a dose of about 350 mg to about 2000 mg. In some instances, the hydrate Ca-AKG is administered at a dose of about 400 mg to about 2000 mg. In some instances, the hydrate Ca-AKG is administered at a dose of about 450 mg to about 2000 mg. In some instances, the hydrate Ca-AKG is administered at a dose of about 500 mg to about 2000 mg. In some instances, the hydrate Ca-AKG is administered at a dose of about 550 mg to about 2000 mg. In some instances, the hydrate Ca-AKG is administered at a dose of about 600 mg to about 2000 mg. In some instances, the hydrate Ca-AKG is administered at a dose of about 650 mg to about 2000 mg. In some instances, the hydrate Ca-AKG is administered at a dose of about 700 mg to about 2000 mg. In some instances, the hydrate Ca-AKG is administered at a dose of about 800 mg to about 2000 mg. In some instances, the hydrate Ca-AKG is administered at a dose of about 900 mg to about 2000 mg. In some instances, the hydrate Ca-AKG is administered at a dose of about 1000 mg to about 2000 mg. In some instances, the hydrate Ca-AKG is administered at a dose of about 1100 mg to about 2000 mg. In some instances, the hydrate Ca-AKG is administered at a dose of about 1200 mg to about 2000 mg. In some instances, the hydrate Ca-AKG is administered at a dose of about 1300 mg to about 2000 mg. In some instances, the hydrate Ca-AKG is administered at a dose of about 1400 mg to about 2000 mg. In some instances, the hydrate Ca-AKG is administered at a dose of about 1500 mg to about 2000 mg. In some instances, the hydrate Ca-AKG is administered at a dose of about 1600 mg to about 2000 mg. In some instances, the hydrate Ca-AKG is administered at a dose of about 1700 mg to about 2000 mg. In some instances, the hydrate Ca-AKG is administered at a dose of about 1800 mg to about 2000 mg. In some instances, the hydrate Ca-AKG is administered at a dose of about 1900 mg to about 2000 mg. In some instances, the hydrate Ca-AKG is administered at a dose of about 350 mg to about 1900 mg. In some instances, the hydrate Ca-AKG is administered at a dose of about 350 mg to about 1800 mg. In some instances, the hydrate Ca-AKG is administered at a dose of about 350 mg to about 1700 mg. In some instances, the hydrate Ca-AKG is administered at a dose of about 350 mg to about 1600 mg. In some instances, the hydrate Ca-AKG is administered at a dose of about 350 mg to about 1500 mg. In some instances, the hydrate Ca-AKG is administered at a dose of about 350 mg to about 1400 mg. In some instances, the hydrate Ca-AKG is administered at a dose of about 350 mg to about 1300 mg. In some instances, the hydrate Ca-AKG is administered at a dose of about 350 mg to about 1200 mg. In some instances, the hydrate Ca-AKG is administered at a dose of about 350 mg to about 1100 mg. In some instances, the hydrate Ca-AKG is administered at a dose of about 350 mg to about 1000 mg. In some instances, the hydrate Ca-AKG is administered at a dose of about 350 mg to about 900 mg. In some instances, the hydrate Ca-AKG is administered at a dose of about 350 mg to about 800 mg. In some instances, the hydrate Ca-AKG is administered at a dose of about 350 mg to about 700 mg. In some instances, the hydrate Ca-AKG is administered at a dose of about 350 mg to about 650 mg. In some instances, the hydrate Ca-AKG is administered at a dose of about 350 mg to about 600 mg. In some instances, the hydrate Ca-AKG is administered at a dose of about 350 mg to about 550 mg. In some instances, the hydrate Ca-AKG is administered at a dose of about 350 mg to about 500 mg. In some instances, the hydrate Ca-AKG is administered at a dose of about 350 mg to about 450 mg. In some instances, the hydrate Ca-AKG is administered at a dose of about 350 mg to about 400 mg. In some instances, the hydrate Ca-AKG is administered at a dose of at least about 350 mg. In some instances, the hydrate Ca-AKG is administered at a dose of at least about 400 mg. In some instances, the hydrate Ca-AKG is administered at a dose of at least about 450 mg. In some instances, the hydrate Ca-AKG is administered at a dose of at least about 500 mg. In some instances, the hydrate Ca-AKG is administered at a dose of at least about 550 mg. In some instances, the hydrate Ca-AKG is administered at a dose of at least about 600 mg. In some instances, the hydrate Ca-AKG is administered at a dose of at least about 650 mg. In some instances, the hydrate Ca-AKG is administered at a dose of at least about 700 mg. In some instances, the hydrate Ca-AKG is administered at a dose of at least about 750 mg. In some instances, the hydrate Ca-AKG is administered at a dose of at least about 800 mg. In some instances, the hydrate Ca-AKG is administered at a dose of at least about 900 mg. In some instances, the hydrate Ca-AKG is administered at a dose of at least about 1000 mg. In some instances, the hydrate Ca-AKG is administered at a dose of at least about 1100 mg. In some instances, the hydrate Ca-AKG is administered at a dose of at least about 1200 mg. In some instances, the hydrate Ca-AKG is administered at a dose of at least about 1300 mg. In some instances, the hydrate Ca-AKG is administered at a dose of at least about 1400 mg. In some instances, the hydrate Ca-AKG is administered at a dose of at least about 1500 mg. In some instances, the hydrate Ca-AKG is administered at a dose of at least about 1600 mg. In some instances, the hydrate Ca-AKG is administered at a dose of at least about 1700 mg. In some instances, the hydrate Ca-AKG is administered at a dose of at least about 1800 mg. In some instances, the hydrate Ca-AKG is administered at a dose of at least about 1900 mg. In some instances, the hydrate Ca-AKG is administered at a dose of at least about 2000 mg.

In some embodiments, the mono-hydrate Ca-AKG is administered at a dose of at least 350 mg and no greater than 2000 mg. In some instances, the mono-hydrate Ca-AKG is administered at a dose of about 350 mg to about 2000 mg. In some instances, the mono-hydrate Ca-AKG is administered at a dose of about 400 mg to about 2000 mg. In some instances, the mono-hydrate Ca-AKG is administered at a dose of about 450 mg to about 2000 mg. In some instances, the mono-hydrate Ca-AKG is administered at a dose of about 500 mg to about 2000 mg. In some instances, the mono-hydrate Ca-AKG is administered at a dose of about 550 mg to about 2000 mg. In some instances, the mono-hydrate Ca-AKG is administered at a dose of about 600 mg to about 2000 mg. In some instances, the mono-hydrate Ca-AKG is administered at a dose of about 650 mg to about 2000 mg. In some instances, the mono-hydrate Ca-AKG is administered at a dose of about 700 mg to about 2000 mg. In some instances, the mono-hydrate Ca-AKG is administered at a dose of about 800 mg to about 2000 mg. In some instances, the mono-hydrate Ca-AKG is administered at a dose of about 900 mg to about 2000 mg. In some instances, the mono-hydrate Ca-AKG is administered at a dose of about 1000 mg to about 2000 mg. In some instances, the mono-hydrate Ca-AKG is administered at a dose of about 1100 mg to about 2000 mg. In some instances, the mono-hydrate Ca-AKG is administered at a dose of about 1200 mg to about 2000 mg. In some instances, the mono-hydrate Ca-AKG is administered at a dose of about 1300 mg to about 2000 mg. In some instances, the mono-hydrate Ca-AKG is administered at a dose of about 1400 mg to about 2000 mg. In some instances, the mono-hydrate Ca-AKG is administered at a dose of about 1500 mg to about 2000 mg. In some instances, the mono-hydrate Ca-AKG is administered at a dose of about 1600 mg to about 2000 mg. In some instances, the mono-hydrate Ca-AKG is administered at a dose of about 1700 mg to about 2000 mg. In some instances, the mono-hydrate Ca-AKG is administered at a dose of about 1800 mg to about 2000 mg. In some instances, the mono-hydrate Ca-AKG is administered at a dose of about 1900 mg to about 2000 mg. In some instances, the mono-hydrate Ca-AKG is administered at a dose of about 350 mg to about 1900 mg. In some instances, the mono-hydrate Ca-AKG is administered at a dose of about 350 mg to about 1800 mg. In some instances, the mono-hydrate Ca-AKG is administered at a dose of about 350 mg to about 1700 mg. In some instances, the mono-hydrate Ca-AKG is administered at a dose of about 350 mg to about 1600 mg. In some instances, the mono-hydrate Ca-AKG is administered at a dose of about 350 mg to about 1500 mg. In some instances, the mono-hydrate Ca-AKG is administered at a dose of about 350 mg to about 1400 mg. In some instances, the mono-hydrate Ca-AKG is administered at a dose of about 350 mg to about 1300 mg. In some instances, the mono-hydrate Ca-AKG is administered at a dose of about 350 mg to about 1200 mg. In some instances, the mono-hydrate Ca-AKG is administered at a dose of about 350 mg to about 1100 mg. In some instances, the mono-hydrate Ca-AKG is administered at a dose of about 350 mg to about 1000 mg. In some instances, the mono-hydrate Ca-AKG is administered at a dose of about 350 mg to about 900 mg. In some instances, the mono-hydrate Ca-AKG is administered at a dose of about 350 mg to about 800 mg. In some instances, the mono-hydrate Ca-AKG is administered at a dose of about 350 mg to about 700 mg. In some instances, the mono-hydrate Ca-AKG is administered at a dose of about 350 mg to about 650 mg. In some instances, the mono-hydrate Ca-AKG is administered at a dose of about 350 mg to about 600 mg. In some instances, the mono-hydrate Ca-AKG is administered at a dose of about 350 mg to about 550 mg. In some instances, the mono-hydrate Ca-AKG is administered at a dose of about 350 mg to about 500 mg. In some instances, the mono-hydrate Ca-AKG is administered at a dose of about 350 mg to about 450 mg. In some instances, the mono-hydrate Ca-AKG is administered at a dose of about 350 mg to about 400 mg. In some instances, the mono-hydrate Ca-AKG is administered at a dose of at least about 350 mg. In some instances, the mono-hydrate Ca-AKG is administered at a dose of at least about 400 mg. In some instances, the mono-hydrate Ca-AKG is administered at a dose of at least about 450 mg. In some instances, the mono-hydrate Ca-AKG is administered at a dose of at least about 500 mg. In some instances, the mono-hydrate Ca-AKG is administered at a dose of at least about 550 mg. In some instances, the mono-hydrate Ca-AKG is administered at a dose of at least about 600 mg. In some instances, the mono-hydrate Ca-AKG is administered at a dose of at least about 650 mg. In some instances, the mono-hydrate Ca-AKG is administered at a dose of at least about 700 mg. In some instances, the mono-hydrate Ca-AKG is administered at a dose of at least about 750 mg. In some instances, the mono-hydrate Ca-AKG is administered at a dose of at least about 800 mg. In some instances, the mono-hydrate Ca-AKG is administered at a dose of at least about 900 mg. In some instances, the mono-hydrate Ca-AKG is administered at a dose of at least about 1000 mg. In some instances, the mono-hydrate Ca-AKG is administered at a dose of at least about 1100 mg. In some instances, the mono-hydrate Ca-AKG is administered at a dose of at least about 1200 mg. In some instances, the mono-hydrate Ca-AKG is administered at a dose of at least about 1300 mg. In some instances, the mono-hydrate Ca-AKG is administered at a dose of at least about 1400 mg. In some instances, the mono-hydrate Ca-AKG is administered at a dose of at least about 1500 mg. In some instances, the mono-hydrate Ca-AKG is administered at a dose of at least about 1600 mg. In some instances, the mono-hydrate Ca-AKG is administered at a dose of at least about 1700 mg. In some instances, the mono-hydrate Ca-AKG is administered at a dose of at least about 1800 mg. In some instances, the mono-hydrate Ca-AKG is administered at a dose of at least about 1900 mg. In some instances, the mono-hydrate Ca-AKG is administered at a dose of at least about 2000 mg.

In some embodiments, the hemi-hydrate Ca-AKG is administered at a dose of at least 350 mg and no greater than 2000 mg. In some instances, the hemi-hydrate Ca-AKG is administered at a dose of about 350 mg to about 2000 mg. In some instances, the hemi-hydrate Ca-AKG is administered at a dose of about 400 mg to about 2000 mg. In some instances, the hemi-hydrate Ca-AKG is administered at a dose of about 450 mg to about 2000 mg. In some instances, the hemi-hydrate Ca-AKG is administered at a dose of about 500 mg to about 2000 mg. In some instances, the hemi-hydrate Ca-AKG is administered at a dose of about 550 mg to about 2000 mg. In some instances, the hemi-hydrate Ca-AKG is administered at a dose of about 600 mg to about 2000 mg. In some instances, the hemi-hydrate Ca-AKG is administered at a dose of about 650 mg to about 2000 mg. In some instances, the hemi-hydrate Ca-AKG is administered at a dose of about 700 mg to about 2000 mg. In some instances, the hemi-hydrate Ca-AKG is administered at a dose of about 800 mg to about 2000 mg. In some instances, the hemi-hydrate Ca-AKG is administered at a dose of about 900 mg to about 2000 mg. In some instances, the hemi-hydrate Ca-AKG is administered at a dose of about 1000 mg to about 2000 mg. In some instances, the hemi-hydrate Ca-AKG is administered at a dose of about 1100 mg to about 2000 mg. In some instances, the hemi-hydrate Ca-AKG is administered at a dose of about 1200 mg to about 2000 mg. In some instances, the hemi-hydrate Ca-AKG is administered at a dose of about 1300 mg to about 2000 mg. In some instances, the hemi-hydrate Ca-AKG is administered at a dose of about 1400 mg to about 2000 mg. In some instances, the hemi-hydrate Ca-AKG is administered at a dose of about 1500 mg to about 2000 mg. In some instances, the hemi-hydrate Ca-AKG is administered at a dose of about 1600 mg to about 2000 mg. In some instances, the hemi-hydrate Ca-AKG is administered at a dose of about 1700 mg to about 2000 mg. In some instances, the hemi-hydrate Ca-AKG is administered at a dose of about 1800 mg to about 2000 mg. In some instances, the hemi-hydrate Ca-AKG is administered at a dose of about 1900 mg to about 2000 mg. In some instances, the hemi-hydrate Ca-AKG is administered at a dose of about 350 mg to about 1900 mg. In some instances, the hemi-hydrate Ca-AKG is administered at a dose of about 350 mg to about 1800 mg. In some instances, the hemi-hydrate Ca-AKG is administered at a dose of about 350 mg to about 1700 mg. In some instances, the hemi-hydrate Ca-AKG is administered at a dose of about 350 mg to about 1600 mg. In some instances, the hemi-hydrate Ca-AKG is administered at a dose of about 350 mg to about 1500 mg. In some instances, the hemi-hydrate Ca-AKG is administered at a dose of about 350 mg to about 1400 mg. In some instances, the hemi-hydrate Ca-AKG is administered at a dose of about 350 mg to about 1300 mg. In some instances, the hemi-hydrate Ca-AKG is administered at a dose of about 350 mg to about 1200 mg. In some instances, the hemi-hydrate Ca-AKG is administered at a dose of about 350 mg to about 1100 mg. In some instances, the hemi-hydrate Ca-AKG is administered at a dose of about 350 mg to about 1000 mg. In some instances, the hemi-hydrate Ca-AKG is administered at a dose of about 350 mg to about 900 mg. In some instances, the hemi-hydrate Ca-AKG is administered at a dose of about 350 mg to about 800 mg. In some instances, the hemi-hydrate Ca-AKG is administered at a dose of about 350 mg to about 700 mg. In some instances, the hemi-hydrate Ca-AKG is administered at a dose of about 350 mg to about 650 mg. In some instances, the hemi-hydrate Ca-AKG is administered at a dose of about 350 mg to about 600 mg. In some instances, the hemi-hydrate Ca-AKG is administered at a dose of about 350 mg to about 550 mg. In some instances, the hemi-hydrate Ca-AKG is administered at a dose of about 350 mg to about 500 mg. In some instances, the hemi-hydrate Ca-AKG is administered at a dose of about 350 mg to about 450 mg. In some instances, the hemi-hydrate Ca-AKG is administered at a dose of about 350 mg to about 400 mg. In some instances, the hemi-hydrate Ca-AKG is administered at a dose of at least about 350 mg. In some instances, the hemi-hydrate Ca-AKG is administered at a dose of at least about 400 mg. In some instances, the hemi-hydrate Ca-AKG is administered at a dose of at least about 450 mg. In some instances, the hemi-hydrate Ca-AKG is administered at a dose of at least about 500 mg. In some instances, the hemi-hydrate Ca-AKG is administered at a dose of at least about 550 mg. In some instances, the hemi-hydrate Ca-AKG is administered at a dose of at least about 600 mg. In some instances, the hemi-hydrate Ca-AKG is administered at a dose of at least about 650 mg. In some instances, the hemi-hydrate Ca-AKG is administered at a dose of at least about 700 mg. In some instances, the hemi-hydrate Ca-AKG is administered at a dose of at least about 750 mg. In some instances, the hemi-hydrate Ca-AKG is administered at a dose of at least about 800 mg. In some instances, the hemi-hydrate Ca-AKG is administered at a dose of at least about 900 mg. In some instances, the hemi-hydrate Ca-AKG is administered at a dose of at least about 1000 mg. In some instances, the hemi-hydrate Ca-AKG is administered at a dose of at least about 1100 mg. In some instances, the hemi-hydrate Ca-AKG is administered at a dose of at least about 1200 mg. In some instances, the hemi-hydrate Ca-AKG is administered at a dose of at least about 1300 mg. In some instances, the hemi-hydrate Ca-AKG is administered at a dose of at least about 1400 mg. In some instances, the hemi-hydrate Ca-AKG is administered at a dose of at least about 1500 mg. In some instances, the hemi-hydrate Ca-AKG is administered at a dose of at least about 1600 mg. In some instances, the hemi-hydrate Ca-AKG is administered at a dose of at least about 1700 mg. In some instances, the hemi-hydrate Ca-AKG is administered at a dose of at least about 1800 mg. In some instances, the hemi-hydrate Ca-AKG is administered at a dose of at least about 1900 mg. In some instances, the hemi-hydrate Ca-AKG is administered at a dose of at least about 2000 mg.

In some embodiments, the anhydrous Ca-AKG is administered at a dose of at least 350 mg and no greater than 2000 mg. In some instances, the anhydrous Ca-AKG is administered at a dose of about 350 mg to about 2000 mg. In some instances, the anhydrous Ca-AKG is administered at a dose of about 400 mg to about 2000 mg. In some instances, the anhydrous Ca-AKG is administered at a dose of about 450 mg to about 2000 mg. In some instances, the anhydrous Ca-AKG is administered at a dose of about 500 mg to about 2000 mg. In some instances, the anhydrous Ca-AKG is administered at a dose of about 550 mg to about 2000 mg. In some instances, the anhydrous Ca-AKG is administered at a dose of about 600 mg to about 2000 mg. In some instances, the anhydrous Ca-AKG is administered at a dose of about 650 mg to about 2000 mg. In some instances, the anhydrous Ca-AKG is administered at a dose of about 700 mg to about 2000 mg. In some instances, the anhydrous Ca-AKG is administered at a dose of about 800 mg to about 2000 mg. In some instances, the anhydrous Ca-AKG is administered at a dose of about 900 mg to about 2000 mg. In some instances, the anhydrous Ca-AKG is administered at a dose of about 1000 mg to about 2000 mg. In some instances, the anhydrous Ca-AKG is administered at a dose of about 1100 mg to about 2000 mg. In some instances, the anhydrous Ca-AKG is administered at a dose of about 1200 mg to about 2000 mg. In some instances, the anhydrous Ca-AKG is administered at a dose of about 1300 mg to about 2000 mg. In some instances, the anhydrous Ca-AKG is administered at a dose of about 1400 mg to about 2000 mg. In some instances, the anhydrous Ca-AKG is administered at a dose of about 1500 mg to about 2000 mg. In some instances, the anhydrous Ca-AKG is administered at a dose of about 1600 mg to about 2000 mg. In some instances, the anhydrous Ca-AKG is administered at a dose of about 1700 mg to about 2000 mg. In some instances, the anhydrous Ca-AKG is administered at a dose of about 1800 mg to about 2000 mg. In some instances, the anhydrous Ca-AKG is administered at a dose of about 1900 mg to about 2000 mg. In some instances, the anhydrous Ca-AKG is administered at a dose of about 350 mg to about 1900 mg. In some instances, the anhydrous Ca-AKG is administered at a dose of about 350 mg to about 1800 mg. In some instances, the anhydrous Ca-AKG is administered at a dose of about 350 mg to about 1700 mg. In some instances, the anhydrous Ca-AKG is administered at a dose of about 350 mg to about 1600 mg. In some instances, the anhydrous Ca-AKG is administered at a dose of about 350 mg to about 1500 mg. In some instances, the anhydrous Ca-AKG is administered at a dose of about 350 mg to about 1400 mg. In some instances, the anhydrous Ca-AKG is administered at a dose of about 350 mg to about 1300 mg. In some instances, the anhydrous Ca-AKG is administered at a dose of about 350 mg to about 1200 mg. In some instances, the anhydrous Ca-AKG is administered at a dose of about 350 mg to about 1100 mg. In some instances, the anhydrous Ca-AKG is administered at a dose of about 350 mg to about 1000 mg. In some instances, the anhydrous Ca-AKG is administered at a dose of about 350 mg to about 900 mg. In some instances, the anhydrous Ca-AKG is administered at a dose of about 350 mg to about 800 mg. In some instances, the anhydrous Ca-AKG is administered at a dose of about 350 mg to about 700 mg. In some instances, the anhydrous Ca-AKG is administered at a dose of about 350 mg to about 650 mg. In some instances, the anhydrous Ca-AKG is administered at a dose of about 350 mg to about 600 mg. In some instances, the anhydrous Ca-AKG is administered at a dose of about 350 mg to about 550 mg. In some instances, the anhydrous Ca-AKG is administered at a dose of about 350 mg to about 500 mg. In some instances, the anhydrous Ca-AKG is administered at a dose of about 350 mg to about 450 mg. In some instances, the anhydrous Ca-AKG is administered at a dose of about 350 mg to about 400 mg. In some instances, the anhydrous Ca-AKG is administered at a dose of at least about 350 mg. In some instances, the anhydrous Ca-AKG is administered at a dose of at least about 400 mg. In some instances, the anhydrous Ca-AKG is administered at a dose of at least about 450 mg. In some instances, the anhydrous Ca-AKG is administered at a dose of at least about 500 mg. In some instances, the anhydrous Ca-AKG is administered at a dose of at least about 550 mg. In some instances, the anhydrous Ca-AKG is administered at a dose of at least about 600 mg. In some instances, the anhydrous Ca-AKG is administered at a dose of at least about 650 mg. In some instances, the anhydrous Ca-AKG is administered at a dose of at least about 700 mg. In some instances, the anhydrous Ca-AKG is administered at a dose of at least about 750 mg. In some instances, the anhydrous Ca-AKG is administered at a dose of at least about 800 mg. In some instances, the anhydrous Ca-AKG is administered at a dose of at least about 900 mg. In some instances, the anhydrous Ca-AKG is administered at a dose of at least about 1000 mg. In some instances, the anhydrous Ca-AKG is administered at a dose of at least about 1100 mg. In some instances, the anhydrous Ca-AKG is administered at a dose of at least about 1200 mg. In some instances, the anhydrous Ca-AKG is administered at a dose of at least about 1300 mg. In some instances, the anhydrous Ca-AKG is administered at a dose of at least about 1400 mg. In some instances, the anhydrous Ca-AKG is administered at a dose of at least about 1500 mg. In some instances, the anhydrous Ca-AKG is administered at a dose of at least about 1600 mg. In some instances, the anhydrous Ca-AKG is administered at a dose of at least about 1700 mg. In some instances, the anhydrous Ca-AKG is administered at a dose of at least about 1800 mg. In some instances, the anhydrous Ca-AKG is administered at a dose of at least about 1900 mg. In some instances, the anhydrous Ca-AKG is administered at a dose of at least about 2000 mg.

In some embodiments, administration of compositions comprising AKG extends healthspan in a subject in need thereof. In some embodiments, administration of compositions comprising berberine, AKG, and vitamin A extends healthspan in a subject in need thereof. In some embodiments, administration of compositions comprising AKG and vitamin A extends healthspan in a subject in need thereof. In some embodiments, administration of compositions comprising AKG and vitamin D extends healthspan in a subject in need thereof. In some embodiments, administration of compositions comprising AKG and NMN extends healthspan in a subject in need thereof.

In some embodiments, extension of healthspan comprises a delay in one or more of the aging phenotypes described above. In some embodiments, administration of compositions comprising AKG delays or reverses graying hair in a subject in need thereof. In some embodiments, administration of compositions comprising berberine, AKG, and vitamin A delays or reverses graying hair in a subject in need thereof. In some embodiments, administration of compositions comprising AKG and vitamin A delays or reverses graying hair in a subject in need thereof. In some embodiments, administration of compositions comprising AKG and vitamin D delays or reverses graying hair in a subject in need thereof. In some embodiments, administration of compositions comprising AKG and NMN delays or reverses graying hair in a subject in need thereof.

In further embodiments, extension of healthspan comprises a delay in one or more of the aging phenotypes described above of at least 1 month, at least 2 months, at least 3 months, at least 4 months, at least 5 months, at least 6 months, at least 7 months, at least 8 months, at least 9 months, at least 10 months, at least 11 months, at least 12 months after administration of the active agent or composition thereof. In other embodiments, extension of healthspan comprises at reversal in one or more of the aging phenotypes described above.

In some embodiments, extending healthspan comprises a delay in onset in one or more age-related diseases. In further embodiments, the age-related disease is selected from the group consisting of osteoporosis, arthritis, cataracts, cancer, kidney disease, obesity, type-2 diabetes, a neurodegenerative disease (such as Parkinson's and Alzheimer's disease), heart disease, macular degeneration, and an autoimmune disease. In further embodiments, the age-related disease is selected from the group consisting of obesity, type-2 diabetes, macular degeneration, and an autoimmune disease. In some instances, the age-related disease is cancer. In some instances, the age-related disease is kidney disease. In some instances, the age-related disease is obesity. In some instances, the age-related disease is type-2 diabetes. In some instances, the age-related disease is a neurodegenerative disease. In some instances, the age-related disease is a heart disease. In some instances, the age-related disease is a macular degeneration. In some instances, the age-related disease is an autoimmune disease.

In some embodiments, the onset of the age-related disease is delayed for at least 1 month after administration of the composition to the subjects. In some embodiments, the onset of the age-related disease is delayed for at least 2 months after administration of the composition to the subjects. In some embodiments, the onset of the age-related disease is delayed for at least 3 months after administration of the composition to the subjects. In some embodiments, the onset of the age-related disease is delayed for at least 4 months after administration of the composition to the subjects. In some embodiments, the onset of the age-related disease is delayed for at least 5 months after administration of the composition to the subjects. In some embodiments, the onset of the age-related disease is delayed for at least 6 months after administration of the composition to the subjects. In some embodiments, the onset of the age-related disease is delayed for at least 7 months after administration of the composition to the subjects. In some embodiments, the onset of the age-related disease delayed for at least 8 months after administration of the composition to the subjects. In some embodiments, the onset of the age-related disease is delayed for at least 9 months after administration of the composition to the subjects. In some embodiments, the onset of the age-related disease is delayed for at least 10 months after administration of the composition to the subjects. In some embodiments the onset of the age-related disease is delayed for at least 11 months after administration of the composition to the subjects. In some embodiments, the onset of the age-related disease is delayed for at least 12 months after administration of the composition to the subjects. In some embodiments, the onset of the age-related disease is delayed for at least 18 months after administration of the composition to the subjects. In some embodiments, the onset of the age-related disease is delayed for at least 24 months after administration of the composition to the subjects. In some embodiments, the onset of the age-related disease is delayed for at least 30 months after administration of the composition to the subjects. In some embodiments, the onset of the age-related disease is delayed for at least 36 months after administration of the composition to the subjects. In other embodiments, extension of healthspan comprises a reversal in one or more age-related disease.

In some embodiments, administration of the compositions slows the rate of progression or reverse changes in biomarkers of non-human or human aging. Biomarker strategies to measure aging are currently being developed and can be employed to test the effects of aging interventions. The most prominent is the epigenetic clock, which likely measures biologic age in cells from humans (see for e.g. Chen et al. Aging, 8:1844-1865(2016), PMID 27690265), dogs (see for e.g. Thompson et al. Aging 9:1055-1068(2017), PMID 28373601), and mice (see for e.g. Petkovich et al. Cell Metab 25:954-960(2017), PMID 28380383). Other biomarker strategies include, but are not limited to, inflammatory cytokine levels, p16INK4A protein levels in specific cell populations, telomere length and levels of specific metabolites. In some embodiments, the composition modulates a biomarker of DNA methylation.

In some embodiments, the subject is a mammal. In some embodiments, the mammal is a human. In some embodiments, the mammal is a dog. In some embodiments, the mammal is livestock. In some embodiments, the livestock is selected from the group consisting of cattle, sheep, goats, swine, poultry, and equine animals.

In some embodiments, the composition is formulated into an orally administered form. In further embodiments, the orally administered form comprises a tablet, a powder, a suspension, a serum, an emulsion, a capsule, a granule, a pill, a gel, a solution, or a syrup. In some instances, the orally administered form is a sustained release dosage form. In some instances, the orally administered form is formulated into animal feed. In some instances, the orally administered form is the gel. In some embodiments, the composition is formulated into an injectable administered form. In further embodiments, the injectable administered form comprises a liquid, a suspension, or a solution. In some instances, the injectable administered form is a sustained release dosage form. In some embodiments, the composition is formulated into a topically administered form. In further embodiments, the topically administered form is a cream, a foam, a gel, a lotion, an ointment, or a serum. In some embodiments, the composition is formulated into a hair care product. In further embodiments, the hair care product is a shampoo, a conditioner, hair spray, or a moisturizer.

In some embodiments, the composition further comprises a pharmaceutically acceptable excipient. In further embodiments, the pharmaceutically acceptable excipient comprises an antiadherent, a binder, a coating, a color, disintegrant, a flavor, a glidant, a lubricant, a preservative, a sorbent, or a vehicle. In some instances, the composition further comprises silymarin.

In some embodiments, the composition is administered for a particular time period. In some embodiments, the composition is administered for a chronic treatment period, which is, for an extended period of time, including throughout the duration of the subject's life in order to ameliorate or otherwise control or limit the symptoms of the subject's disease or condition. Within the treatment period, the composition is administered on a particular time schedule. In further embodiments, the composition is administered one, two, three, or four times daily. In some embodiments, the composition is administered once daily. In some embodiments, the composition is administered twice daily. In some embodiments, the composition is administered in the morning and evening. In some embodiments, the composition is administered one, two, three, or four times weekly. In some embodiments, the composition is administered once a week. In some embodiments the composition is administered one, two, three, or four times monthly. In some embodiments, the composition is administered once a month. In some embodiments, the composition is administered for at least three months of every one year. In some embodiments, the composition is administered one month of every six months.

### Compression of morbidity

In an aspect, the disclosure provides methods of compressing morbidity using the active agents or compositions thereof described herein. Compression of morbidity occurs if the age of first appearance of aging manifestations and chronic disease symptoms increases more rapidly than life expectancy. The period between marker of morbidity (e.g. first heart attack, first dyspnea from emphysema, first disability from osteoarthritis, first memory loss of a certain magnitude) and the end of life is shortened when the average onset age of the marker increases more rapidly than life expectancy from the same age. This disproportionally increases the healthy years of life, and dramatically reduces the end stage costs of healthcare.

In an aspect, the disclosure provides methods of compressing morbidity using the active agents or compositions thereof described herein. In some aspects, the composition comprises two or more active agents selected from the group consisting of: alpha-ketoglutarate, berberine, vitamin A, vitamin D and nicotinamide mononucleotide (NMN). In certain aspects, the composition comprises berberine, α-ketoglutarate (AKG), and vitamin A. In certain aspects, the composition comprises berberine, α-ketoglutarate (AKG), and retinoic acid (RA). In certain aspects, the composition comprises berberine, α-ketoglutarate (AKG), and retinyl palmitate (RP). In certain aspects, the composition comprises AKG and vitamin A. In certain aspects, the composition comprises AKG and retinoic acid (RA). In certain aspects, the composition comprises AKG and retinyl palmitate (RP). In certain aspects, the composition comprises AKG and vitamin D. In certain aspects, the composition comprises AKG and vitamin D3. In certain aspects, the composition comprises AKG and nicotinamide mononucleotide (NMN). In some instances, the AKG in the compositions is a salt of AKG. In some instances, the AKG provided in the compositions is a calcium salt of AKG (Ca-AKG). In some embodiments, the two or more compounds comprise alpha-ketoglutarate and berberine. In yet other embodiments, the two or more compounds comprise berberine and vitamin A. In some aspects, disclosure further provides compositions comprising an active agent selected from the group consisting of: alpha-ketoglutarate, berberine, vitamin A and vitamin D. In certain aspects, the compound is AKG. In some embodiments, AKG is provided as a salt. In some embodiments, AKG is provided as a calcium salt (Ca-AKG).

In certain aspects, the disclosure provides methods of compressing morbidity in a subject in need thereof comprising administering to the subject a composition comprising a calcium salt of alpha-ketoglutarate (Ca-AKG). In another aspect, the disclosure provides methods of compressing morbidity in a subject in need thereof comprising administering to the subject a composition consisting essentially of a calcium salt of alpha-ketoglutarate (Ca-AKG). In some embodiments, calcium α-ketoglutarate can be a hydrate calcium α-ketoglutarate. In some embodiments, calcium α-ketoglutarate can be a mono-hydrate calcium α-ketoglutarate. In some embodiments, calcium α-ketoglutarate can be hemi-hydrate calcium α-ketoglutarate. In some embodiments, calcium α-ketoglutarate can be anhydrous calcium α-ketoglutarate.

In some embodiments, the Ca-AKG is administered at a dose of at least 350 mg and no greater than 2000 mg. In some instances, the Ca-AKG is administered at a dose of about 350 mg to about 2000 mg. In some instances, the Ca-AKG is administered at a dose of about 400 mg to about 2000 mg. In some instances, the Ca-AKG is administered at a dose of about 450 mg to about 2000 mg. In some instances, the Ca-AKG is administered at a dose of about 500 mg to about 2000 mg. In some instances, the Ca-AKG is administered at a dose of about 550 mg to about 2000 mg. In some instances, the Ca-AKG is administered at a dose of about 600 mg to about 2000 mg. In some instances, the Ca-AKG is administered at a dose of about 650 mg to about 2000 mg. In some instances, the Ca-AKG is administered at a dose of about 700 mg to about 2000 mg. In some instances, the Ca-AKG is administered at a dose of about 800 mg to about 2000 mg. In some instances, the Ca-AKG is administered at a dose of about 900 mg to about 2000 mg. In some instances, the Ca-AKG is administered at a dose of about 1000 mg to about 2000 mg. In some instances, the Ca-AKG is administered at a dose of about 1100 mg to about 2000 mg. In some instances, the Ca-AKG is administered at a dose of about 1200 mg to about 2000 mg. In some instances, the Ca-AKG is administered at a dose of about 1300 mg to about 2000 mg. In some instances, the Ca-AKG is administered at a dose of about 1400 mg to about 2000 mg. In some instances, the Ca-AKG is administered at a dose of about 1500 mg to about 2000 mg. In some instances, the Ca-AKG is administered at a dose of about 1600 mg to about 2000 mg. In some instances, the Ca-AKG is administered at a dose of about 1700 mg to about 2000 mg. In some instances, the Ca-AKG is administered at a dose of about 1800 mg to about 2000 mg. In some instances, the Ca-AKG is administered at a dose of about 1900 mg to about 2000 mg. In some instances, the Ca-AKG is administered at a dose of about 350 mg to about 1900 mg. In some instances, the Ca-AKG is administered at a dose of about 350 mg to about 1800 mg. In some instances, the Ca-AKG is administered at a dose of about 350 mg to about 1700 mg. In some instances, the Ca-AKG is administered at a dose of about 350 mg to about 1600 mg. In some instances, the Ca-AKG is administered at a dose of about 350 mg to about 1500 mg. In some instances, the Ca-AKG is administered at a dose of about 350 mg to about 1400 mg. In some instances, the Ca-AKG is administered at a dose of about 350 mg to about 1300 mg. In some instances, the Ca-AKG is administered at a dose of about 350 mg to about 1200 mg. In some instances, the Ca-AKG is administered at a dose of about 350 mg to about 1100 mg. In some instances, the Ca-AKG is administered at a dose of about 350 mg to about 1000 mg. In some instances, the Ca-AKG is administered at a dose of about 350 mg to about 900 mg. In some instances, the Ca-AKG is administered at a dose of about 350 mg to about 800 mg. In some instances, the Ca-AKG is administered at a dose of about 350 mg to about 700 mg. In some instances, the Ca-AKG is administered at a dose of about 350 mg to about 650 mg. In some instances, the Ca-AKG is administered at a dose of about 350 mg to about 600 mg. In some instances, the Ca-AKG is administered at a dose of about 350 mg to about 550 mg. In some instances, the Ca-AKG is administered at a dose of about 350 mg to about 500 mg. In some instances, the Ca-AKG is administered at a dose of about 350 mg to about 450 mg. In some instances, the Ca-AKG is administered at a dose of about 350 mg to about 400 mg. In some instances, the Ca-AKG is administered at a dose of at least about 350 mg. In some instances, the Ca-AKG is administered at a dose of at least about 400 mg. In some instances, the Ca-AKG is administered at a dose of at least about 450 mg. In some instances, the Ca-AKG is administered at a dose of at least about 500 mg. In some instances, the Ca-AKG is administered at a dose of at least about 550 mg. In some instances, the Ca-AKG is administered at a dose of at least about 600 mg. In some instances, the Ca-AKG is administered at a dose of at least about 650 mg. In some instances, the Ca-AKG is administered at a dose of at least about 700 mg. In some instances, the Ca-AKG is administered at a dose of at least about 750 mg. In some instances, the Ca-AKG is administered at a dose of at least about 800 mg. In some instances, the Ca-AKG is administered at a dose of at least about 900 mg. In some instances, the Ca-AKG is administered at a dose of at least about 1000 mg. In some instances, the Ca-AKG is administered at a dose of at least about 1100 mg. In some instances, the Ca-AKG is administered at a dose of at least about 1200 mg. In some instances, the Ca-AKG is administered at a dose of at least about 1300 mg. In some instances, the Ca-AKG is administered at a dose of at least about 1400 mg. In some instances, the Ca-AKG is administered at a dose of at least about 1500 mg. In some instances, the Ca-AKG is administered at a dose of at least about 1600 mg. In some instances, the Ca-AKG is administered at a dose of at least about 1700 mg. In some instances, the Ca-AKG is administered at a dose of at least about 1800 mg. In some instances, the Ca-AKG is administered at a dose of at least about 1900 mg. In some instances, the Ca-AKG is administered at a dose of at least about 2000 mg.

In some embodiments, the hydrate Ca-AKG is administered at a dose of at least 350 mg and no greater than 2000 mg. In some instances, the hydrate Ca-AKG is administered at a dose of about 350 mg to about 2000 mg. In some instances, the hydrate Ca-AKG is administered at a dose of about 400 mg to about 2000 mg. In some instances, the hydrate Ca-AKG is administered at a dose of about 450 mg to about 2000 mg. In some instances, the hydrate Ca-AKG is administered at a dose of about 500 mg to about 2000 mg. In some instances, the hydrate Ca-AKG is administered at a dose of about 550 mg to about 2000 mg. In some instances, the hydrate Ca-AKG is administered at a dose of about 600 mg to about 2000 mg. In some instances, the hydrate Ca-AKG is administered at a dose of about 650 mg to about 2000 mg. In some instances, the hydrate Ca-AKG is administered at a dose of about 700 mg to about 2000 mg. In some instances, the hydrate Ca-AKG is administered at a dose of about 800 mg to about 2000 mg. In some instances, the hydrate Ca-AKG is administered at a dose of about 900 mg to about 2000 mg. In some instances, the hydrate Ca-AKG is administered at a dose of about 1000 mg to about 2000 mg. In some instances, the hydrate Ca-AKG is administered at a dose of about 1100 mg to about 2000 mg. In some instances, the hydrate Ca-AKG is administered at a dose of about 1200 mg to about 2000 mg. In some instances, the hydrate Ca-AKG is administered at a dose of about 1300 mg to about 2000 mg. In some instances, the hydrate Ca-AKG is administered at a dose of about 1400 mg to about 2000 mg. In some instances, the hydrate Ca-AKG is administered at a dose of about 1500 mg to about 2000 mg. In some instances, the hydrate Ca-AKG is administered at a dose of about 1600 mg to about 2000 mg. In some instances, the hydrate Ca-AKG is administered at a dose of about 1700 mg to about 2000 mg. In some instances, the hydrate Ca-AKG is administered at a dose of about 1800 mg to about 2000 mg. In some instances, the hydrate Ca-AKG is administered at a dose of about 1900 mg to about 2000 mg. In some instances, the hydrate Ca-AKG is administered at a dose of about 350 mg to about 1900 mg. In some instances, the hydrate Ca-AKG is administered at a dose of about 350 mg to about 1800 mg. In some instances, the hydrate Ca-AKG is administered at a dose of about 350 mg to about 1700 mg. In some instances, the hydrate Ca-AKG is administered at a dose of about 350 mg to about 1600 mg. In some instances, the hydrate Ca-AKG is administered at a dose of about 350 mg to about 1500 mg. In some instances, the hydrate Ca-AKG is administered at a dose of about 350 mg to about 1400 mg. In some instances, the hydrate Ca-AKG is administered at a dose of about 350 mg to about 1300 mg. In some instances, the hydrate Ca-AKG is administered at a dose of about 350 mg to about 1200 mg. In some instances, the hydrate Ca-AKG is administered at a dose of about 350 mg to about 1100 mg. In some instances, the hydrate Ca-AKG is administered at a dose of about 350 mg to about 1000 mg. In some instances, the hydrate Ca-AKG is administered at a dose of about 350 mg to about 900 mg. In some instances, the hydrate Ca-AKG is administered at a dose of about 350 mg to about 800 mg. In some instances, the hydrate Ca-AKG is administered at a dose of about 350 mg to about 700 mg. In some instances, the hydrate Ca-AKG is administered at a dose of about 350 mg to about 650 mg. In some instances, the hydrate Ca-AKG is administered at a dose of about 350 mg to about 600 mg. In some instances, the hydrate Ca-AKG is administered at a dose of about 350 mg to about 550 mg. In some instances, the hydrate Ca-AKG is administered at a dose of about 350 mg to about 500 mg. In some instances, the hydrate Ca-AKG is administered at a dose of about 350 mg to about 450 mg. In some instances, the hydrate Ca-AKG is administered at a dose of about 350 mg to about 400 mg. In some instances, the hydrate Ca-AKG is administered at a dose of at least about 350 mg. In some instances, the hydrate Ca-AKG is administered at a dose of at least about 400 mg. In some instances, the hydrate Ca-AKG is administered at a dose of at least about 450 mg. In some instances, the hydrate Ca-AKG is administered at a dose of at least about 500 mg. In some instances, the hydrate Ca-AKG is administered at a dose of at least about 550 mg. In some instances, the hydrate Ca-AKG is administered at a dose of at least about 600 mg. In some instances, the hydrate Ca-AKG is administered at a dose of at least about 650 mg. In some instances, the hydrate Ca-AKG is administered at a dose of at least about 700 mg. In some instances, the hydrate Ca-AKG is administered at a dose of at least about 750 mg. In some instances, the hydrate Ca-AKG is administered at a dose of at least about 800 mg. In some instances, the hydrate Ca-AKG is administered at a dose of at least about 900 mg. In some instances, the hydrate Ca-AKG is administered at a dose of at least about 1000 mg. In some instances, the hydrate Ca-AKG is administered at a dose of at least about 1100 mg. In some instances, the hydrate Ca-AKG is administered at a dose of at least about 1200 mg. In some instances, the hydrate Ca-AKG is administered at a dose of at least about 1300 mg. In some instances, the hydrate Ca-AKG is administered at a dose of at least about 1400 mg. In some instances, the hydrate Ca-AKG is administered at a dose of at least about 1500 mg. In some instances, the hydrate Ca-AKG is administered at a dose of at least about 1600 mg. In some instances, the hydrate Ca-AKG is administered at a dose of at least about 1700 mg. In some instances, the hydrate Ca-AKG is administered at a dose of at least about 1800 mg. In some instances, the hydrate Ca-AKG is administered at a dose of at least about 1900 mg. In some instances, the hydrate Ca-AKG is administered at a dose of at least about 2000 mg.

In some embodiments, the mono-hydrate Ca-AKG is administered at a dose of at least 350 mg and no greater than 2000 mg. In some instances, the mono-hydrate Ca-AKG is administered at a dose of about 350 mg to about 2000 mg. In some instances, the mono-hydrate Ca-AKG is administered at a dose of about 400 mg to about 2000 mg. In some instances, the mono-hydrate Ca-AKG is administered at a dose of about 450 mg to about 2000 mg. In some instances, the mono-hydrate Ca-AKG is administered at a dose of about 500 mg to about 2000 mg. In some instances, the mono-hydrate Ca-AKG is administered at a dose of about 550 mg to about 2000 mg. In some instances, the mono-hydrate Ca-AKG is administered at a dose of about 600 mg to about 2000 mg. In some instances, the mono-hydrate Ca-AKG is administered at a dose of about 650 mg to about 2000 mg. In some instances, the mono-hydrate Ca-AKG is administered at a dose of about 700 mg to about 2000 mg. In some instances, the mono-hydrate Ca-AKG is administered at a dose of about 800 mg to about 2000 mg. In some instances, the mono-hydrate Ca-AKG is administered at a dose of about 900 mg to about 2000 mg. In some instances, the mono-hydrate Ca-AKG is administered at a dose of about 1000 mg to about 2000 mg. In some instances, the mono-hydrate Ca-AKG is administered at a dose of about 1100 mg to about 2000 mg. In some instances, the mono-hydrate Ca-AKG is administered at a dose of about 1200 mg to about 2000 mg. In some instances, the mono-hydrate Ca-AKG is administered at a dose of about 1300 mg to about 2000 mg. In some instances, the mono-hydrate Ca-AKG is administered at a dose of about 1400 mg to about 2000 mg. In some instances, the mono-hydrate Ca-AKG is administered at a dose of about 1500 mg to about 2000 mg. In some instances, the mono-hydrate Ca-AKG is administered at a dose of about 1600 mg to about 2000 mg. In some instances, the mono-hydrate Ca-AKG is administered at a dose of about 1700 mg to about 2000 mg. In some instances, the mono-hydrate Ca-AKG is administered at a dose of about 1800 mg to about 2000 mg. In some instances, the mono-hydrate Ca-AKG is administered at a dose of about 1900 mg to about 2000 mg. In some instances, the mono-hydrate Ca-AKG is administered at a dose of about 350 mg to about 1900 mg. In some instances, the mono-hydrate Ca-AKG is administered at a dose of about 350 mg to about 1800 mg. In some instances, the mono-hydrate Ca-AKG is administered at a dose of about 350 mg to about 1700 mg. In some instances, the mono-hydrate Ca-AKG is administered at a dose of about 350 mg to about 1600 mg. In some instances, the mono-hydrate Ca-AKG is administered at a dose of about 350 mg to about 1500 mg. In some instances, the mono-hydrate Ca-AKG is administered at a dose of about 350 mg to about 1400 mg. In some instances, the mono-hydrate Ca-AKG is administered at a dose of about 350 mg to about 1300 mg. In some instances, the mono-hydrate Ca-AKG is administered at a dose of about 350 mg to about 1200 mg. In some instances, the mono-hydrate Ca-AKG is administered at a dose of about 350 mg to about 1100 mg. In some instances, the mono-hydrate Ca-AKG is administered at a dose of about 350 mg to about 1000 mg. In some instances, the mono-hydrate Ca-AKG is administered at a dose of about 350 mg to about 900 mg. In some instances, the mono-hydrate Ca-AKG is administered at a dose of about 350 mg to about 800 mg. In some instances, the mono-hydrate Ca-AKG is administered at a dose of about 350 mg to about 700 mg. In some instances, the mono-hydrate Ca-AKG is administered at a dose of about 350 mg to about 650 mg. In some instances, the mono-hydrate Ca-AKG is administered at a dose of about 350 mg to about 600 mg. In some instances, the mono-hydrate Ca-AKG is administered at a dose of about 350 mg to about 550 mg. In some instances, the mono-hydrate Ca-AKG is administered at a dose of about 350 mg to about 500 mg. In some instances, the mono-hydrate Ca-AKG is administered at a dose of about 350 mg to about 450 mg. In some instances, the mono-hydrate Ca-AKG is administered at a dose of about 350 mg to about 400 mg. In some instances, the mono-hydrate Ca-AKG is administered at a dose of at least about 350 mg. In some instances, the mono-hydrate Ca-AKG is administered at a dose of at least about 400 mg. In some instances, the mono-hydrate Ca-AKG is administered at a dose of at least about 450 mg. In some instances, the mono-hydrate Ca-AKG is administered at a dose of at least about 500 mg. In some instances, the mono-hydrate Ca-AKG is administered at a dose of at least about 550 mg. In some instances, the mono-hydrate Ca-AKG is administered at a dose of at least about 600 mg. In some instances, the mono-hydrate Ca-AKG is administered at a dose of at least about 650 mg. In some instances, the mono-hydrate Ca-AKG is administered at a dose of at least about 700 mg. In some instances, the mono-hydrate Ca-AKG is administered at a dose of at least about 750 mg. In some instances, the mono-hydrate Ca-AKG is administered at a dose of at least about 800 mg. In some instances, the mono-hydrate Ca-AKG is administered at a dose of at least about 900 mg. In some instances, the mono-hydrate Ca-AKG is administered at a dose of at least about 1000 mg. In some instances, the mono-hydrate Ca-AKG is administered at a dose of at least about 1100 mg. In some instances, the mono-hydrate Ca-AKG is administered at a dose of at least about 1200 mg. In some instances, the mono-hydrate Ca-AKG is administered at a dose of at least about 1300 mg. In some instances, the mono-hydrate Ca-AKG is administered at a dose of at least about 1400 mg. In some instances, the mono-hydrate Ca-AKG is administered at a dose of at least about 1500 mg. In some instances, the mono-hydrate Ca-AKG is administered at a dose of at least about 1600 mg. In some instances, the mono-hydrate Ca-AKG is administered at a dose of at least about 1700 mg. In some instances, the mono-hydrate Ca-AKG is administered at a dose of at least about 1800 mg. In some instances, the mono-hydrate Ca-AKG is administered at a dose of at least about 1900 mg. In some instances, the mono-hydrate Ca-AKG is administered at a dose of at least about 2000 mg.

In some embodiments, the hemi-hydrate Ca-AKG is administered at a dose of at least 350 mg and no greater than 2000 mg. In some instances, the hemi-hydrate Ca-AKG is administered at a dose of about 350 mg to about 2000 mg. In some instances, the hemi-hydrate Ca-AKG is administered at a dose of about 400 mg to about 2000 mg. In some instances, the hemi-hydrate Ca-AKG is administered at a dose of about 450 mg to about 2000 mg. In some instances, the hemi-hydrate Ca-AKG is administered at a dose of about 500 mg to about 2000 mg. In some instances, the hemi-hydrate Ca-AKG is administered at a dose of about 550 mg to about 2000 mg. In some instances, the hemi-hydrate Ca-AKG is administered at a dose of about 600 mg to about 2000 mg. In some instances, the hemi-hydrate Ca-AKG is administered at a dose of about 650 mg to about 2000 mg. In some instances, the hemi-hydrate Ca-AKG is administered at a dose of about 700 mg to about 2000 mg. In some instances, the hemi-hydrate Ca-AKG is administered at a dose of about 800 mg to about 2000 mg. In some instances, the hemi-hydrate Ca-AKG is administered at a dose of about 900 mg to about 2000 mg. In some instances, the hemi-hydrate Ca-AKG is administered at a dose of about 1000 mg to about 2000 mg. In some instances, the hemi-hydrate Ca-AKG is administered at a dose of about 1100 mg to about 2000 mg. In some instances, the hemi-hydrate Ca-AKG is administered at a dose of about 1200 mg to about 2000 mg. In some instances, the hemi-hydrate Ca-AKG is administered at a dose of about 1300 mg to about 2000 mg. In some instances, the hemi-hydrate Ca-AKG is administered at a dose of about 1400 mg to about 2000 mg. In some instances, the hemi-hydrate Ca-AKG is administered at a dose of about 1500 mg to about 2000 mg. In some instances, the hemi-hydrate Ca-AKG is administered at a dose of about 1600 mg to about 2000 mg. In some instances, the hemi-hydrate Ca-AKG is administered at a dose of about 1700 mg to about 2000 mg. In some instances, the hemi-hydrate Ca-AKG is administered at a dose of about 1800 mg to about 2000 mg. In some instances, the hemi-hydrate Ca-AKG is administered at a dose of about 1900 mg to about 2000 mg. In some instances, the hemi-hydrate Ca-AKG is administered at a dose of about 350 mg to about 1900 mg. In some instances, the hemi-hydrate Ca-AKG is administered at a dose of about 350 mg to about 1800 mg. In some instances, the hemi-hydrate Ca-AKG is administered at a dose of about 350 mg to about 1700 mg. In some instances, the hemi-hydrate Ca-AKG is administered at a dose of about 350 mg to about 1600 mg. In some instances, the hemi-hydrate Ca-AKG is administered at a dose of about 350 mg to about 1500 mg. In some instances, the hemi-hydrate Ca-AKG is administered at a dose of about 350 mg to about 1400 mg. In some instances, the hemi-hydrate Ca-AKG is administered at a dose of about 350 mg to about 1300 mg. In some instances, the hemi-hydrate Ca-AKG is administered at a dose of about 350 mg to about 1200 mg. In some instances, the hemi-hydrate Ca-AKG is administered at a dose of about 350 mg to about 1100 mg. In some instances, the hemi-hydrate Ca-AKG is administered at a dose of about 350 mg to about 1000 mg. In some instances, the hemi-hydrate Ca-AKG is administered at a dose of about 350 mg to about 900 mg. In some instances, the hemi-hydrate Ca-AKG is administered at a dose of about 350 mg to about 800 mg. In some instances, the hemi-hydrate Ca-AKG is administered at a dose of about 350 mg to about 700 mg. In some instances, the hemi-hydrate Ca-AKG is administered at a dose of about 350 mg to about 650 mg. In some instances, the hemi-hydrate Ca-AKG is administered at a dose of about 350 mg to about 600 mg. In some instances, the hemi-hydrate Ca-AKG is administered at a dose of about 350 mg to about 550 mg. In some instances, the hemi-hydrate Ca-AKG is administered at a dose of about 350 mg to about 500 mg. In some instances, the hemi-hydrate Ca-AKG is administered at a dose of about 350 mg to about 450 mg. In some instances, the hemi-hydrate Ca-AKG is administered at a dose of about 350 mg to about 400 mg. In some instances, the hemi-hydrate Ca-AKG is administered at a dose of at least about 350 mg. In some instances, the hemi-hydrate Ca-AKG is administered at a dose of at least about 400 mg. In some instances, the hemi-hydrate Ca-AKG is administered at a dose of at least about 450 mg. In some instances, the hemi-hydrate Ca-AKG is administered at a dose of at least about 500 mg. In some instances, the hemi-hydrate Ca-AKG is administered at a dose of at least about 550 mg. In some instances, the hemi-hydrate Ca-AKG is administered at a dose of at least about 600 mg. In some instances, the hemi-hydrate Ca-AKG is administered at a dose of at least about 650 mg. In some instances, the hemi-hydrate Ca-AKG is administered at a dose of at least about 700 mg. In some instances, the hemi-hydrate Ca-AKG is administered at a dose of at least about 750 mg. In some instances, the hemi-hydrate Ca-AKG is administered at a dose of at least about 800 mg. In some instances, the hemi-hydrate Ca-AKG is administered at a dose of at least about 900 mg. In some instances, the hemi-hydrate Ca-AKG is administered at a dose of at least about 1000 mg. In some instances, the hemi-hydrate Ca-AKG is administered at a dose of at least about 1100 mg. In some instances, the hemi-hydrate Ca-AKG is administered at a dose of at least about 1200 mg. In some instances, the hemi-hydrate Ca-AKG is administered at a dose of at least about 1300 mg. In some instances, the hemi-hydrate Ca-AKG is administered at a dose of at least about 1400 mg. In some instances, the hemi-hydrate Ca-AKG is administered at a dose of at least about 1500 mg. In some instances, the hemi-hydrate Ca-AKG is administered at a dose of at least about 1600 mg. In some instances, the hemi-hydrate Ca-AKG is administered at a dose of at least about 1700 mg. In some instances, the hemi-hydrate Ca-AKG is administered at a dose of at least about 1800 mg. In some instances, the hemi-hydrate Ca-AKG is administered at a dose of at least about 1900 mg. In some instances, the hemi-hydrate Ca-AKG is administered at a dose of at least about 2000 mg.

In some embodiments, the anhydrous Ca-AKG is administered at a dose of at least 350 mg and no greater than 2000 mg. In some instances, the anhydrous Ca-AKG is administered at a dose of about 350 mg to about 2000 mg. In some instances, the anhydrous Ca-AKG is administered at a dose of about 400 mg to about 2000 mg. In some instances, the anhydrous Ca-AKG is administered at a dose of about 450 mg to about 2000 mg. In some instances, the anhydrous Ca-AKG is administered at a dose of about 500 mg to about 2000 mg. In some instances, the anhydrous Ca-AKG is administered at a dose of about 550 mg to about 2000 mg. In some instances, the anhydrous Ca-AKG is administered at a dose of about 600 mg to about 2000 mg. In some instances, the anhydrous Ca-AKG is administered at a dose of about 650 mg to about 2000 mg. In some instances, the anhydrous Ca-AKG is administered at a dose of about 700 mg to about 2000 mg. In some instances, the anhydrous Ca-AKG is administered at a dose of about 800 mg to about 2000 mg. In some instances, the anhydrous Ca-AKG is administered at a dose of about 900 mg to about 2000 mg. In some instances, the anhydrous Ca-AKG is administered at a dose of about 1000 mg to about 2000 mg. In some instances, the anhydrous Ca-AKG is administered at a dose of about 1100 mg to about 2000 mg. In some instances, the anhydrous Ca-AKG is administered at a dose of about 1200 mg to about 2000 mg. In some instances, the anhydrous Ca-AKG is administered at a dose of about 1300 mg to about 2000 mg. In some instances, the anhydrous Ca-AKG is administered at a dose of about 1400 mg to about 2000 mg. In some instances, the anhydrous Ca-AKG is administered at a dose of about 1500 mg to about 2000 mg. In some instances, the anhydrous Ca-AKG is administered at a dose of about 1600 mg to about 2000 mg. In some instances, the anhydrous Ca-AKG is administered at a dose of about 1700 mg to about 2000 mg. In some instances, the anhydrous Ca-AKG is administered at a dose of about 1800 mg to about 2000 mg. In some instances, the anhydrous Ca-AKG is administered at a dose of about 1900 mg to about 2000 mg. In some instances, the anhydrous Ca-AKG is administered at a dose of about 350 mg to about 1900 mg. In some instances, the anhydrous Ca-AKG is administered at a dose of about 350 mg to about 1800 mg. In some instances, the anhydrous Ca-AKG is administered at a dose of about 350 mg to about 1700 mg. In some instances, the anhydrous Ca-AKG is administered at a dose of about 350 mg to about 1600 mg. In some instances, the anhydrous Ca-AKG is administered at a dose of about 350 mg to about 1500 mg. In some instances, the anhydrous Ca-AKG is administered at a dose of about 350 mg to about 1400 mg. In some instances, the anhydrous Ca-AKG is administered at a dose of about 350 mg to about 1300 mg. In some instances, the anhydrous Ca-AKG is administered at a dose of about 350 mg to about 1200 mg. In some instances, the anhydrous Ca-AKG is administered at a dose of about 350 mg to about 1100 mg. In some instances, the anhydrous Ca-AKG is administered at a dose of about 350 mg to about 1000 mg. In some instances, the anhydrous Ca-AKG is administered at a dose of about 350 mg to about 900 mg. In some instances, the anhydrous Ca-AKG is administered at a dose of about 350 mg to about 800 mg. In some instances, the anhydrous Ca-AKG is administered at a dose of about 350 mg to about 700 mg. In some instances, the anhydrous Ca-AKG is administered at a dose of about 350 mg to about 650 mg. In some instances, the anhydrous Ca-AKG is administered at a dose of about 350 mg to about 600 mg. In some instances, the anhydrous Ca-AKG is administered at a dose of about 350 mg to about 550 mg. In some instances, the anhydrous Ca-AKG is administered at a dose of about 350 mg to about 500 mg. In some instances, the anhydrous Ca-AKG is administered at a dose of about 350 mg to about 450 mg. In some instances, the anhydrous Ca-AKG is administered at a dose of about 350 mg to about 400 mg. In some instances, the anhydrous Ca-AKG is administered at a dose of at least about 350 mg. In some instances, the anhydrous Ca-AKG is administered at a dose of at least about 400 mg. In some instances, the anhydrous Ca-AKG is administered at a dose of at least about 450 mg. In some instances, the anhydrous Ca-AKG is administered at a dose of at least about 500 mg. In some instances, the anhydrous Ca-AKG is administered at a dose of at least about 550 mg. In some instances, the anhydrous Ca-AKG is administered at a dose of at least about 600 mg. In some instances, the anhydrous Ca-AKG is administered at a dose of at least about 650 mg. In some instances, the anhydrous Ca-AKG is administered at a dose of at least about 700 mg. In some instances, the anhydrous Ca-AKG is administered at a dose of at least about 750 mg. In some instances, the anhydrous Ca-AKG is administered at a dose of at least about 800 mg. In some instances, the anhydrous Ca-AKG is administered at a dose of at least about 900 mg. In some instances, the anhydrous Ca-AKG is administered at a dose of at least about 1000 mg. In some instances, the anhydrous Ca-AKG is administered at a dose of at least about 1100 mg. In some instances, the anhydrous Ca-AKG is administered at a dose of at least about 1200 mg. In some instances, the anhydrous Ca-AKG is administered at a dose of at least about 1300 mg. In some instances, the anhydrous Ca-AKG is administered at a dose of at least about 1400 mg. In some instances, the anhydrous Ca-AKG is administered at a dose of at least about 1500 mg. In some instances, the anhydrous Ca-AKG is administered at a dose of at least about 1600 mg. In some instances, the anhydrous Ca-AKG is administered at a dose of at least about 1700 mg. In some instances, the anhydrous Ca-AKG is administered at a dose of at least about 1800 mg. In some instances, the anhydrous Ca-AKG is administered at a dose of at least about 1900 mg. In some instances, the anhydrous Ca-AKG is administered at a dose of at least about 2000 mg.

In some embodiments, administration of compositions comprising AKG compresses morbidity in a subject in need thereof. In some embodiments, administration of compositions comprising berberine, AKG, and vitamin A compresses morbidity in a subject in need thereof. In some embodiments, administration of compositions comprising AKG and vitamin A compresses morbidity in a subject in need thereof. In some embodiments, administration of compositions comprising AKG and vitamin D compresses morbidity in a subject in need thereof. In some embodiments, administration of compositions comprising AKG and NMN compresses morbidity in a subject in need thereof.

In some embodiments, compressing morbidity comprises a reduction in time the subject is afflicted with a morbidity prior to death. In further embodiments, the morbidity is an age-related disease selected from the group consisting of cancer, kidney disease, obesity, type-2 diabetes, a neurodegenerative disease, heart disease, macular degeneration, and an autoimmune disease. In further embodiments, the morbidity is an age-related disease selected from the group consisting of obesity, type-2 diabetes, macular degeneration, and an autoimmune disease. In some instances, the age-related disease is cancer. In some instances, the age-related disease is kidney disease. In some instances, the age-related disease is obesity. In some instances, the age-related disease is type-2 diabetes. In some instances, the age-related disease is a neurodegenerative disease. In some instances, the age-related disease is a heart disease. In some instances, the age-related disease is a macular degeneration. In some instances, the age-related disease is an autoimmune disease.

In some embodiments, the reduction in time the subject is afflicted with the morbidity prior to death is at least 1 month after administration of the composition to the subject. In some embodiments, the reduction in time the subject is afflicted with the morbidity prior to death is at least 2 months after administration of the composition to the subject. In some embodiments, the reduction in time the subject is afflicted with the morbidity prior to death is at least 3 months after administration of the composition to the subject. In some embodiments, the reduction in time the subject is afflicted with the morbidity prior to death is at least 4 months after administration of the composition to the subject. In some embodiments, the reduction in time the subject is afflicted with the morbidity prior to death is at least 5 months after administration of the composition to the subject. In some embodiments, the reduction in time the subject is afflicted with the morbidity prior to death is at least 6 months after administration of the composition to the subject. In some embodiments, the reduction in time the subject is afflicted with the morbidity prior to death is at least 7 months after administration of the composition to the subject. In some embodiments, the reduction in time the subject is afflicted with the morbidity prior to death is at least 8 months after administration of the composition to the subject. In some embodiments, the reduction in time the subject is afflicted with the morbidity prior to death is at least 9 months after administration of the composition to the subject. In some embodiments, the reduction in time the subject is afflicted with the morbidity prior to death is at least 10 months after administration of the composition to the subject. In some embodiments the reduction in time the subject is afflicted with the morbidity prior to death is at least 11 months after administration of the composition to the subject. In some embodiments, the reduction in time the subject is afflicted with the morbidity prior to death is at least 12 months after administration of the composition to the subject. In some embodiments, the reduction in time the subject is afflicted with the morbidity prior to death is at least 18 months after administration of the composition to the subject. In some embodiments, the reduction in time the subject is afflicted with the morbidity prior to death is at least 24 months after administration of the composition to the subject. In some embodiments, the reduction in time the subject is afflicted with the morbidity prior to death is at least 30 months after administration of the composition to the subject. In some embodiments, the reduction in time the subject is afflicted with the morbidity prior to death is at least 36 months after administration of the composition to the subject. In some embodiments, the reduction in time the subject is afflicted with the morbidity prior to death is at least 4 years after administration of the composition to the subject. In some embodiments, the reduction in time the subject is afflicted with the morbidity prior to death is at least 5 years after administration of the composition to the subject. In some embodiments, the reduction in time the subject is afflicted with the morbidity prior to death is at least 10 years after administration of the composition to the subject.

In some embodiments, administration of the compositions slows the rate of progression or reverse changes in biomarkers of non-human or human aging. Biomarker strategies to measure aging are currently being developed and can be employed to test the effects of aging interventions. The most prominent is the epigenetic clock, which likely measures biologic age in cells from humans (see for e.g. Chen et al. Aging, 8:1844-1865(2016), PMID 27690265), dogs (see for e.g. Thompson et al. Aging 9:1055-1068(2017), PMID 28373601), and mice (see for e.g. Petkovich et al. Cell Metab 25:954-960(2017), PMID 28380383). Other biomarker strategies include, but are not limited to, inflammatory cytokine levels, p16INK4A protein levels in specific cell populations, telomere length and levels of specific metabolites. In some embodiments, the composition modulates a biomarker of DNA methylation.

In some embodiments, the subject is a mammal. In some embodiments, the mammal is a human. In some embodiments, the mammal is a dog. In some embodiments, the mammal is livestock. In some embodiments, the livestock is selected from the group consisting of cattle, sheep, goats, swine, poultry, and equine animals.

In some embodiments, the composition is formulated into an orally administered form. In further embodiments, the orally administered form comprises a tablet, a powder, a suspension, a serum, an emulsion, a capsule, a granule, a pill, a gel, a solution, or a syrup. In some instances, the orally administered form is a sustained release dosage form. In some instances, the orally administered form is formulated into animal feed. In some instances, the orally administered form is the gel. In some embodiments, the composition is formulated into an injectable administered form. In further embodiments, the injectable administered form comprises a liquid, a suspension, or a solution. In some instances, the injectable administered form is a sustained release dosage form. In some embodiments, the composition is formulated into a topically administered form. In further embodiments, the topically administered form is a cream, a foam, a gel, a lotion, an ointment, or a serum. In some embodiments, the composition is formulated into a hair care product. In further embodiments, the hair care product is a shampoo, a conditioner, hair spray, or a moisturizer.

In some embodiments, the composition further comprises a pharmaceutically acceptable excipient. In further embodiments, the pharmaceutically acceptable excipient comprises an antiadherent, a binder, a coating, a color, disintegrant, a flavor, a glidant, a lubricant, a preservative, a sorbent, or a vehicle. In some instances, the composition further comprises silymarin.

In some embodiments, the composition is administered for a particular time period. In some embodiments, the composition is administered for a chronic treatment period, which is, for an extended period of time, including throughout the duration of the subject's life in order to ameliorate or otherwise control or limit the symptoms of the subject's disease or condition. Within the treatment period, the composition is administered on a particular time schedule. In further embodiments, the composition is administered one, two, three, or four times daily. In some embodiments, the composition is administered once daily. In some embodiments, the composition is administered twice daily. In some embodiments, the composition is administered in the morning and evening. In some embodiments, the composition is administered one, two, three, or four times weekly. In some embodiments, the composition is administered once a week. In some embodiments the composition is administered one, two, three, or four times monthly. In some embodiments, the composition is administered once a month. In some embodiments, the composition is administered for at least three months of every one year. In some embodiments, the composition is administered one month of every six months.

### Inhibition of Senescent Cell Formation

In an aspect, the disclosure provides methods of reducing formation of senescent cells using the active agents or compositions thereof disclosed herein. A "senescent cell" is generally thought to be derived from a cell type that typically replicates, but as a result of aging or other event that causes a change in cell state, can no longer replicate. The nucleus of senescent cells is often characterized by senescence-associated heterochromatin foci and DNA segments with chromatin alterations reinforcing senescence. It remains metabolically active and commonly adopts a senescence associated secretory phenotype (SASP). The SASP can include a combination of inflammatory cytokines, growth factors, and proteases. Without being bound by a particulary theory, the SASP secreted by senescent cells can contribute to the onset and progression of age-related diseases.

In an aspect, the disclosure provides methods of reducing formation of senescent cells using the active agents or compositions thereof disclosed herein. In some aspects, the composition comprises two or more active agents selected from the group consisting of: alpha-ketoglutarate, berberine, vitamin A, vitamin D and nicotinamide mononucleotide (NMN). In certain aspects, the composition comprises berberine, α-ketoglutarate (AKG), and vitamin A. In certain aspects, the composition comprises berberine, α-ketoglutarate (AKG), and retinoic acid (RA). In certain aspects, the composition comprises berberine, α-ketoglutarate (AKG), and retinyl palmitate (RP). In certain aspects, the composition comprises AKG and vitamin A. In certain aspects, the composition comprises AKG and retinoic acid (RA). In certain aspects, the composition comprises AKG and retinyl palmitate (RP). In certain aspects, the composition comprises AKG and vitamin D. In certain aspects, the composition comprises AKG and vitamin D3. In certain aspects, the composition comprises AKG and nicotinamide mononucleotide (NMN). In some instances, the AKG in the compositions is a salt of AKG. In some instances, the AKG provided in the compositions is a calcium salt of AKG (Ca-AKG). In some embodiments, the two or more compounds comprise alpha-ketoglutarate and berberine. In yet other embodiments, the two or more compounds comprise berberine and vitamin A. In some aspects, disclosure further provides compositions comprising an active agent selected from the group consisting of: alpha-ketoglutarate, berberine, vitamin A and vitamin D. In certain aspects, the compound is AKG. In some embodiments, AKG is provided as a salt. In some embodiments, AKG is provided as a calcium salt (Ca-AKG).

In an aspect, the disclosure provides methods of reducing formation of senescent cells in a subject caused by a senescence stimulus comprising administering to the subject a composition comprising an effective amount of Ca-AKG to reduce the formation of senescent cells upon exposure to the senescence stimulus. In another aspect, the disclosure provides methods of reducing formation of senescent cells in a subject caused by a senescence stimulus comprising administering to the subject a composition consisting essentially of an effective amount of Ca-AKG to reduce the formation of senescent cells upon exposure to the senescence stimulus. In some embodiments, calcium α-ketoglutarate can be a hydrate calcium α-ketoglutarate. In some embodiments, calcium α-ketoglutarate can be a mono-hydrate calcium α-ketoglutarate. In some embodiments, calcium α-ketoglutarate can be hemi-hydrate calcium α-ketoglutarate. In some embodiments, calcium α-ketoglutarate can be anhydrous calcium α-ketoglutarate.

In some embodiments, the Ca-AKG is administered at a dose of at least 350 mg and no greater than 2000 mg. In some instances, the Ca-AKG is administered at a dose of about 350 mg to about 2000 mg. In some instances, the Ca-AKG is administered at a dose of about 400 mg to about 2000 mg. In some instances, the Ca-AKG is administered at a dose of about 450 mg to about 2000 mg. In some instances, the Ca-AKG is administered at a dose of about 500 mg to about 2000 mg. In some instances, the Ca-AKG is administered at a dose of about 550 mg to about 2000 mg. In some instances, the Ca-AKG is administered at a dose of about 600 mg to about 2000 mg. In some instances, the Ca-AKG is administered at a dose of about 650 mg to about 2000 mg. In some instances, the Ca-AKG is administered at a dose of about 700 mg to about 2000 mg. In some instances, the Ca-AKG is administered at a dose of about 800 mg to about 2000 mg. In some instances, the Ca-AKG is administered at a dose of about 900 mg to about 2000 mg. In some instances, the Ca-AKG is administered at a dose of about 1000 mg to about 2000 mg. In some instances, the Ca-AKG is administered at a dose of about 1100 mg to about 2000 mg. In some instances, the Ca-AKG is administered at a dose of about 1200 mg to about 2000 mg. In some instances, the Ca-AKG is administered at a dose of about 1300 mg to about 2000 mg. In some instances, the Ca-AKG is administered at a dose of about 1400 mg to about 2000 mg. In some instances, the Ca-AKG is administered at a dose of about 1500 mg to about 2000 mg. In some instances, the Ca-AKG is administered at a dose of about 1600 mg to about 2000 mg. In some instances, the Ca-AKG is administered at a dose of about 1700 mg to about 2000 mg. In some instances, the Ca-AKG is administered at a dose of about 1800 mg to about 2000 mg. In some instances, the Ca-AKG is administered at a dose of about 1900 mg to about 2000 mg. In some instances, the Ca-AKG is administered at a dose of about 350 mg to about 1900 mg. In some instances, the Ca-AKG is administered at a dose of about 350 mg to about 1800 mg. In some instances, the Ca-AKG is administered at a dose of about 350 mg to about 1700 mg. In some instances, the Ca-AKG is administered at a dose of about 350 mg to about 1600 mg. In some instances, the Ca-AKG is administered at a dose of about 350 mg to about 1500 mg. In some instances, the Ca-AKG is administered at a dose of about 350 mg to about 1400 mg. In some instances, the Ca-AKG is administered at a dose of about 350 mg to about 1300 mg. In some instances, the Ca-AKG is administered at a dose of about 350 mg to about 1200 mg. In some instances, the Ca-AKG is administered at a dose of about 350 mg to about 1100 mg. In some instances, the Ca-AKG is administered at a dose of about 350 mg to about 1000 mg. In some instances, the Ca-AKG is administered at a dose of about 350 mg to about 900 mg. In some instances, the Ca-AKG is administered at a dose of about 350 mg to about 800 mg. In some instances, the Ca-AKG is administered at a dose of about 350 mg to about 700 mg. In some instances, the Ca-AKG is administered at a dose of about 350 mg to about 650 mg. In some instances, the Ca-AKG is administered at a dose of about 350 mg to about 600 mg. In some instances, the Ca-AKG is administered at a dose of about 350 mg to about 550 mg. In some instances, the Ca-AKG is administered at a dose of about 350 mg to about 500 mg. In some instances, the Ca-AKG is administered at a dose of about 350 mg to about 450 mg. In some instances, the Ca-AKG is administered at a dose of about 350 mg to about 400 mg. In some instances, the Ca-AKG is administered at a dose of at least about 350 mg. In some instances, the Ca-AKG is administered at a dose of at least about 400 mg. In some instances, the Ca-AKG is administered at a dose of at least about 450 mg. In some instances, the Ca-AKG is administered at a dose of at least about 500 mg. In some instances, the Ca-AKG is administered at a dose of at least about 550 mg. In some instances, the Ca-AKG is administered at a dose of at least about 600 mg. In some instances, the Ca-AKG is administered at a dose of at least about 650 mg. In some instances, the Ca-AKG is administered at a dose of at least about 700 mg. In some instances, the Ca-AKG is administered at a dose of at least about 750 mg. In some instances, the Ca-AKG is administered at a dose of at least about 800 mg. In some instances, the Ca-AKG is administered at a dose of at least about 900 mg. In some instances, the Ca-AKG is administered at a dose of at least about 1000 mg. In some instances, the Ca-AKG is administered at a dose of at least about 1100 mg. In some instances, the Ca-AKG is administered at a dose of at least about 1200 mg. In some instances, the Ca-AKG is administered at a dose of at least about 1300 mg. In some instances, the Ca-AKG is administered at a dose of at least about 1400 mg. In some instances, the Ca-AKG is administered at a dose of at least about 1500 mg. In some instances, the Ca-AKG is administered at a dose of at least about 1600 mg. In some instances, the Ca-AKG is administered at a dose of at least about 1700 mg. In some instances, the Ca-AKG is administered at a dose of at least about 1800 mg. In some instances, the Ca-AKG is administered at a dose of at least about 1900 mg. In some instances, the Ca-AKG is administered at a dose of at least about 2000 mg.

In some embodiments, the hydrate Ca-AKG is administered at a dose of at least 350 mg and no greater than 2000 mg. In some instances, the hydrate Ca-AKG is administered at a dose of about 350 mg to about 2000 mg. In some instances, the hydrate Ca-AKG is administered at a dose of about 400 mg to about 2000 mg. In some instances, the hydrate Ca-AKG is administered at a dose of about 450 mg to about 2000 mg. In some instances, the hydrate Ca-AKG is administered at a dose of about 500 mg to about 2000 mg. In some instances, the hydrate Ca-AKG is administered at a dose of about 550 mg to about 2000 mg. In some instances, the hydrate Ca-AKG is administered at a dose of about 600 mg to about 2000 mg. In some instances, the hydrate Ca-AKG is administered at a dose of about 650 mg to about 2000 mg. In some instances, the hydrate Ca-AKG is administered at a dose of about 700 mg to about 2000 mg. In some instances, the hydrate Ca-AKG is administered at a dose of about 800 mg to about 2000 mg. In some instances, the hydrate Ca-AKG is administered at a dose of about 900 mg to about 2000 mg. In some instances, the hydrate Ca-AKG is administered at a dose of about 1000 mg to about 2000 mg. In some instances, the hydrate Ca-AKG is administered at a dose of about 1100 mg to about 2000 mg. In some instances, the hydrate Ca-AKG is administered at a dose of about 1200 mg to about 2000 mg. In some instances, the hydrate Ca-AKG is administered at a dose of about 1300 mg to about 2000 mg. In some instances, the hydrate Ca-AKG is administered at a dose of about 1400 mg to about 2000 mg. In some instances, the hydrate Ca-AKG is administered at a dose of about 1500 mg to about 2000 mg. In some instances, the hydrate Ca-AKG is administered at a dose of about 1600 mg to about 2000 mg. In some instances, the hydrate Ca-AKG is administered at a dose of about 1700 mg to about 2000 mg. In some instances, the hydrate Ca-AKG is administered at a dose of about 1800 mg to about 2000 mg. In some instances, the hydrate Ca-AKG is administered at a dose of about 1900 mg to about 2000 mg. In some instances, the hydrate Ca-AKG is administered at a dose of about 350 mg to about 1900 mg. In some instances, the hydrate Ca-AKG is administered at a dose of about 350 mg to about 1800 mg. In some instances, the hydrate Ca-AKG is administered at a dose of about 350 mg to about 1700 mg. In some instances, the hydrate Ca-AKG is administered at a dose of about 350 mg to about 1600 mg. In some instances, the hydrate Ca-AKG is administered at a dose of about 350 mg to about 1500 mg. In some instances, the hydrate Ca-AKG is administered at a dose of about 350 mg to about 1400 mg. In some instances, the hydrate Ca-AKG is administered at a dose of about 350 mg to about 1300 mg. In some instances, the hydrate Ca-AKG is administered at a dose of about 350 mg to about 1200 mg. In some instances, the hydrate Ca-AKG is administered at a dose of about 350 mg to about 1100 mg. In some instances, the hydrate Ca-AKG is administered at a dose of about 350 mg to about 1000 mg. In some instances, the hydrate Ca-AKG is administered at a dose of about 350 mg to about 900 mg. In some instances, the hydrate Ca-AKG is administered at a dose of about 350 mg to about 800 mg. In some instances, the hydrate Ca-AKG is administered at a dose of about 350 mg to about 700 mg. In some instances, the hydrate Ca-AKG is administered at a dose of about 350 mg to about 650 mg. In some instances, the hydrate Ca-AKG is administered at a dose of about 350 mg to about 600 mg. In some instances, the hydrate Ca-AKG is administered at a dose of about 350 mg to about 550 mg. In some instances, the hydrate Ca-AKG is administered at a dose of about 350 mg to about 500 mg. In some instances, the hydrate Ca-AKG is administered at a dose of about 350 mg to about 450 mg. In some instances, the hydrate Ca-AKG is administered at a dose of about 350 mg to about 400 mg. In some instances, the hydrate Ca-AKG is administered at a dose of at least about 350 mg. In some instances, the hydrate Ca-AKG is administered at a dose of at least about 400 mg. In some instances, the hydrate Ca-AKG is administered at a dose of at least about 450 mg. In some instances, the hydrate Ca-AKG is administered at a dose of at least about 500 mg. In some instances, the hydrate Ca-AKG is administered at a dose of at least about 550 mg. In some instances, the hydrate Ca-AKG is administered at a dose of at least about 600 mg. In some instances, the hydrate Ca-AKG is administered at a dose of at least about 650 mg. In some instances, the hydrate Ca-AKG is administered at a dose of at least about 700 mg. In some instances, the hydrate Ca-AKG is administered at a dose of at least about 750 mg. In some instances, the hydrate Ca-AKG is administered at a dose of at least about 800 mg. In some instances, the hydrate Ca-AKG is administered at a dose of at least about 900 mg. In some instances, the hydrate Ca-AKG is administered at a dose of at least about 1000 mg. In some instances, the hydrate Ca-AKG is administered at a dose of at least about 1100 mg. In some instances, the hydrate Ca-AKG is administered at a dose of at least about 1200 mg. In some instances, the hydrate Ca-AKG is administered at a dose of at least about 1300 mg. In some instances, the hydrate Ca-AKG is administered at a dose of at least about 1400 mg. In some instances, the hydrate Ca-AKG is administered at a dose of at least about 1500 mg. In some instances, the hydrate Ca-AKG is administered at a dose of at least about 1600 mg. In some instances, the hydrate Ca-AKG is administered at a dose of at least about 1700 mg. In some instances, the hydrate Ca-AKG is administered at a dose of at least about 1800 mg. In some instances, the hydrate Ca-AKG is administered at a dose of at least about 1900 mg. In some instances, the hydrate Ca-AKG is administered at a dose of at least about 2000 mg.

In some embodiments, the mono-hydrate Ca-AKG is administered at a dose of at least 350 mg and no greater than 2000 mg. In some instances, the mono-hydrate Ca-AKG is administered at a dose of about 350 mg to about 2000 mg. In some instances, the mono-hydrate Ca-AKG is administered at a dose of about 400 mg to about 2000 mg. In some instances, the mono-hydrate Ca-AKG is administered at a dose of about 450 mg to about 2000 mg. In some instances, the mono-hydrate Ca-AKG is administered at a dose of about 500 mg to about 2000 mg. In some instances, the mono-hydrate Ca-AKG is administered at a dose of about 550 mg to about 2000 mg. In some instances, the mono-hydrate Ca-AKG is administered at a dose of about 600 mg to about 2000 mg. In some instances, the mono-hydrate Ca-AKG is administered at a dose of about 650 mg to about 2000 mg. In some instances, the mono-hydrate Ca-AKG is administered at a dose of about 700 mg to about 2000 mg. In some instances, the mono-hydrate Ca-AKG is administered at a dose of about 800 mg to about 2000 mg. In some instances, the mono-hydrate Ca-AKG is administered at a dose of about 900 mg to about 2000 mg. In some instances, the mono-hydrate Ca-AKG is administered at a dose of about 1000 mg to about 2000 mg. In some instances, the mono-hydrate Ca-AKG is administered at a dose of about 1100 mg to about 2000 mg. In some instances, the mono-hydrate Ca-AKG is administered at a dose of about 1200 mg to about 2000 mg. In some instances, the mono-hydrate Ca-AKG is administered at a dose of about 1300 mg to about 2000 mg. In some instances, the mono-hydrate Ca-AKG is administered at a dose of about 1400 mg to about 2000 mg. In some instances, the mono-hydrate Ca-AKG is administered at a dose of about 1500 mg to about 2000 mg. In some instances, the mono-hydrate Ca-AKG is administered at a dose of about 1600 mg to about 2000 mg. In some instances, the mono-hydrate Ca-AKG is administered at a dose of about 1700 mg to about 2000 mg. In some instances, the mono-hydrate Ca-AKG is administered at a dose of about 1800 mg to about 2000 mg. In some instances, the mono-hydrate Ca-AKG is administered at a dose of about 1900 mg to about 2000 mg. In some instances, the mono-hydrate Ca-AKG is administered at a dose of about 350 mg to about 1900 mg. In some instances, the mono-hydrate Ca-AKG is administered at a dose of about 350 mg to about 1800 mg. In some instances, the mono-hydrate Ca-AKG is administered at a dose of about 350 mg to about 1700 mg. In some instances, the mono-hydrate Ca-AKG is administered at a dose of about 350 mg to about 1600 mg. In some instances, the mono-hydrate Ca-AKG is administered at a dose of about 350 mg to about 1500 mg. In some instances, the mono-hydrate Ca-AKG is administered at a dose of about 350 mg to about 1400 mg. In some instances, the mono-hydrate Ca-AKG is administered at a dose of about 350 mg to about 1300 mg. In some instances, the mono-hydrate Ca-AKG is administered at a dose of about 350 mg to about 1200 mg. In some instances, the mono-hydrate Ca-AKG is administered at a dose of about 350 mg to about 1100 mg. In some instances, the mono-hydrate Ca-AKG is administered at a dose of about 350 mg to about 1000 mg. In some instances, the mono-hydrate Ca-AKG is administered at a dose of about 350 mg to about 900 mg. In some instances, the mono-hydrate Ca-AKG is administered at a dose of about 350 mg to about 800 mg. In some instances, the mono-hydrate Ca-AKG is administered at a dose of about 350 mg to about 700 mg. In some instances, the mono-hydrate Ca-AKG is administered at a dose of about 350 mg to about 650 mg. In some instances, the mono-hydrate Ca-AKG is administered at a dose of about 350 mg to about 600 mg. In some instances, the mono-hydrate Ca-AKG is administered at a dose of about 350 mg to about 550 mg. In some instances, the mono-hydrate Ca-AKG is administered at a dose of about 350 mg to about 500 mg. In some instances, the mono-hydrate Ca-AKG is administered at a dose of about 350 mg to about 450 mg. In some instances, the mono-hydrate Ca-AKG is administered at a dose of about 350 mg to about 400 mg. In some instances, the mono-hydrate Ca-AKG is administered at a dose of at least about 350 mg. In some instances, the mono-hydrate Ca-AKG is administered at a dose of at least about 400 mg. In some instances, the mono-hydrate Ca-AKG is administered at a dose of at least about 450 mg. In some instances, the mono-hydrate Ca-AKG is administered at a dose of at least about 500 mg. In some instances, the mono-hydrate Ca-AKG is administered at a dose of at least about 550 mg. In some instances, the mono-hydrate Ca-AKG is administered at a dose of at least about 600 mg. In some instances, the mono-hydrate Ca-AKG is administered at a dose of at least about 650 mg. In some instances, the mono-hydrate Ca-AKG is administered at a dose of at least about 700 mg. In some instances, the mono-hydrate Ca-AKG is administered at a dose of at least about 750 mg. In some instances, the mono-hydrate Ca-AKG is administered at a dose of at least about 800 mg. In some instances, the mono-hydrate Ca-AKG is administered at a dose of at least about 900 mg. In some instances, the mono-hydrate Ca-AKG is administered at a dose of at least about 1000 mg. In some instances, the mono-hydrate Ca-AKG is administered at a dose of at least about 1100 mg. In some instances, the mono-hydrate Ca-AKG is administered at a dose of at least about 1200 mg. In some instances, the mono-hydrate Ca-AKG is administered at a dose of at least about 1300 mg. In some instances, the mono-hydrate Ca-AKG is administered at a dose of at least about 1400 mg. In some instances, the mono-hydrate Ca-AKG is administered at a dose of at least about 1500 mg. In some instances, the mono-hydrate Ca-AKG is administered at a dose of at least about 1600 mg. In some instances, the mono-hydrate Ca-AKG is administered at a dose of at least about 1700 mg. In some instances, the mono-hydrate Ca-AKG is administered at a dose of at least about 1800 mg. In some instances, the mono-hydrate Ca-AKG is administered at a dose of at least about 1900 mg. In some instances, the mono-hydrate Ca-AKG is administered at a dose of at least about 2000 mg.

In some embodiments, the hemi-hydrate Ca-AKG is administered at a dose of at least 350 mg and no greater than 2000 mg. In some instances, the hemi-hydrate Ca-AKG is administered at a dose of about 350 mg to about 2000 mg. In some instances, the hemi-hydrate Ca-AKG is administered at a dose of about 400 mg to about 2000 mg. In some instances, the hemi-hydrate Ca-AKG is administered at a dose of about 450 mg to about 2000 mg. In some instances, the hemi-hydrate Ca-AKG is administered at a dose of about 500 mg to about 2000 mg. In some instances, the hemi-hydrate Ca-AKG is administered at a dose of about 550 mg to about 2000 mg. In some instances, the hemi-hydrate Ca-AKG is administered at a dose of about 600 mg to about 2000 mg. In some instances, the hemi-hydrate Ca-AKG is administered at a dose of about 650 mg to about 2000 mg. In some instances, the hemi-hydrate Ca-AKG is administered at a dose of about 700 mg to about 2000 mg. In some instances, the hemi-hydrate Ca-AKG is administered at a dose of about 800 mg to about 2000 mg. In some instances, the hemi-hydrate Ca-AKG is administered at a dose of about 900 mg to about 2000 mg. In some instances, the hemi-hydrate Ca-AKG is administered at a dose of about 1000 mg to about 2000 mg. In some instances, the hemi-hydrate Ca-AKG is administered at a dose of about 1100 mg to about 2000 mg. In some instances, the hemi-hydrate Ca-AKG is administered at a dose of about 1200 mg to about 2000 mg. In some instances, the hemi-hydrate Ca-AKG is administered at a dose of about 1300 mg to about 2000 mg. In some instances, the hemi-hydrate Ca-AKG is administered at a dose of about 1400 mg to about 2000 mg. In some instances, the hemi-hydrate Ca-AKG is administered at a dose of about 1500 mg to about 2000 mg. In some instances, the hemi-hydrate Ca-AKG is administered at a dose of about 1600 mg to about 2000 mg. In some instances, the hemi-hydrate Ca-AKG is administered at a dose of about 1700 mg to about 2000 mg. In some instances, the hemi-hydrate Ca-AKG is administered at a dose of about 1800 mg to about 2000 mg. In some instances, the hemi-hydrate Ca-AKG is administered at a dose of about 1900 mg to about 2000 mg. In some instances, the hemi-hydrate Ca-AKG is administered at a dose of about 350 mg to about 1900 mg. In some instances, the hemi-hydrate Ca-AKG is administered at a dose of about 350 mg to about 1800 mg. In some instances, the hemi-hydrate Ca-AKG is administered at a dose of about 350 mg to about 1700 mg. In some instances, the hemi-hydrate Ca-AKG is administered at a dose of about 350 mg to about 1600 mg. In some instances, the hemi-hydrate Ca-AKG is administered at a dose of about 350 mg to about 1500 mg. In some instances, the hemi-hydrate Ca-AKG is administered at a dose of about 350 mg to about 1400 mg. In some instances, the hemi-hydrate Ca-AKG is administered at a dose of about 350 mg to about 1300 mg. In some instances, the hemi-hydrate Ca-AKG is administered at a dose of about 350 mg to about 1200 mg. In some instances, the hemi-hydrate Ca-AKG is administered at a dose of about 350 mg to about 1100 mg. In some instances, the hemi-hydrate Ca-AKG is administered at a dose of about 350 mg to about 1000 mg. In some instances, the hemi-hydrate Ca-AKG is administered at a dose of about 350 mg to about 900 mg. In some instances, the hemi-hydrate Ca-AKG is administered at a dose of about 350 mg to about 800 mg. In some instances, the hemi-hydrate Ca-AKG is administered at a dose of about 350 mg to about 700 mg. In some instances, the hemi-hydrate Ca-AKG is administered at a dose of about 350 mg to about 650 mg. In some instances, the hemi-hydrate Ca-AKG is administered at a dose of about 350 mg to about 600 mg. In some instances, the hemi-hydrate Ca-AKG is administered at a dose of about 350 mg to about 550 mg. In some instances, the hemi-hydrate Ca-AKG is administered at a dose of about 350 mg to about 500 mg. In some instances, the hemi-hydrate Ca-AKG is administered at a dose of about 350 mg to about 450 mg. In some instances, the hemi-hydrate Ca-AKG is administered at a dose of about 350 mg to about 400 mg. In some instances, the hemi-hydrate Ca-AKG is administered at a dose of at least about 350 mg. In some instances, the hemi-hydrate Ca-AKG is administered at a dose of at least about 400 mg. In some instances, the hemi-hydrate Ca-AKG is administered at a dose of at least about 450 mg. In some instances, the hemi-hydrate Ca-AKG is administered at a dose of at least about 500 mg. In some instances, the hemi-hydrate Ca-AKG is administered at a dose of at least about 550 mg. In some instances, the hemi-hydrate Ca-AKG is administered at a dose of at least about 600 mg. In some instances, the hemi-hydrate Ca-AKG is administered at a dose of at least about 650 mg. In some instances, the hemi-hydrate Ca-AKG is administered at a dose of at least about 700 mg. In some instances, the hemi-hydrate Ca-AKG is administered at a dose of at least about 750 mg. In some instances, the hemi-hydrate Ca-AKG is administered at a dose of at least about 800 mg. In some instances, the hemi-hydrate Ca-AKG is administered at a dose of at least about 900 mg. In some instances, the hemi-hydrate Ca-AKG is administered at a dose of at least about 1000 mg. In some instances, the hemi-hydrate Ca-AKG is administered at a dose of at least about 1100 mg. In some instances, the hemi-hydrate Ca-AKG is administered at a dose of at least about 1200 mg. In some instances, the hemi-hydrate Ca-AKG is administered at a dose of at least about 1300 mg. In some instances, the hemi-hydrate Ca-AKG is administered at a dose of at least about 1400 mg. In some instances, the hemi-hydrate Ca-AKG is administered at a dose of at least about 1500 mg. In some instances, the hemi-hydrate Ca-AKG is administered at a dose of at least about 1600 mg. In some instances, the hemi-hydrate Ca-AKG is administered at a dose of at least about 1700 mg. In some instances, the hemi-hydrate Ca-AKG is administered at a dose of at least about 1800 mg. In some instances, the hemi-hydrate Ca-AKG is administered at a dose of at least about 1900 mg. In some instances, the hemi-hydrate Ca-AKG is administered at a dose of at least about 2000 mg.

In some embodiments, the anhydrous Ca-AKG is administered at a dose of at least 350 mg and no greater than 2000 mg. In some instances, the anhydrous Ca-AKG is administered at a dose of about 350 mg to about 2000 mg. In some instances, the anhydrous Ca-AKG is administered at a dose of about 400 mg to about 2000 mg. In some instances, the anhydrous Ca-AKG is administered at a dose of about 450 mg to about 2000 mg. In some instances, the anhydrous Ca-AKG is administered at a dose of about 500 mg to about 2000 mg. In some instances, the anhydrous Ca-AKG is administered at a dose of about 550 mg to about 2000 mg. In some instances, the anhydrous Ca-AKG is administered at a dose of about 600 mg to about 2000 mg. In some instances, the anhydrous Ca-AKG is administered at a dose of about 650 mg to about 2000 mg. In some instances, the anhydrous Ca-AKG is administered at a dose of about 700 mg to about 2000 mg. In some instances, the anhydrous Ca-AKG is administered at a dose of about 800 mg to about 2000 mg. In some instances, the anhydrous Ca-AKG is administered at a dose of about 900 mg to about 2000 mg. In some instances, the anhydrous Ca-AKG is administered at a dose of about 1000 mg to about 2000 mg. In some instances, the anhydrous Ca-AKG is administered at a dose of about 1100 mg to about 2000 mg. In some instances, the anhydrous Ca-AKG is administered at a dose of about 1200 mg to about 2000 mg. In some instances, the anhydrous Ca-AKG is administered at a dose of about 1300 mg to about 2000 mg. In some instances, the anhydrous Ca-AKG is administered at a dose of about 1400 mg to about 2000 mg. In some instances, the anhydrous Ca-AKG is administered at a dose of about 1500 mg to about 2000 mg. In some instances, the anhydrous Ca-AKG is administered at a dose of about 1600 mg to about 2000 mg. In some instances, the anhydrous Ca-AKG is administered at a dose of about 1700 mg to about 2000 mg. In some instances, the anhydrous Ca-AKG is administered at a dose of about 1800 mg to about 2000 mg. In some instances, the anhydrous Ca-AKG is administered at a dose of about 1900 mg to about 2000 mg. In some instances, the anhydrous Ca-AKG is administered at a dose of about 350 mg to about 1900 mg. In some instances, the anhydrous Ca-AKG is administered at a dose of about 350 mg to about 1800 mg. In some instances, the anhydrous Ca-AKG is administered at a dose of about 350 mg to about 1700 mg. In some instances, the anhydrous Ca-AKG is administered at a dose of about 350 mg to about 1600 mg. In some instances, the anhydrous Ca-AKG is administered at a dose of about 350 mg to about 1500 mg. In some instances, the anhydrous Ca-AKG is administered at a dose of about 350 mg to about 1400 mg. In some instances, the anhydrous Ca-AKG is administered at a dose of about 350 mg to about 1300 mg. In some instances, the anhydrous Ca-AKG is administered at a dose of about 350 mg to about 1200 mg. In some instances, the anhydrous Ca-AKG is administered at a dose of about 350 mg to about 1100 mg. In some instances, the anhydrous Ca-AKG is administered at a dose of about 350 mg to about 1000 mg. In some instances, the anhydrous Ca-AKG is administered at a dose of about 350 mg to about 900 mg. In some instances, the anhydrous Ca-AKG is administered at a dose of about 350 mg to about 800 mg. In some instances, the anhydrous Ca-AKG is administered at a dose of about 350 mg to about 700 mg. In some instances, the anhydrous Ca-AKG is administered at a dose of about 350 mg to about 650 mg. In some instances, the anhydrous Ca-AKG is administered at a dose of about 350 mg to about 600 mg. In some instances, the anhydrous Ca-AKG is administered at a dose of about 350 mg to about 550 mg. In some instances, the anhydrous Ca-AKG is administered at a dose of about 350 mg to about 500 mg. In some instances, the anhydrous Ca-AKG is administered at a dose of about 350 mg to about 450 mg. In some instances, the anhydrous Ca-AKG is administered at a dose of about 350 mg to about 400 mg. In some instances, the anhydrous Ca-AKG is administered at a dose of at least about 350 mg. In some instances, the anhydrous Ca-AKG is administered at a dose of at least about 400 mg. In some instances, the anhydrous Ca-AKG is administered at a dose of at least about 450 mg. In some instances, the anhydrous Ca-AKG is administered at a dose of at least about 500 mg. In some instances, the anhydrous Ca-AKG is administered at a dose of at least about 550 mg. In some instances, the anhydrous Ca-AKG is administered at a dose of at least about 600 mg. In some instances, the anhydrous Ca-AKG is administered at a dose of at least about 650 mg. In some instances, the anhydrous Ca-AKG is administered at a dose of at least about 700 mg. In some instances, the anhydrous Ca-AKG is administered at a dose of at least about 750 mg. In some instances, the anhydrous Ca-AKG is administered at a dose of at least about 800 mg. In some instances, the anhydrous Ca-AKG is administered at a dose of at least about 900 mg. In some instances, the anhydrous Ca-AKG is administered at a dose of at least about 1000 mg. In some instances, the anhydrous Ca-AKG is administered at a dose of at least about 1100 mg. In some instances, the anhydrous Ca-AKG is administered at a dose of at least about 1200 mg. In some instances, the anhydrous Ca-AKG is administered at a dose of at least about 1300 mg. In some instances, the anhydrous Ca-AKG is administered at a dose of at least about 1400 mg. In some instances, the anhydrous Ca-AKG is administered at a dose of at least about 1500 mg. In some instances, the anhydrous Ca-AKG is administered at a dose of at least about 1600 mg. In some instances, the anhydrous Ca-AKG is administered at a dose of at least about 1700 mg. In some instances, the anhydrous Ca-AKG is administered at a dose of at least about 1800 mg. In some instances, the anhydrous Ca-AKG is administered at a dose of at least about 1900 mg. In some instances, the anhydrous Ca-AKG is administered at a dose of at least about 2000 mg.

In some embodiments, administration of compositions comprising AKG reduces formation of senescent cells in a subject in need thereof. In some embodiments, administration of compositions comprising berberine, AKG, and vitamin A reduces formation of senescent cells in a subject in need thereof. In some embodiments, administration of compositions comprising AKG and vitamin A reduces formation of senescent cells in a subject in need thereof. In some embodiments, administration of compositions comprising AKG and vitamin D reduces formation of senescent cells in a subject in need thereof. In some embodiments, administration of compositions comprising AKG and NMN reduces formation of senescent cells in a subject in need thereof.

In some embodiments, prior to administering the composition to the subject, a biological sample is collected from the subject. In some embodiments, the biological sample is selected from the group consisting of blood, plasma, saliva, urine, tissue, and cerebrospinal fluid. In some embodiments, the biological sample is split into a control biological sample and a test biological sample wherein the composition is applied to the test biological sample after the biological sample is split. In some embodiments, the senescence stimulus is applied to the control biological sample and the test biological sample, and the formation of senescent cells is measured in the control biological sample and the test biological sample. In some embodiments, the formation of senescent cells is measured by measuring the presence one or more senescence biomarkers. In some embodiments, the senescence biomarker is a molecule that is part of SASP. In some embodiments, the molecule that is part of SASP includes, but is not limited to GM-CSF, GROα, GROα,β,γ, IGFBP-7, IL-1α, IL-6, IL-7, IL-8, MCP-1, MCP-2, MIP-1α, MMP-1, MMP-10, MMP-3, Amphiregulin, ENA-78, Eotaxin-3, GCP-2, GITR, HGF, ICAM-1, IGFBP-2, IGFBP-4, IGFBP-5, IGFBP-6, IL-13, IL-1β, MCP-4, MIF, MIP-3α, MMP-12, MMP-13, MMP-14, NAP2, Oncostatin M, osteoprotegerin, PIGF, RANTES, sgp130, TIMP-2, TRAIL-R3, Acrp30, angiogenin, Axl, bFGF, BLC, BTC, CTACK, EGF-R, Fas, FGF-7, G-CSF, GDNF, HCC-4, I-309, IFN-γ, IGFBP-1, IGFBP-3, IL-1 R1, IL-11, IL-15, IL-2R-α, IL-6 R, I-TAC, Leptin, LIF, MMP-2, MSP-a, PAI-1, PAI-2, PDGF-BB, SCF, SDF-1, sTNF RI, sTNF RII, Thrombopoietin, TIMP-1, tPA, uPA, uPAR, VEGF, MCP-3, IGF-1, TGF-β3, MIP-1-delta, IL-4, FGF-7, PDGF-BB, IL-16, BMP-4, MDC, MCP-4, IL-10, TIMP-1, Fit-3 Ligand, ICAM-1, Axl, CNTF, INF-γ, EGF, BMP-6. In some embodiments, the senescence biomarker is selected from the group consisting of senescence-associated β-galactosidase, mmp-3, IL-6, p21, p16, and p53. In some embodiments, the senescence biomarker is senescence-associated β-galactosidase.In some embodiments, the formation of senescent cells is characterized by senescence associated heterochromatin foci (SAHF) or DNA segments with chromatin alteration reinforcing senescence (DNA-SCARS). In some embodiments, the test biological sample has reduced senescence biomarkers compared to the control biological sample which indicates a reduction in the formation of senescent cells in the test biological sample.

In some embodiments, the senescence stimulus is selected from the group consisting of chemotherapy treatment, irradiation, oxidative stress, and aging.

In some embodiments, the subject is a mammal. In some embodiments, the mammal is a human. In some embodiments, the mammal is a dog. In some embodiments, the mammal is livestock. In some embodiments, the livestock is selected from the group consisting of cattle, sheep, goats, swine, poultry, and equine animals.

In some embodiments, the composition is administered to the subject in an orally administered form. In some embodiments, the orally administered form comprises a tablet, a powder, a suspension, a serum, an emulsion, a capsule, a granule, a pill, a gel, a solution, or a syrup. In some embodiments, the orally administered form is a sustained release dosage form. In some embodiments, the orally administered form is formulated into animal feed. In some embodiments, the orally administered form is the gel. In some embodiments, the composition is administered to the subject in an injectable administered form. In some embodiments, the injectable administered form comprises a liquid, a suspension, or a solution. In some embodiments, the injectable administered form is a sustained release dosage form. In some embodiments, the composition is administered to the subject in a topically administered form. In some embodiments, the topically administered form is a cream, a foam, a gel, a lotion, an ointment, or a serum. In some embodiments, the composition is administered to the subject in a hair care product. In some embodiments, the hair care product is a shampoo, a conditioner, hair spray, or a moisturizer. In some embodiments, the composition further comprises a pharmaceutically acceptable excipient. In some embodiments, the pharmaceutically acceptable excipient comprises an antiadherent, a binder, a coating, a color, disintegrant, a flavor, a glidant, a lubricant, a preservative, a sorbent, or a vehicle. In some embodiments, the composition further comprises silymarin. In some embodiments, the composition is administered to the subject once daily.

In some embodiments, the composition is administered to the subject for a particular time period. In some embodiments, the composition is administered to the subject for a chronic treatment period, which is, for an extended period of time, including throughout the duration of the subject's life in order to ameliorate or otherwise control or limit the symptoms of the subject's disease or condition. Within the treatment period, the composition is administered to the subject on a particular time schedule. In further embodiments, the composition is administered to the subject one, two, three, or four times daily. In some embodiments, the composition is administered to the subject once daily. In some embodiments, the composition is administered to the subject twice daily. In some embodiments, the composition is administered to the subject in the morning and evening. In some embodiments, the composition is administered to the subject one, two, three, or four times weekly. In some embodiments, the composition is administered to the subject once a week. In some embodiments the composition is administered to the subject one, two, three, or four times monthly. In some embodiments, the composition is administered to the subject once a month. In some embodiments, the composition is administered to the subject for at least three months of every one year. In some embodiments, the composition is administered to the subject one month of every six months.

### Routes of Administration

The compositions according to the disclosure herein may be administered via a variety of routes. In some embodiments, the pharmaceutical composition is formulated for oral administration. In other embodiments, the pharmaceutical composition is formulated for injection. In some embodiments, the pharmaceutical composition is formulated for topical administration.

In some embodiments, the compounds described herein are formulated in oral dosage forms. Two or more compounds according to the invention are formulated by combining them with, e.g., pharmaceutically acceptable carriers or excipients. In various embodiments the compounds according to the invention are formulated in oral dosage forms including, by way of example only, tablets, powders, granules, pills, dragees, capsules, liquids, serums, gels, solutions, syrups, elixirs, slurries, suspensions, emulsions and the like.

### Orally administered forms

In certain embodiments, pharmaceutical preparations containing the active agents for oral use are obtained by mixing one or more solid excipient with one or more of the compounds described herein, optionally grinding the resulting mixture, and processing the mixture of granules, after adding suitable auxiliaries, if desired, to obtain tablets, pills, or dragee cores. Suitable excipients are, in particular, fillers such as sugars, including lactose, sucrose, mannitol, or sorbitol; cellulose preparations such as: for example, maize starch, wheat starch, rice starch, potato starch, gelatin, gum tragacanth, methylcellulose, microcrystalline cellulose, hydroxypropylmethylcellulose, sodium carboxymethylcellulose; or others such as: polyvinylpyrrolidone (PVP or povidone) or calcium phosphate. In specific embodiments, disintegrating agents are optionally added. Disintegrating agents include, by way of example only, cross linked croscarmellose sodium, polyvinylpyrrolidone, agar, or alginic acid or a salt thereof such as sodium alginate.

In one embodiment, dosage forms, such as dragee cores, pills, and tablets, are provided with one or more suitable coatings. In specific embodiments, concentrated sugar solutions are used for coating the dosage form. The sugar solutions, optionally contain additional components, such as by way of example only, gum arabic, talc, polyvinylpyrrolidone, carbopol gel, polyethylene glycol, and/or titanium dioxide, lacquer solutions, and suitable organic solvents or solvent mixtures. Dyestuffs and/or pigments are also optionally added to the coatings for identification purposes. Additionally, the dyestuffs and/or pigments are optionally utilized to characterize different combinations of active compound doses.

In certain embodiments, therapeutically effective amounts of the active agents described herein are formulated into other solid oral dosage forms. Oral dosage forms include push fit capsules made of gelatin, as well as soft, sealed capsules made of gelatin and a plasticizer, such as glycerol or sorbitol. In specific embodiments, push fit capsules contain the active ingredients in admixture with one or more filler. Fillers include, by way of example only, lactose, binders such as starches, and/or lubricants such as talc or magnesium stearate and, optionally, stabilizers. In other embodiments, soft capsules, contain one or more active compound that is dissolved or suspended in a suitable liquid. Suitable liquids include, by way of example only, one or more fatty oil, liquid paraffin, or liquid polyethylene glycol. In addition, stabilizers are optionally added.

In other embodiments, active agents described herein are formulated into oral liquid dosage forms. Exemplary liquid preparations for oral use include solutions, emulsions, serums, solutions, syrups or suspensions containing one or more active ingredients in a suitable vehicle. Syrups are clear viscous oral liquids containing high concentrations of sugar or other sweetening agents, in which active agents are solubilized in a pharmaceutically acceptable vehicle. Suspensions consist of finely divided particles of active agent suspended in pharmaceutically acceptable vehicle in which the particles are poorly soluble. Oral emulsions contain liquid forms of active agents dispersed as droplets in a continuous phase of another immiscible vehicle with the help of emulsifying agents (e.g. carbohydrates, gelatin, high molecular weight alcohols, wetting agents, colloidal clays, and the like).

In some embodiments, active agents are formulated into semi-solid oral dosage forms such as gels. Gels or jelly-like formulations have particular relevance for elderly or dysphagic patients with difficulty consuming other oral dosage forms. Gels are formed by adding active agents to water, adding a low critical concentration (e.g. 0.5-2.5%) of a gelling agent, heating, and cooling. Examples of suitable gelling agents include agar, gelatin, carrageenan, sodium caseinate, glycerogelatin, silk fibroin, gellan gum, kelcogel, xyloglucan, gellan, and pectin.

### Animal Feed

In certain embodiments, the active agents are included in a diet which can comprise any suitable pet food formulation which also provides adequate nutrition for a non-human animal. For example, a typical canine diet for use in the present invention may contain about 18-40% crude protein, about 4-30% fat, and about 4-20% total dietary fiber. However, no specific ratios or percentages of these or other nutrients are required. Examples of detailed preparation of animal feed from base ingredients are found elsewhere, for e.g. in US 4,045,585, US20100303968, and US 3,875,304.

### Altered Release

A pharmaceutical composition comprising any of the active agents described herein may be formulated for sustained or slow release, also called timed release or controlled release. Such compositions may generally be prepared using well known technology and administered by, for example, oral, rectal, intradermal, or subcutaneous implantation, or by implantation at the desired target site. Sustained-release formulations may contain the compound dispersed in a carrier matrix and/or contained within a reservoir surrounded by a rate controlling membrane. Excipients for use within such formulations are biocompatible, and may also be biodegradable; preferably the formulation provides a relatively constant level of active component release. The amount of pharmaceutical agent contained within a sustained release formulation depends upon the site of implantation, the rate and expected duration of release, and the nature of the condition, disease or disorder to be treated or prevented.

### Injectable

In still other embodiments, the active agents described herein are formulated for parental injection, including formulations suitable for bolus injection or continuous infusion. In specific embodiments, formulations for injection are presented in unit dosage form (e.g., in ampoules) or in multi-dose containers. Preservatives are, optionally, added to the injection formulations. In still other embodiments, the pharmaceutical compositions are formulated in a form suitable for parenteral injection as sterile suspensions, solutions or emulsions in oily or aqueous vehicles. Parenteral injection formulations optionally contain formulatory agents such as suspending, stabilizing and/or dispersing agents. In specific embodiments, pharmaceutical formulations for parenteral administration include aqueous solutions of the active compounds in water-soluble form. In additional embodiments, suspensions of the active agents are prepared as appropriate oily injection suspensions. Suitable lipophilic solvents or vehicles for use in the pharmaceutical compositions described herein include, by way of example only, fatty oils such as sesame oil, or synthetic fatty acid esters, such as ethyl oleate or triglycerides, or liposomes. In certain specific embodiments, aqueous injection suspensions contain substances which increase the viscosity of the suspension, such as sodium carboxymethyl cellulose, sorbitol, or dextran. Optionally, the suspension contains suitable stabilizers or agents which increase the solubility of the compounds to allow for the preparation of highly concentrated solutions. Alternatively, in other embodiments, the active ingredient is in powder form for constitution with a suitable vehicle, e.g., sterile pyrogen-free water, before use.

### Topical

In still other embodiments, the active agents are administered topically. The compounds described herein are formulated into a variety of topically administrable compositions, such as solutions, suspensions, lotions, gels, foams, serums, pastes, medicated sticks, balms, creams or ointments. Such pharmaceutical compositions optionally contain solubilizers, stabilizers, tonicity enhancing agents, buffers and preservatives.

Pharmaceutical compositions comprising a pharmaceutical agent can be formulated as emulsions for topical application. An emulsion contains one liquid distributed in the body of a second liquid. The emulsion may be an oil-in-water emulsion or a water-in-oil emulsion. Either or both of the oil phase and the aqueous phase may contain one or more surfactants, emulsifiers, emulsion stabilizers, buffers, and other excipients. The oil phase may contain other oily pharmaceutically approved excipients. Suitable surfactants include, but are not limited to, anionic surfactants, non-ionic surfactants, cationic surfactants, and amphoteric surfactants. Compositions for topical application may also include at least one suitable suspending agent, antioxidant, chelating agent, emollient, or humectant.

In certain embodiments, the pharmaceutical compositions comprising the active agents are formulated for topical administration using a bandage or transdermal patch, or as a powder/talc or other solid, liquid, spray, aerosol, ointment, foam, cream, gel, or paste. This preferably is in the form of a controlled release formulation or sustained release formulation administered topically or injected directly into the skin adjacent to or within the area to be treated, e.g., intradermally or subcutaneously. The active compositions can also be delivered via iontophoresis. Preservatives can be used to prevent the growth of fungi and other microorganisms. Suitable preservatives include, but are not limited to, benzoic acid, butylparaben, ethyl paraben, methyl paraben, propylparaben, sodium benzoate, sodium propionate, benzalkonium chloride, benzethonium chloride, benzyl alcohol, cetypyridinium chloride, chlorobutanol, phenol, phenylethyl alcohol, thimerosal, and combinations thereof.

### Hair care products

In some embodiments, the active agents according to the invention are formulated into a hair care product. In some embodiments, the active agent in the hair care product comprises alpha-ketoglutarate salt. Examples of hair care products useful for administering the two or more compounds include a shampoo, a conditioner, a hair spray, or a moisturizer. In some embodiments, the alpha-ketoglutarate is formulated in a shampoo. A shampoo is a preparation comprising a surfactant (for e.g. sodium lauryl sulfate) and other additives specifically selected to remove surface grease, dirt, and skin debris from the hair shaft and scalp. An exemplary liquid shampoo formulation would be an aqueous solution containing 40% sodium lauryl sulfate, 2-4% sodium chloride (adjusted to desired viscosity), an effective amount of the 2 or more compounds, a preservative, and optional perfumes or colors. A conditioner or moisturizer is a preparation comprising a conditioning or moisturizing substance which adheres to hair in the presence of water. Examples of conditioning or moisturizing substances suitable for use in conditioners include quaternized surfactants, cationic polymers, silicone compounds (for e.g. polydimethylsiloxane, cyclomethicone), emollients, and humectants. An exemplary hair spray comprises a near 50:50 mix of buffered water and ethanol as diluents, alongside low concentrations of a humectant (e.g. glycerol), a conditioner, and a hair styling polymer (e.g. PVP K-30).

### Administration Protocol and Schedule

In some embodiments one or more of the active agents are administered separately. In some embodiments, the active agents administered separately are administered in separate dosage units (e.g. pills, dragees, tablets). In some embodiments, the active agents administered separately are administered via separate routes of administration. In certain embodiments, two or more of the active agents are administered separately. In certain embodiments, three of the active agents are administered separately. In certain embodiments, the active agents administered separately are not administered simultaneously.

In certain embodiments, the active agents not administered simultaneously are administered within a defined window. In specific embodiments, the defined window is 48, 36, 24, 12, or 6 hours. In some embodiments one or more, two or more, or three of the active agents are administered within the defined window.

In some embodiments, the active agents or compositions thereof used for treatment are administered for a particular treatment period. In some embodiments, the active agents or compositions thereof are administered for a chronic treatment period, that is, for an extended period of time, including throughout the duration of the patient's life in order to ameliorate or otherwise control or limit the symptoms of the patient's disease or condition. Within the treatment period, the active agents or compositions thereof used for treatment are administered on a particular time schedule. In further embodiments, the active agents or compositions thereof are administered one, two, three, or four times daily. In some embodiments, the active agents or compositions thereof are administered in the morning and evening. In some embodiments, the active agents or compositions thereof are administered one, two, three, or four times weekly. In some embodiments, the active agents or compositions thereof are administered one, two, three, or four times monthly. In some embodiments the active agents or compositions are taken for at least three months with one year. In some embodiments, the active agents or compositions are taken one month of every six months.

### EXAMPLES

### Example 1. GRAS compound screens for lifespan extension in a Caenorhabditis elegans longevity assay.

### Nematode Strains and Growth

Strains were cultured under standard laboratory conditions. Strains used in this work include N2, CF1038 [daf-16(mu86)I], TJ1052 [age-l (hx546)II], PS3551 [hsf-l(sy441)I], and TJ356 [zis356 IV [daf-16::daf-16-gfp; pRF4 (rol-6(sul006))]. Wild-type N2 and age-1 strains served as negative and positive controls, respectively, and daf-16/hsf-1 strains were used in epistasis experiments to interrogate the pathway involvement of lifespan extension compounds. For interrogating mTOR involvement in compound activity without effects on development, RNAi treatment using TOR-associated genes was used (see, for e.g. Sobida-Stubbs, S. et al. TOR signaling and rapamycin influence longevity by regulating SKN-1/Nrf and DAF-16/FoxO. Cell Metab. 15, 713-724 (2012)).

### Epistasis experiments to assess pathway involvement

For epistasis experiments, an RNAi-treated/mutant nematode strain was treated compound and vehicle, the magnitude of the effect on a given phenotype was observed (lifespan, fertility, disease-related protein markers) to identify a less than additive, equal, or more than additive effect in combination with genetic interventions.

### Assay

Lifespan assays were performed essentially as described previously (McColl, G. et al. Pharmacogenetic analysis of lithium-induced delayed aging in Caenorhabditis elegans. J Biol Chem 283, 350-357 (2008)).

Briefly, the nematode growth media (NGM) plates were prepared under sterile conditions. 100 µL of concentrated stocks of each of the compounds used in this study were added onto a previously prepared NGM small plate (3 mL volume) immediately spread over the surface of the plate. The final concentrations quoted in the text assume an even distribution of compound throughout the 3 ml plate. The plates were then placed in a laminar flow hood at room temperature for 30 min and then 60 µL of a concentrated suspension of *E*. *coli* OP50 was spotted to form a circular lawn on the center of each plate. Thirty late L4 larvae growing at 20° C. were transferred to fresh NGM plates with fluorodeoxyuridine (FudR,75 µM, to synchronize aging of nematodes) in presence or absence of the specified compounds and incubated at 20° C. The first day of adulthood is Day 3 in survival curves.

Animals were scored as alive, dead or lost every other day. Animals that failed to display touch-provoked movement were scored as dead. Animals that died from causes other than ageing, such as sticking to the plate walls, internal hatching of eggs ("bagging") or gonadal extrusion were censored as were lost worms. Animals were transferred to fresh plates every 3-6 days. All lifespan experiments were performed at 20° C. unless otherwise stated. Survival curves were plotted and statistical analyses (log-rank test) were performed using the Prism 4 software.

### Demographic Analysis of Nematodes

Estimates of initial mortality rate and rate of increase with age and model fitting were made using WinModest or the R-port of the program, Rmodest. Gompertz mortality curves, ln(ux)=ln(a)+bx, where ux defines the age-specific hazard, were fitted with log-likelihood ratios used to examined the effects of constraining the intercept (a) or gradient (b) variables.

### RNA Interference Knockdown of Gene Expression

RNAi bacterial strains expressing double-stranded RNA that inactivates specified genes were cultured and utilized as previously described. (Timmons, L., Court, D. L. & Fire, A. Ingestion of bacterially expressed dsRNAs can produce specific and potent genetic interference in Caenorhabditis elegans. Gene 263, 103-112 (2001).) Briefly, eggs isolated from synchronous populations of N2 cultures were placed on fresh RNAi plates and allowed to grow at 15° C.; 3 days later, L4 molt animals were transferred to new plates seeded with the same bacteria in presence or absence of compound and switched to 25° C. The cultures were scored for paralysis after 48 h of treatment as described above. In all cases, 1 mM isopropyl-beta-D-thiogalactopyranoside (IPTG) was used for induction of double stranded RNA. In all the cases the identity of the clones was confirmed by sequencing.

### Immunostaining and Photomicroscopy

For fluorescent microscopy, GFP-labeled nematode strains were paralyzed with 1 mM levamisole mounted on 1% agarose pads and imaged using Olympus BX51 (60x objective) and HCimage software (Hamamatsu). For immunofluorescence, N2 wildtype or mutant worms were treated for 24-36 h with or without selected lifespan extension compounds at 25° C. After this period the worms were collected, rinsed, and fixed in 4% paraformaldehyde overnight. After fixation, worms were rinsed twice with 1 ml of 10 mM Tris-HCl pH 7.5 and then permeabilized by 24 h exposure to β-mercaptoethanol at 37° C. followed by collagenase treatment (2 mg/ml for 1-1.5 at 37° C.) to allow for digestion of the cuticle. Samples were then treated with commercially acquired primary monoclonal antibodies according to manufacturer's standard conditions and AlexaFluor 633 goat anti-mouse (Molecular Probes) secondary antibody according to manufacturer's conditions. Images were processed and quantified by using Image Analyst MK II (Novato, Calif.).

### Example 2. GRAS compound trial in a frailty mouse model.

### Animals

C57BL/6J mice of both sexes are aged in group caging in an approved animal facility. Mice are maintained on a 12-hour light/dark cycle, with free access to food and water. In general, initiation of interventions occurs in mice after 12 months of age and is maintained throughout the lifespan of the mice. However, short term or periodic interventional strategies may also be tested.

### Intervention and Assessment

Mice are randomly assigned to either treatment or control group, and administered compositions of active agents or placebo (control) in food for a sufficient treatment period to observe effects on frailty characteristics.

Mice are periodically evaluated on the 31-item clinical frailty index previously described herein. This was first reported by Whitehead et al. (Journals of Gerontology: BIOLOGICAL SCIENCES, 69:621-632; 2014). This has been used in studies recently as a valuable metric of mouse frailty that involves non-invasive scoring ideal for longitudinal studies and corresponds well with the onset of human frailty (Rockwood et al., Scientific Reports, 7, 43068, 2017).

### Cohort 1

| **Group** | **# of Males** | **# of Females** |
|---|---|---|
| Control | 24 | 24 |
| Ca-AKG | 22 | 20 |
| Berberine | 22 | 20 |
| Retinoic Acid (RA) | 23 | 20 |
| Double (Ca-AKG +Berberine) | 0 | 19 |
| Triple (Ca-AKG + Berberine + RA) | 22 | 24 |

### Cohort 2

| **Group** | **# of Males** | **# of Females** |
|---|---|---|
| Control | 32 | 25 |
| Ca-AKG | 25 | 25 |
| Retinyl palmitate (RP) | 25 | 25 |
| Ca-AKG + RP | 30 | 25 |
| Vitamin D3 | 20 | 25 |
| Ca-AKG + Vitamin D3 | 0 | 25 |

The 31 point metric for assessment of animal frailty is included in the following table:

| | | |
|---|---|---|
| Alopecia | Tumors | Diarrhea |
| Coat Condition | **Vestibulocochlear/Auditory** | Malocclusions |
| Dermatitis | Hearing loss | Rectal prolapse |
| Loss of Fur Color | Vestibular disturbance | **Vaginal/uterine/penile prolapse** |
| Loss of whiskers | **Ocular/Nasal** | **Respiratory** |
| **Physical/Musculoskeletal** | Cataracts | Breathing rate/depth |
| Body condition score | Corneal discharge | Discomfort |
| Distended abdomen | Eye discharge/swelling | Mouse grimace scale |
| Gait disorder | Menace reflex | Piloerection |
| Grip strength | Microphthalmia | **Other** |
| Kyphosis | Nasal discharge | Temperature |
| Tail stiffaning | Vision loss | Weight |

For all individual frailty metrics shown, a similar strategy was used to assess frailty score. Using a double blind method, scorers were asked to assess the relative score of each animal either as 0 (healthy), .5 (moderately unhealthy) and 1 (unhealthy). For total frailty, all 31 metrics are added and divided by 31. For cohort 2, we report the total frailty score simply as the sum of all 31 metrics without normalization.

To make all scoring consistent, each scorer is periodically asked to assess the same animal and any differences in scores are mediated and resolved.

Loss of fur color and appearance of grey hair is used interchangeably.

Alopecia or hair loss is used interchangeably.

Kyphosis is a measure of exaggerated outward curvature of the lower cervical/thoracic vertebral column, and can reflect either loss of bone density or increased muscle weakness.

Tremors are a measure of involuntary shaking at rest or during movement.

Dermatitis is a routine occurrence in aging C57/Bl6 mice.

Body condition score involves visual scoring and/or palpitation to assess the state of health of the animal. Signs of frailness result in higher frailty scores.

Piloerection is defined as involuntary hair bristling and can be thought of similarly to "goose bumps." This increases with age and can be triggered by coldness, reflecting an inability of the mice to maintain body temperature.

### Example 3. Cell senescence assay

To measure the effects of Ca-AKG on cell senescence, IMR-90 fibroblasts in culture were exposed to gamma-irradiation either after pretreatment with AKG or prior to (post) treatment with Ca-AKG. Cell senescence was measured by β-Galactosidase fluorescence. Pretreatment of Ca-AKG protected cells from irradiation-induced senescence, suggesting that one mechanism by which Ca-AKG might mediate lifespan extension and frailty reduction is through prevention of cell senescence.

### Example 4. Ca-AKG trial in a frailty mouse model.

### Animals

C57BL/6J mice of both sexes are aged in group caging in an approved animal facility. Mice are maintained on a 12-hour light/dark cycle, with free access to food and water. In general, initiation of interventions occurs in mice after 12 months of age and is maintained throughout the lifespan of the mice. However, short term or periodic interventional strategies may also be tested.

### Intervention and Assessment

Mice are randomly assigned to either treatment or control group, and administered compositions comprising Ca-AKG or placebo in food for a sufficient treatment period to observe effects on frailty characteristics.

Mice are periodically evaluated on the 31-item clinical frailty index previously described herein.

### Example 5. Trial of Ca-AKG dietary supplements for improving frailty in a human.

### Patient Selection

A random population of adult individuals above the age of 40 and with no signs of terminal or mental illness is subjected to analysis using the 70-item CSHA Frailty Index (Theou et al. Age Ageing 42: 614-619 (2013)) as described previously herein. A subset may also be assessed for biomarkers of aging and physiologic markers of age-related chronic disease states.

Individuals with a score suggesting that they are pre-frail are then evaluated for secondary measures: individuals with uncontrolled diabetes mellitus, cognitive impairment, malnourishment, or who regularly take alpha-ketoglutarate supplements are excluded.

Individuals not excluded by the secondary evaluation are then randomized into two groups (experimental group receiving a composition of the Ca-AKG and control group receiving placebo) using block randomization and stratification by sex. Numbers are assigned to subjects and randomized selection of the numbers is performed by a research team member uninvolved in the study. Subjects are directed to take the Ca-AKG or placebo according to the schedule established.

### Data Collection

Individuals selected are subjected to a baseline assessment at the start of the study, including detailed sociodemographic data, medical history, a frailty status assessment, evaluation of physical function, evaluation of cognitive function, assessment of nutritional status, and evaluation of selected frailty biomarkers via blood sampling.

A second assessment is performed at the midpoint of the study, including a frailty status assessment, evaluation of physical function, evaluation of cognitive function, and assessment of nutritional status.

A third assessment is performed at the end of the study, including the same assessments as at baseline. All assessments are performed by medical professionals blinded to patient assignment to treatment or control groups.

At the end of the study, appropriate statistical methods (for e.g. Mann-Whitney test was employed for the continuous variables and chi-square test for the categorical variables) are applied to determine the characteristic differences between the two groups. Differences in frailty index and frailty biomarkers are evaluated to determine the effect of administration of Ca-AKG to pre-frail humans.

### Example 6. Trial of Ca-AKG dietary supplements.

The study is an open label safety study. Volunteers take a composition comprising Ca-AKG over a 9-month period in the form of a tablet. The volunteers are divided into three cohorts. The volunteers are monitored for changes in general health, for example, via responses to a questionnaire, and lab panels such as comprehensive metabolic profile, complete blood count and lipid panel, blood pressure, heart rate, cardiac output, stem cells, cells senescent, IGF1, and inflammation CRP.
**Study Type:**
   Safety trial
**Study Design:**
   Length: Nine months
   Allocation: Randomized
   Masking: Double (Participant Investigator)
   Primary Purpose: Safety assessment

### Cohorts:

| Cohort | # of Volunteers | Dose (# of Tablets) | Regimen |
|---|---|---|---|
| 1 | 10-30 | 1 | QD/ evening after a meal & without other medication^{a} |
| 2 | 10-30 | 2 | QD/ evening after a meal & without other medication^{a} |
| 3 | 10-30 | 3 | QD/ evening after a meal & without other medication^{a} |

### a. All medications should be taken in the morning

### Primary Outcome Measures:

Safety and tolerability of single and multiple doses of a composition described herein may be measured by adverse events (AEs), physical examinations (PE), vital signs (VS), laboratory safety tests, urinalysis and 12-lead electrocardiograms (ECG).

### Secondary Outcome Measures:

- Pharmacokinetics of single and multiple doses of a composition described herein may be measured by maximum observed concentration (Cmax). Time Frame: 30 days
- Pharmacokinetics of single and multiple doses of a composition described herein may be measured by area under the curve (AUC). Time Frame: 30 days
- Pharmacokinetics of single and multiple doses of a composition described herein may be measured by terminal half life (t1/2). Time Frame: 30 days
- Pharmacokinetics of single and multiple doses of a composition described herein may be measured by volume of distribution (Vd). Time Frame: 30 days
- Pharmacokinetics of single and multiple doses of a composition described herein may be measured by elimination rate constant (Kel). Time Frame: 30 days
- Pharmacokinetics of single and multiple doses of a composition described herein may be measured by accumulation ratio. Time Frame: 30 days

### Inclusion Criteria:

- Men 45-60 years
- Female 45-60 years

### Exclusion Criteria:

- Females of child-bearing age
- Pregnant females
- Lactating females
- Diabetics
- Heart disease
- Cancer
- Morbidly obese

### Exemplary Questionnaire:

- How do you feel today?
- Have you noticed any changes in your health?
- Have you had any gastrointestinal problems?
- Have you noticed any changes in hair?
- Have you noticed any changes in hair color (such as darkening of the hair)?
- Have you noticed any improvements in your memory, such as better short term recall?
- Any improvements in your balance?
- Any improvements in your vision?
- Any improvements in your stamina or sense of well-being?

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

### CLAUSES

1. A method of extending lifespan in a subject in need thereof comprising administering to the subject a therapeutically effective amount of berberine, a vitamin A compound, and a-ketoglutarate (AKG).
2. The method of clause 1, wherein berberine, the vitamin A compound and AKG is administered to the subject as a single composition.
3. The method of clause 1, wherein the berberine, the vitamin A compound, and a-ketoglutarate are administered to the subject separately.
4. The method of clause 3, wherein the berberine, the vitamin A compound, and a-ketoglutarate are administered to the subject within a 24 hour period.
5. The method of clause 1, wherein the vitamin A compound is selected from the group consisting of retinol, retinal, retinoic acid, retinyl palmitate, alpha-carotene, beta-carotene, and gamma- carotene.
6. The method of clause 5, wherein the vitamin A compound is retinoic acid or retinyl palmitate.
7. The method of clause 1, wherein AKG is formulated as a calcium salt.
8. The method of clause 1, wherein the subject is a mammal.
9. The method of clause 8, wherein the mammal is a human.
10. The method of clause 8, wherein the mammal is a dog.
11. The method of clause 8, wherein the mammal is a cat.
12. The method of clause 8, wherein the mammal is livestock.
13. The method of clause 12, wherein the livestock is selected from the group consisting of cattle, sheep, goats, swine, poultry, and equine animals.
14. The method of clause 1, wherein the berberine, the vitamin A compound, and a-ketoglutarate are administered once daily.
15. The method of clause 1, wherein the berberine, the vitamin A compound, and a-ketoglutarate are administered twice daily.
16. The method of clause 1, wherein the berberine, the vitamin A compound, and a-ketoglutarate are administered in the morning and evening.
17. The method of clause 1, wherein the berberine, the vitamin A compound, and a-ketoglutarate are administered once a week.
18. The method of clause 1, wherein the berberine, the vitamin A compound, and a-ketoglutarate are administered once a month.
19. A method of delaying onset or delaying progression of frailty in a subject in need thereof comprising administering to the subject a therapeutically effective amount of berberine, a vitamin A compound, and α-ketoglutarate (AKG).
20. The method of clause 19, wherein berberine, the vitamin A compound and AKG is administered to the subject as a single composition.
21. The method of clause 19, wherein the berberine, the vitamin A compound, and a- ketoglutarate are administered to the subject separately.
22. The method of clause 21, wherein the berberine, the vitamin A compound, and a- ketoglutarate are administered to the subject within a 24 hour period.
23. The method of clause 19, wherein the vitamin A compound is selected from the group consisting of retinol, retinal, retinoic acid, retinyl palmitate, alpha-carotene, beta-carotene, and gamma- carotene.
24. The method of clause 23, wherein the vitamin A compound is retinoic acid or retinyl palmitate.
25. The method of clause 19, wherein AKG is formulated as a calcium salt.
26. The method of clause 19, wherein the subject is a mammal.
27. The method of clause 26, wherein the mammal is a human.
28. The method of clause 26, wherein the mammal is a dog.
29. The method of clause 26, wherein the mammal is a cat.
30. The method of clause 26, wherein the mammal is livestock.
31. The method of clause 30, wherein the livestock is selected from the group consisting of cattle, sheep, goats, swine, poultry, and equine animals.
32. The method of clause 19, wherein the berberine, the vitamin A compound, and a- ketoglutarate are administered once daily.
33. The method of clause 19, wherein the berberine, the vitamin A compound, and a- ketoglutarate are administered twice daily.
34. The method of clause 19, wherein the berberine, the vitamin A compound, and a- ketoglutarate are administered in the morning and evening.
35. The method of clause 19, wherein the berberine, the vitamin A compound, and a- ketoglutarate are administered once a week.
36. The method of clause 19, wherein the berberine, the vitamin A compound, and a- ketoglutarate are administered once a month.
37. A composition comprising a therapeutically effective amount of berberine, a therapeutically effective amount of a vitamin A compound, a therapeutically effective amount of AKG, and a pharmaceutically acceptable excipient.
38. The composition of clause 37, wherein the vitamin A compound is selected from the group consisting of retinol, retinal, retinoic acid, retinyl palmitate, alpha-carotene, beta-carotene, and gamma- carotene.
39. The composition of clause 38, wherein the vitamin A compound is retinoic acid or retinyl palmitate.
40. The composition of clause 37, wherein AKG is formulated as a calcium salt.
41. The composition of clause 37, wherein the composition further comprises silymarin.
42. The composition of clause 37, wherein the therapeutically effective amount of berberine is at least 1,000 mg and no greater than 5,000 mg.
43. The composition of clause 37, wherein the therapeutically effective amount of the vitamin A compound is at least 10,000 IU and no greater than 20,000 IU.
44. The composition of clause 37, wherein the therapeutically effective amount of a-ketoglutarate is at least 350 mg and no greater than 750 mg.
45. The composition of clause 37, wherein the composition is formulated into an orally administered form.
46. The composition of clause 45, wherein the orally administered form comprises a tablet, a powder, a suspension, a serum, an emulsion, a capsule, a granule, a pill, a gel, a solution, or a syrup.
47. The composition of clause 45, wherein the orally administered form is a sustained release dosage form.
48. The composition of clause 45, wherein the orally administered form is formulated into animal feed.
49. The composition of clause 46, wherein the orally administered form is the gel.
50. The composition of clause 37, wherein the composition is formulated into an injectable administered form.
51. The composition of clause 50, wherein the injectable administered form comprises a liquid, a suspension, or a solution.
52. The composition of clause 50, wherein the injectable administered form is a sustained release dosage form.
53. The composition of clause 37, wherein the composition is formulated into a topically administered form.
54. The composition of clause 53, wherein the topically administered form is a cream, a foam, a gel, a lotion, an ointment, or a serum.
55. The composition of clause 37, wherein the composition is formulated into a hair care product.
56. The composition of clause 55, wherein the hair care product is a shampoo, a conditioner, hair spray, or a moisturizer.
57. The composition of clause 37, wherein the pharmaceutically acceptable excipient comprises an antiadherent, a binder, a coating, a color, disintegrant, a flavor, a glidant, a lubricant, a preservative, a sorbent, or a vehicle.
58. A method of delaying onset or delaying progression of frailty in a subject in need thereof comprising administering to the subject a therapeutically effective amount of AKG and a vitamin A compound.
59. The method of clause 58, wherein AKG and the vitamin A compound is administered to the subject as a single composition.
60. The method of clause 58, wherein AKG and the vitamin A compound are administered to the subject separately.
61. The method of clause 60, wherein AKG and the vitamin A compound are administered to the subject within a 24 hour period.
62. The method of clause 58, wherein the vitamin A compound is selected from the group consisting of retinol, retinal, retinoic acid, retinyl palmitate, alpha-carotene, beta-carotene, and gamma- carotene.
63. The method of clause 62, wherein the vitamin A compound is retinoic acid or retinyl palmitate.
64. The method of clause 58, wherein AKG is formulated as a calcium salt.
65. The method of clause 58, wherein the subject is a mammal.
66. The method of clause 65, wherein the mammal is a human.
67. The method of clause 65, wherein the mammal is a dog.
68. The method of clause 65, wherein the mammal is a cat.
69. The method of clause 65, wherein the mammal is livestock.
70. The method of clause 69, wherein the livestock is selected from the group consisting of cattle, sheep, goats, swine, poultry, and equine animals.
71. The method of clause 58, wherein the AKG and the vitamin A compound are administered once daily.
72. The method of clause 58, wherein AKG and the vitamin A compound are administered twice daily.
73. The method of clause 58, wherein AKG and the vitamin A compound are administered in the morning and evening.
74. The method of clause 58, wherein AKG and the vitamin A compound are administered once a week.
75. The method of clause 58, wherein AKG and the vitamin A compound are administered once a month.
76. The method of clause 58, wherein the subject is female.
77. A method of treating or preventing alopecia in a subject in need thereof comprising administering to the subject a therapeutically effective amount of AKG and a vitamin A compound.
78. The method of clause 77, wherein AKG and the vitamin A compound is administered to the subject as a single composition.
79. The method of clause 77, wherein AKG and the vitamin A compound are administered to the subject separately.
80. The method of clause 79, wherein AKG and the vitamin A compound are administered to the subject within a 24 hour period.
81. The method of clause 77, wherein the vitamin A compound is selected from the group consisting of retinol, retinal, retinoic acid, retinyl palmitate, alpha-carotene, beta-carotene, and gamma- carotene.
82. The method of clause 81, wherein the vitamin A compound is retinoic acid or retinyl palmitate.
83. The method of clause 77, wherein AKG is formulated as a calcium salt.
84. The method of clause 77, wherein the subject is a mammal.
85. The method of clause 84, wherein the mammal is a human.
86. The method of clause 84, wherein the mammal is a dog.
87. The method of clause 84, wherein the mammal is a cat.
88. The method of clause 84, wherein the mammal is livestock.
89. The method of clause 88, wherein the livestock is selected from the group consisting of cattle, sheep, goats, swine, poultry, and equine animals.
90. The method of clause 77, wherein the AKG and the vitamin A compound are administered once daily.
91. The method of clause 77, wherein AKG and the vitamin A compound are administered twice daily.
92. The method of clause 77, wherein AKG and the vitamin A compound are administered in the morning and evening.
93. The method of clause 77, wherein AKG and the vitamin A compound are administered once a week.
94. The method of clause 77, wherein AKG and the vitamin A compound are administered once a month.
95. A method of extending lifespan in a subject in need thereof comprising administering to the subject a therapeutically effective amount of AKG and vitamin D.
96. The method of clause 95, wherein AKG and vitamin D is administered to the subject as a single composition.
97. The method of clause 95, wherein AKG and vitamin D are administered to the subject separately.
98. The method of clause 97, wherein AKG and vitamin D are administered to the subject within a 24 hour period.
99. The method of clause 95, wherein AKG is formulated as a calcium salt.
100. The method of clause 95, wherein the subject is a mammal.
101. The method of clause 100, wherein the mammal is a human.
102. The method of clause 100, wherein the mammal is a dog.
103. The method of clause 100, wherein the mammal is a cat.
104. The method of clause 100, wherein the mammal is livestock.
105. The method of clause 104, wherein the livestock is selected from the group consisting of cattle, sheep, goats, swine, poultry, and equine animals.
106. The method of clause 95, wherein AKG and vitamin D are administered once daily.
107. The method of clause 95, wherein AKG and vitamin D are administered twice daily.
108. The method of clause 95, wherein AKG and vitamin D are administered in the morning and evening.
109. The method of clause 95, wherein AKG and vitamin D are administered once a week.
110. The method of clause 95, wherein AKG and vitamin D are administered once a month.
111. The method of clause 95, wherein the subject is female.
112. A method of delaying onset or delaying progression of frailty in a subject in need thereof comprising administering to the subject a therapeutically effective amount of AKG and vitamin D.
113. The method of clause 112, wherein AKG and vitamin D is administered to the subject as a single composition.
114. The method of clause 112, wherein AKG and vitamin D are administered to the subject separately.
115. The method of clause 114, wherein AKG and vitamin D are administered to the subject within a 24 hour period.
116. The method of clause 112, wherein AKG is formulated as a calcium salt.
117. The method of clause 112, wherein the subject is a mammal.
118. The method of clause 117, wherein the mammal is a human.
119. The method of clause 117, wherein the mammal is a dog.
120. The method of clause 117, wherein the mammal is a cat.
121. The method of clause 117, wherein the mammal is livestock.
122. The method of clause 121, wherein the livestock is selected from the group consisting of cattle, sheep, goats, swine, poultry, and equine animals.
123. The method of clause 112, wherein AKG and vitamin D are administered once daily.
124. The method of clause 112, wherein AKG and vitamin D are administered twice daily.
125. The method of clause 112, wherein AKG and vitamin D are administered in the morning and evening.
126. The method of clause 112, wherein AKG and vitamin D are administered once a week.
127. The method of clause 112, wherein AKG and vitamin D are administered once a month.
128. The method of clause 112, wherein the subject is female.
129. A method of extending lifespan in a subject in need thereof comprising administering to the subject a composition comprising a therapeutically effective amount of a calcium salt of alpha-ketoglutarate (Ca-AKG).
130. The method of clause 129, wherein the composition consists essentially of a therapeutically effective amount of a calcium salt of alpha-ketoglutarate (Ca-AKG).
131. The method of clause 129, wherein the subject is a mammal.
132. The method of clause 131, wherein the mammal is a human.
133. The method of clause 131, wherein the mammal is a dog.
134. The method of clause 131, wherein the mammal is a cat.
135. The method of clause 131, wherein the mammal is livestock.
136. The method of clause 135, wherein the livestock is selected from the group consisting of cattle, sheep, goats, swine, poultry, and equine animals.
137. The method of clause 129, wherein the composition is administered once daily.
138. The method of clause 129, wherein the composition is administered twice daily.
139. The method of clause 129, wherein the composition is administered in the morning and evening.
140. The method of clause 129, wherein the composition is administered once a week.
141. The method of clause 129, wherein the composition is administered once a month.
142. The method of clause 129, wherein the subject is female.
143. A method of delaying onset or delaying progression of frailty in a subject in need thereof comprising administering to the subject a composition comprising a therapeutically effective amount of a calcium salt of alpha-ketoglutarate (Ca-AKG).
144. The method of clause 143, wherein the delaying onset or delaying progression of frailty comprises delaying onset or delaying progression of a frailty phenotype.
145. The method of clause 144, wherein the frailty phenotype is selected from the group consisting of dermatitis, kyphosis, tremor, and alopecia.
146. The method of clause 145, wherein the frailty phenotype is dermatitis.
147. The method of clause 145, wherein the frailty phenotype is kyphosis.
148. The method of clause 145, wherein the frailty phenotype is tremor.
149. The method of clause 145, wherein the frailty phenotype is alopecia.
150. The method of clause 143, wherein the composition consists essentially of a therapeutically effective amount of a calcium salt of alpha-ketoglutarate (Ca-AKG).
151. The method of clause 143, wherein the subject is a mammal.
152. The method of clause 151, wherein the mammal is a human.
153. The method of clause 151, wherein the mammal is a dog.
154. The method of clause 151, wherein the mammal is a cat.
155. The method of clause 151, wherein the mammal is livestock.
156. The method of clause 155, wherein the livestock is selected from the group consisting of cattle, sheep, goats, swine, poultry, and equine animals.
157. The method of clause 143, wherein the composition is administered once daily.
158. The method of clause 143, wherein the composition is administered twice daily.
159. The method of clause 143, wherein the composition is administered in the morning and evening.
160. The method of clause 143, wherein the composition is administered once a week.
161. The method of clause 143, wherein the composition is administered once a month.
162. The method of clause 143, wherein the subject is female.
163. The method of clause 143, wherein the composition is formulated into an orally administered form.
164. The method of clause 163, wherein the orally administered form comprises a tablet, a powder, a suspension, a serum, an emulsion, a capsule, a granule, a pill, a gel, a solution, or a syrup.
165. The method of clause 163, wherein the orally administered form is a sustained release dosage form.
166. The method of clause 163, wherein the orally administered form is formulated into animal feed.
167. The method of clause 164, wherein the orally administered form is the gel.
168. The method of clause 143, wherein the composition is formulated into an injectable administered form.
169. The method of clause 168, wherein the injectable administered form comprises a liquid, a suspension, or a solution.
170. The method of clause 168, wherein the injectable administered form is a sustained release dosage form.
171. The method of clause 143, wherein the composition is formulated into a topically administered form.
172. The method of clause 171, wherein the topically administered form is a cream, a foam, a gel, a lotion, an ointment, or a serum.
173. The method of clause 143, wherein the composition is formulated into a hair care product.
174. The method of clause 173, wherein the hair care product is a shampoo, a conditioner, hair spray, or a moisturizer.
175. The method of clause 143, wherein the composition further comprises a pharmaceutically acceptable excipient selected from the group consisting of an antiadherent, a binder, a coating, a color, disintegrant, a flavor, a glidant, a lubricant, a preservative, a sorbent, or a vehicle.
176. The method of clause 143, wherein the composition is administered to the subject for at least 4 months.
177. The method of clause 176, wherein the composition is administered to the subject for at least 6 months, at least 8 months, at least 10 months, at least 12 months, at least 14 months, at least 16 months, or at least 18 months.
178. A method of extending healthspan in a subject in need thereof comprising administering to the subject a composition comprising a therapeutically effective amount of a calcium salt of alpha-ketoglutarate (Ca-AKG).
179. The method of clause 178, wherein extending healthspan comprises a delay in onset or progression of an age-related phenotype.
180. The method of clause 179, wherein the age-related phenotype is selected from the group consisting of graying hair, hair loss, and increased cell senescence.
181. The method of clause 180, wherein the age-related phenotype is graying hair.
182. The method of clause 180, wherein the age-related phenotype is hair loss.
183. The method of clause 180, wherein the age-related phenotype is increased cell senescence.
184. The method of clause 178, wherein the composition consists essentially of a therapeutically effective amount of a calcium salt of alpha-ketoglutarate (Ca-AKG).
185. The method of clause 178, wherein the subject is a mammal.
186. The method of clause 185, wherein the mammal is a human.
187. The method of clause 185, wherein the mammal is a dog.
188. The method of clause 185, wherein the mammal is a cat.
189. The method of clause 185, wherein the mammal is livestock.
190. The method of clause 189, wherein the livestock is selected from the group consisting of cattle, sheep, goats, swine, poultry, and equine animals.
191. The method of clause 178, wherein the composition is administered once daily.
192. The method of clause 178, wherein the composition is administered twice daily.
193. The method of clause 178, wherein the composition is administered in the morning and evening.
194. The method of clause 178, wherein the composition is administered once a week.
195. The method of clause 178, wherein the composition is administered once a month.
196. The method of clause 178, wherein the subject is female.
197. The method of clause 178, wherein the composition is formulated into an orally administered form.
198. The method of clause 197, wherein the orally administered form comprises a tablet, a powder, a suspension, a serum, an emulsion, a capsule, a granule, a pill, a gel, a solution, or a syrup.
199. The method of clause 197, wherein the orally administered form is a sustained release dosage form.
200. The method of clause 197, wherein the orally administered form is formulated into animal feed.
201. The method of clause 198, wherein the orally administered form is the gel.
202. The method of clause 178, wherein the composition is formulated into an injectable administered form.
203. The method of clause 202, wherein the injectable administered form comprises a liquid, a suspension, or a solution.
204. The method of clause 202, wherein the injectable administered form is a sustained release dosage form.
205. The method of clause 178, wherein the composition is formulated into a topically administered form.
206. The method of clause 205, wherein the topically administered form is a cream, a foam, a gel, a lotion, an ointment, or a serum.
207. The method of clause 178, wherein the composition is formulated into a hair care product.
208. The method of clause 207, wherein the hair care product is a shampoo, a conditioner, hair spray, or a moisturizer.
209. The method of clause 178, wherein the composition further comprises a pharmaceutically acceptable excipient selected from the group consisting of an antiadherent, a binder, a coating, a color, disintegrant, a flavor, a glidant, a lubricant, a preservative, a sorbent, or a vehicle.
210. The method of clause 178, wherein the composition is administered to the subject for at least 4 months.
211. The method of clause 178, wherein the composition is administered to the subject for at least 6 months, at least 8 months, at least 10 months, at least 12 months, at least 14 months, at least 16 months, or at least 18 months.
212. A method for helping to maintain health in a subject comprising administering to the subject a composition comprising a therapeutically effective amount of berberine, a vitamin A compound, and a-ketoglutarate (AKG).
213. The method of clause 212, wherein the vitamin A compound is selected from the group consisting of retinol, retinal, retinoic acid, retinyl palmitate, alpha-carotene, beta-carotene, and gamma- carotene.
214. The method of clause 213, wherein the vitamin A compound is retinoic acid or retinyl palmitate.
215. The method of clause 212, wherein AKG is formulated as a calcium salt.
216. The method of clause 212, wherein the helping to maintain health comprises helping to maintain a healthy musculoskeletal system.
217. The method of clause 212, wherein the composition is formulated into an orally administered form.
218. The method of clause 217, wherein the orally administered form comprises a tablet, a powder, a suspension, a serum, an emulsion, a capsule, a granule, a pill, a gel, a solution, or a syrup.
219. The method of clause 212, wherein the subject is a human.
220. The method of clause 212, wherein the composition is administered to the subject once daily.
221. The method of clause 212, wherein the composition is administered to the subject twice daily.
222. A method for helping to maintain health in need thereof comprising administering to the subject a composition comprising a therapeutically effective amount of a vitamin A compound, and a-ketoglutarate (AKG).
223. The method of clause 222, wherein the vitamin A compound is selected from the group consisting of retinol, retinal, retinoic acid, retinyl palmitate, alpha-carotene, beta-carotene, and gamma- carotene.
224. The method of clause 223, wherein the vitamin A compound is retinoic acid or retinyl palmitate.
225. The method of clause 222, wherein AKG is formulated as a calcium salt.
226. The method of clause 222, wherein the helping to maintain health comprises helping to maintain a healthy musculoskeletal system.
227. The method of clause 222, wherein the composition is formulated into an orally administered form.
228. The method of clause 227, wherein the orally administered form comprises a tablet, a powder, a suspension, a serum, an emulsion, a capsule, a granule, a pill, a gel, a solution, or a syrup.
229. The method of clause 222, wherein the subject is a human.
230. The method of clause 222, wherein the composition is administered to the subject once daily.
231. The method of clause 222, wherein the composition is administered to the subject twice daily.
232. A method for helping to maintain health in a subject comprising administering to the subject a composition comprising a therapeutically effective amount of a vitamin **D,** and a-ketoglutarate (AKG).
233. The method of clause 232, wherein AKG is formulated as a calcium salt.
234. The method of clause 232, wherein the helping to maintain health comprises helping to maintain a healthy musculoskeletal system.
235. The method of clause 232, wherein the composition is formulated into an orally administered form.
236. The method of clause 235, wherein the orally administered form comprises a tablet, a powder, a suspension, a serum, an emulsion, a capsule, a granule, a pill, a gel, a solution, or a syrup.
237. The method of clause 232, wherein the subject is a human.
238. The method of clause 232, wherein the composition is administered to the subject once daily.
239. The method of clause 232, wherein the composition is administered to the subject twice daily.
240. A method for helping to maintain health in a subject comprising administering to the subject a composition comprising a therapeutically effective amount of a calcium salt of alpha- ketoglutarate (Ca-AKG).
241. The method of clause 240, wherein the helping to maintain health comprises helping to maintain a healthy musculoskeletal system.
242. The method of clause 240, wherein the composition is formulated into an orally administered form.
243. The composition of clause 242, wherein the orally administered form comprises a tablet, a powder, a suspension, a serum, an emulsion, a capsule, a granule, a pill, a gel, a solution, or a syrup.
244. The method of clause 240, wherein the subject is a human.
245. The method of clause 240, wherein the composition is administered to the subject once daily.
246. The method of clause 240, wherein the composition is administered to the subject twice daily.
247. The method of clause 240, wherein the helping to maintain health comprises helping to maintain hair density.
248. The method of clause 240, wherein the helping to maintain health comprises helping to maintain hair pigmentation.
249. The method of clause 240, wherein the helping to maintain health comprises helping to maintain skin health.
250. The method of clause 240, wherein the helping to maintain health comprises helping to maintain normal spine curvature.
251. A method of treating or preventing alopecia in a subject in need thereof comprising administering to the subject a therapeutically effective amount of AKG and vitamin D.
252. The method of clause 251, wherein AKG and vitamin D are administered to the subject as a single composition.
253. The method of clause 251, wherein AKG and vitamin D are administered to the subject separately.
254. The method of clause 253, wherein AKG and vitamin D are administered to the subject within a 24 hour period.
255. The method of clause 251, wherein AKG is formulated as a calcium salt.
256. The method of clause 251, wherein the subject is a mammal.
257. The method of clause 256, wherein the mammal is a human.
258. The method of clause 256, wherein the mammal is a dog.
259. The method of clause 256, wherein the mammal is a cat.
260. The method of clause 256, wherein the mammal is livestock.
261. The method of clause 260, wherein the livestock is selected from the group consisting cattle, sheep, goats, swine, poultry, and equine animals.
262. The method of clause 251, wherein the AKG and vitamin D are administered once daily.
263. The method of clause 251, wherein AKG and vitamin D are administered twice daily.
264. The method of clause 251, wherein AKG and vitamin D are administered in the morning and evening.
265. The method of clause 251, wherein AKG and vitamin D are administered once a week.
266. The method of clause 251, wherein AKG and vitamin D are administered once a month.
267. A method of extending lifespan in a subject in need thereof comprising administering to the subject a therapeutically effective amount of a vitamin A compound and a-ketoglutarate (AKG).
268. The method of clause 267, wherein the vitamin A compound and AKG is administered to the subject as a single composition.
269. The method of clause 267, wherein the vitamin A compound and α-ketoglutarate are administered to the subject separately.
270. The method of clause 269, wherein the vitamin A compound and α-ketoglutarate are administered to the subject within a 24 hour period.
271. The method of clause 267, wherein the vitamin A compound is selected from the group consisting of retinol, retinal, retinoic acid, retinyl palmitate, alpha-carotene, beta-carotene, and gamma- carotene.
272. The method of clause 271, wherein the vitamin A compound is retinoic acid or retinyl palmitate.
273. The method of clause 267, wherein AKG is formulated as a calcium salt.
274. The method of clause 267, wherein the subject is a mammal.
275. The method of clause 274, wherein the mammal is a human.
276. The method of clause 274, wherein the mammal is a dog.
277. The method of clause 274, wherein the mammal is a cat.
278. The method of clause 274, wherein the mammal is livestock.
279. The method of clause 278, wherein the livestock is selected from the group consisting of cattle, sheep, goats, swine, poultry, and equine animals.
280. The method of clause 267, wherein the vitamin A compound and a-ketoglutarate are administered once daily.
281. The method of clause 267, wherein the vitamin A compound and α-ketoglutarate are administered twice daily.
282. The method of clause 267, wherein the vitamin A compound and α-ketoglutarate are administered in the morning and evening.
283. The method of clause 267, wherein the vitamin A compound and α-ketoglutarate are administered once a week.
284. The method of clause 267, wherein the vitamin A compound and α-ketoglutarate are administered once a month.
285. A method of extending healthspan in a subject in need thereof comprising administering to the subject a therapeutically effective amount of a vitamin A compound and α-ketoglutarate (AKG).
286. The method of clause 285, wherein the vitamin A compound and AKG is administered to the subject as a single composition.
287. The method of clause 285, wherein the vitamin A compound and α-ketoglutarate are administered to the subject separately.
288. The method of clause 287, wherein the vitamin A compound and α-ketoglutarate are administered to the subject within a 24 hour period.
289. The method of clause 285, wherein the vitamin A compound is selected from the group consisting of retinol, retinal, retinoic acid, retinyl palmitate, alpha-carotene, beta-carotene, and gamma- carotene.
290. The method of clause 289, wherein the vitamin A compound is retinoic acid or retinyl palmitate.
291. The method of clause 285, wherein AKG is formulated as a calcium salt.
292. The method of clause 285, wherein extending healthspan comprises a delay in onset or progression of an age-related phenotype.
293. The method of clause 292, wherein the age-related phenotype is selected from the group consisting of graying hair, hair loss, and increased cell senescence.
294. The method of clause 293, wherein the age-related phenotype is graying hair.
295. The method of clause 293, wherein the age-related phenotype is hair loss.
296. The method of clause 293, wherein the age-related phenotype is increased cell senescence.
297. The method of clause 285, wherein the subject is a mammal.
298. The method of clause 297, wherein the mammal is a human.
299. A method of extending healthspan in a subject in need thereof comprising administering to the subject a therapeutically effective amount of vitamin D and a-ketoglutarate (AKG).
300. The method of clause 299, wherein vitamin D and AKG is administered to the subject as a single composition.
301. The method of clause 299, wherein vitamin D and AKG are administered to the subject separately.
302. The method of clause 301, wherein vitamin D and α-ketoglutarate are administered to the subject within a 24 hour period.
303. The method of clause 299, wherein AKG is formulated as a calcium salt.
304. The method of clause 299, wherein extending healthspan comprises a delay in onset or progression of an age-related phenotype.
305. The method of clause 304, wherein the age-related phenotype is selected from the group consisting of graying hair, hair loss, and increased cell senescence.
306. The method of clause 305, wherein the age-related phenotype is graying hair.
307. The method of clause 305, wherein the age-related phenotype is hair loss.
308. The method of clause 305, wherein the age-related phenotype is increased cell senescence.
309. The method of clause 305, wherein the subject is a mammal.
310. The method of clause 305, wherein the mammal is a human.
311. A method for helping to maintain health in a subject comprising administering to the subject a composition that modulates pathways associated with normal aging process wherein the composition comprises a therapeutically effective amount of berberine, a vitamin A compound, and a-ketoglutarate (AKG).
312. A method for helping to maintain health in a subject comprising administering to the subject a composition that modulates pathways associated with normal aging process wherein the composition comprises a therapeutically effective amount of a vitamin A compound and a-ketoglutarate (AKG).
313. A method for helping to maintain health in a subject comprising administering to the subject a composition that modulates pathways associated with normal aging process wherein the composition comprises a therapeutically effective amount of vitamin D and α-ketoglutarate (AKG).
314. A method for helping to maintain health in a subject comprising administering to the subject a composition that modulates pathways associated with normal aging process wherein the composition comprises a therapeutically effective amount of a calcium salt of alpha-ketoglutarate (Ca- AKG).
315. A method for helping to maintain health in a subject comprising administering to the subject a composition that modulates pathways associated with normal aging process wherein the composition comprises a therapeutically effective amount of nicotinamide mononucleotide (NMN) and α-ketoglutarate (AKG).

## Claims

1. Calcium salt of α-ketoglutarate (Ca-AKG) and vitamin D for use in delaying onset or delaying progression of frailty, wherein delaying onset or delaying progression of frailty comprises delaying onset or delaying progression of one or more one frailty phenotype selected from the group consisting of hair loss, grip strength, a gait disorder, dermatitis, kyphosis, hearing loss, cataracts, corneal opacity, eye discharge, vision loss, nasal discharge, age-related fat loss, and tremors.

2. Ca-AKG and vitamin D for the use according to claim 1, wherein the Ca-AKG comprises a calcium α-ketoglutarate mono-hydrate.

3. Ca-AKG and vitamin D for the use according to claim 1 or 2, wherein the Ca-AKG and the vitamin D are comprised in a composition.

4. Ca-AKG and vitamin D for the use according to claim 3, wherein the composition further comprises a pharmaceutically acceptable excipient; typically wherein the pharmaceutically acceptable excipient is selected from the group consisting of an anti-adherent, a binder, a coating, a color, a disintegrant, a flavor, a glidant, a lubricant, a preservative, a sorbent, a vehicle, and any combination thereof.

5. Ca-AKG and vitamin D for the use according to claim 3 or 4, wherein the composition is formulated for oral administration; typically wherein the composition is selected from a tablet, a powder, a suspension, a serum, an emulsion, a capsule, a granule, a pill, a gel, a solution, and a syrup.

6. Ca-AKG and vitamin D for the use according to any one of claims 3 to 5, wherein the composition is formulated for sustained release.

7. Ca-AKG and vitamin D for the use according to any one of claims 3 to 6, wherein the composition comprises a therapeutically effective amount of Ca-AKG and vitamin D; typically wherein the therapeutically effective of amount of the Ca-AKG is from 350 mg to 2000 mg.

8. Ca-AKG and vitamin D for the use according to claim 7, wherein the therapeutically effective amount of the Ca-AKG is at least 500 mg.

9. Ca-AKG and vitamin D for the use according to any one of claims 1 to 8, wherein the Ca-AKG and the vitamin D are administered once daily.

10. Ca-AKG and vitamin D for the use according to any one of claims 1 to 8, wherein the Ca-AKG and the vitamin D are administered twice daily.

11. Ca-AKG and vitamin D for the use according to any one of claims 1 to 10, wherein the use is for a mammal; typically wherein the use is for a human, a dog, a cat, or livestock; more typically wherein the use is for a human.

12. Ca-AKG and vitamin D for the use according to any one of claims 1 to 11, wherein the use is for a female.
